# EUROPEAN PATENT APPLICATION

(11) **EP 4 840 510 A2**
(43) Date of publication of application: **18.11.2026**
(21) Application number: 26209563.1
(22) Date of filing: 17.03.2022
(51) Int. Cl.: A61P 25/28

(54) **ZERVIMESINE FOR USE IN THE TREATMENT OF PARKINSON'S DISEASE**

(30) Priority: 19.03.2021 US 202163163546 P
(62) Document number(s) of the earlier application(s) in accordance with Art. 76 EPC: 22772183.4 / 4 308 565
(71) Applicant: Cognition Therapeutics, Inc., Purchase, NY 10577 (US)
(72) Inventor: CAGGIANO, Anthony, Pennsylvania, 15203 (US); CATALANO, Susan M, Pennsylvania, 15203 (US); HAMBY, Mary, Pennsylvania, 15203 (US); IZZO, Nicholas, Pennsylvania, 15217 (US); LOOK, Gary C., California, 95051 (US); RISHTON, Gilbert M., California, 90049 (US)
(74) Representative: HGF

(57) **Abstract**

The present disclosure relates to methods of treating neurological diseases, comprising administering to a subject in need thereof, a therapeutically effective amount of a compound or pharmaceutical composition according to any embodiment described herein. The present disclosure relates to the methods of treating Parkinson's disease and Dementia with Lewy Bodies, comprising administering to a subject in need thereof, a therapeutically effective amount of a compound or pharmaceutical composition according to any embodiment described herein.

## Description

### CROSS-REFERENCE TO RELATED APPLICATIONS

This Application claims priority to U.S. Provisional Application No. 63/163,546 filed on March 19, 2021, which is incorporated herein by reference.

### SUMMARY

The disclosure provides methods of treating neurological diseases, comprising administering to a subject in need thereof, a therapeutically effective amount of a compound or pharmaceutical composition according to any embodiment described herein.

Some embodiments describe a method of treating a neurologic disease, comprising administering to a subject in need thereof, a therapeutically effective amount of a compound selected from the group consisting of:
A. a compound of Formula I, 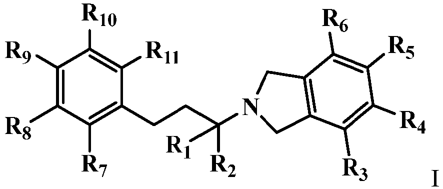 or a pharmaceutically acceptable salt thereof:
   wherein:
   each of R₁ and R₂ is independently selected from H, C₁-C₆ alkyl, or CH₂OR'; wherein each R' if present in R₁, and R₂ is independently H or C₁-C₆ alkyl;
   each of R₃, R₄, R₅, and R₆ is independently selected from the group consisting of H, C₁-C₆ alkyl, OH, OCH₃, OCH(CH₃)₂, OCH₂CH(CH₃)₂, OC(CH₃)₃, O(C₁-C₆ alkyl), OCF₃, OCH₂CH₂OH, O(C₁-C₆ alkyl)OH, O(C₁-C₆ haloalkyl), F, Cl, Br, I, CF₃, CN, NO₂, NH₂, C₁-C₆ haloalkyl, C₁-C₆ hydroxyalkyl, C₁-C₆ alkoxy C₁-C₆alkyl, aryl, heteroaryl, C₃-C₇ cycloalkyl, heterocycloalkyl, alkylaryl, CO₂R', C(O)R', NH(C₁-C₄ alkyl), N(C₁-C₄ alkyl)₂, NH(C₃-C₇ cycloalkyl), NHC(O)(C₁-C₄ alkyl), CONR'₂, NC(O)R', NS(O)ₙR', S(O)ₙNR'₂, S(O)ₙR', C(O)O(C₁-C₄ alkyl), OC(O)N(R')₂, C(O)(C₁-C₄ alkyl), and C(O)NH(C₁-C₄ alkyl), wherein each R' if present in R₃, R₄, R₅, and R₆ is independently selected from the group consisting of H, CH₃, CH₂CH₃, C₃-C₆ alkyl, C₁-C₆ haloalkyl, or optionally substituted aryl, alkylaryl, piperazin-1-yl, piperidin-1-yl, morpholinyl, heterocycloalkyl, heteroaryl, C₁-C₆ alkoxy, NH(C₁-C₄ alkyl), and N(C₁-C₄ alkyl)₂, wherein the optionally substituted group is selected from C₁-C₆ alkyl or C₂-C₇ acyl;
   or R₃ and R₄, together with the C atom to which they are attached form a 4-, 5-, 6- 7-or 8- membered cycloalkyl, aryl, heteroaryl, or heterocycloalkyl that is optionally substituted with 1, 2, 3, 4, or 5 substituents independently selected from OH, amino, halo, C₁₋C₆ alkyl, C₁-C₆ haloalkyl, C₁-C₆ alkoxy, C₁-C₆ haloalkoxy, aryl, arylalkyl, heteroaryl, heteroarylalkyl, cycloalkyl, and heterocycloalkyl or R₃ and R₄ are linked together to form a - O-C₁-C₂ methylene-O- group;
   or R₄ and R₅, together with the C atom to which they are attached form a 4-, 5-, 6- 7-or 8- membered cycloalkyl, aryl, heteroaryl, or heterocycloalkyl that is optionally substituted with 1, 2, 3, 4, or 5 substituents independently selected from OH, amino, halo, C₁₋C₆ alkyl, C₁-C₆ haloalkyl, C₁-C₆ alkoxy, C₁-C₆ haloalkoxy, aryl, arylalkyl, heteroaryl, heteroarylalkyl, cycloalkyl, and heterocycloalkyl or R₄ and R₅ are linked together to form a - O-C₁₋₂ methylene-O- group;
   each of R₇, R₈, R₉, R₁₀, and R₁₁ is independently selected from the group consisting of H, C₁-C₆ alkyl, OH, OCH₃, OCH(CH₃)₂, OCH₂CH(CH₃)₂, OC(CH₃)₃, O(C₁-C₆ alkyl), OCF₃, OCH₂CH₂OH, O(C₁-C₆ alkyl)OH, O(C₁-C₆ haloalkyl), O(CO)R', F, Cl, Br, I, CF₃, CN, NO₂, NH₂, C₁-C₆ haloalkyl, C₁-C₆ hydroxyalkyl, C₁-C₆ alkoxy C₁-C₆ alkyl, aryl, heteroaryl, C₃-C₇ cycloalkyl, heterocycloalkyl, alkylaryl, heteroaryl, CO₂R', C(O)R', NH(C₁-C₄ alkyl), N(C₁-C₄ alkyl)₂, NH(C₃-C₇ cycloalkyl), NHC(O)(C₁-C₄ alkyl), CONR'₂, NC(O)R', NS(O)ₙR', S(O)ₙNR'₂, S(O)ₙR', C(O)O(C₁-C₄ alkyl), OC(O)N(R')₂, C(O)(C₁-C₄ alkyl), and C(O)NH(C₁-C₄ alkyl); wherein each R' if present in R₇, R₈, R₉, R₁₀, and R₁₁ is independently selected from the group consisting of H, CH₃, CH₂CH₃, C₃-C₆ alkyl, C₁-C₆ haloalkyl, aryl, alkylaryl, piperazin-1-yl, piperidin-1-yl, morpholinyl, heterocycloalkyl, heteroaryl, C₁-C₆ alkoxy, NH(C₁-C₄ alkyl), and N(C₁-C₄ alkyl)₂;
   or R₇ and R₈, together with the N or C atoms to which they are attached form a 4-, 5-, 6- 7- or 8- membered cycloalkyl, aryl, heterocycloalkyl or heteroaryl group that is optionally substituted with 1, 2, 3, 4, or 5 substituents independently selected from OH, amino, halo, C₁-C₆ alkyl, C₁-C₆ haloalkyl, C₁-C₆ alkoxy, C₁-C₆ haloalkoxy, aryl, arylalkyl, heteroaryl, heteroarylalkyl, cycloalkyl, and heterocycloalkyl or R₇ and R₈ are linked together to form a -O-C₁₋₂ methylene-O- group;
   or R₈ and R₉, together with the N or C atoms to which they are attached form a 4-, 5-, 6- 7-or 8- membered cycloalkyl, aryl, heterocycloalkyl or heteroaryl group that is optionally substituted with 1, 2, 3, 4, or 5 substituents independently selected from OH, amino, halo, C₁-C₆ alkyl, C₁-C₆ haloalkyl, C₁-C₆ alkoxy, C₁-C₆ haloalkoxy, aryl, arylalkyl, heteroaryl, heteroarylalkyl, cycloalkyl, and heterocycloalkyl or R₈ and R₉ are linked together to form a -O-C₁₋₂ methylene-O- group;
   each n is independently 0, 1, or 2;
   with the proviso that R₇, R₈, R₉, R₁₀, and R₁₁ are not all H; and
   with the proviso that the following compounds, or pharmaceutically acceptable salts thereof are excluded: 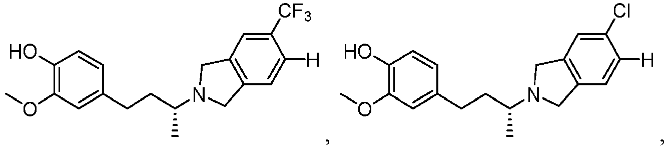 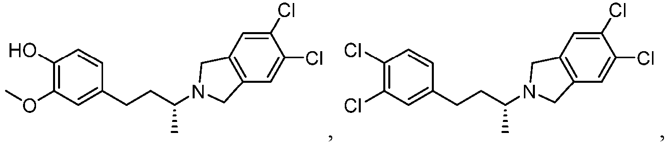 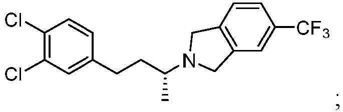 or
B. a compound of Formula IA 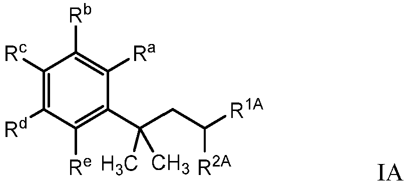 or pharmaceutically acceptable salt thereof:
   wherein:
   each of R^{a}, R^{b}, R^{c}, R^{d} and R^{e} is independently selected from the group consisting of, H, hydroxyl, Cl, F, methyl, -OCH₃, -OC(CH₃)₃, O-CH(CH₃)₂, CF₃, SO₂CH₃, and morpholino;
   R^{1A} is selected from the group consisting of hydrogen, alkyl, phenyl, or - CH=C(CH₃)₂; and
   R^{2A} is an optionally substituted cyclic amino group.

Some embodiments of the present disclosure are directed to a method of treating a neurologic disease, comprising administering to a subject in need thereof, a therapeutically effective amount of a compound selected from the group consisting of:

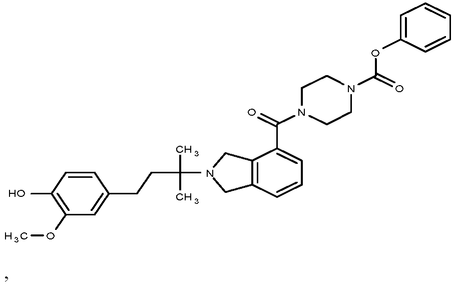

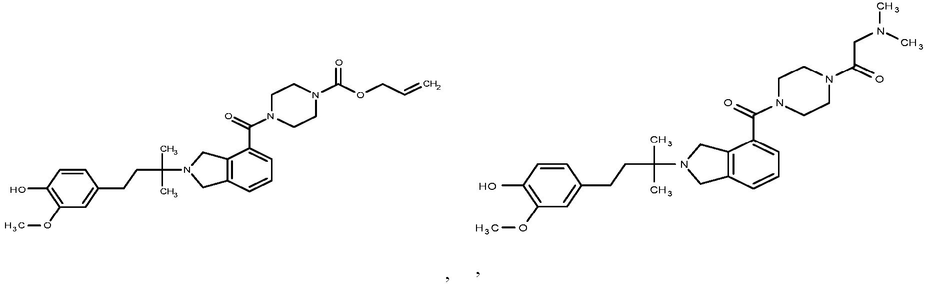

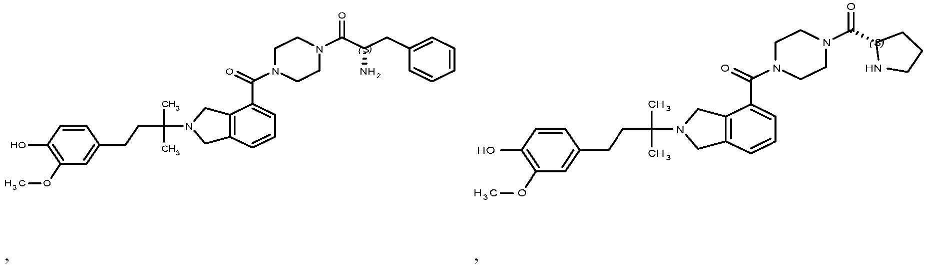

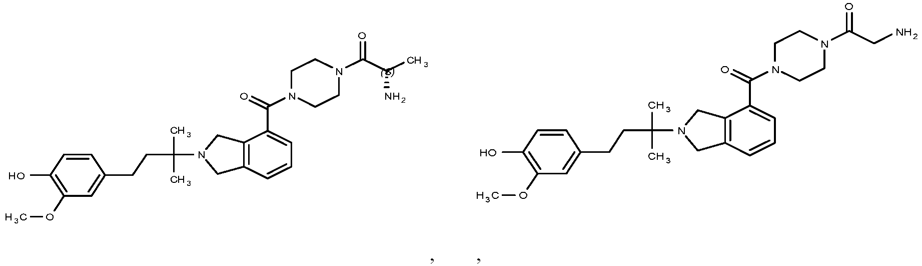

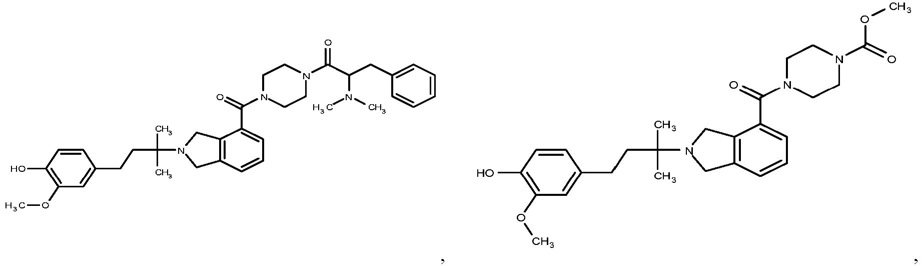

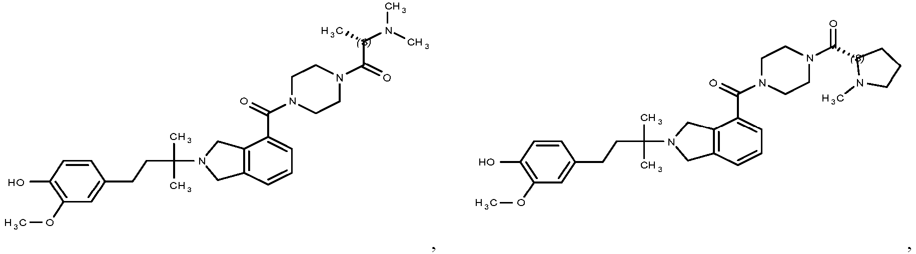

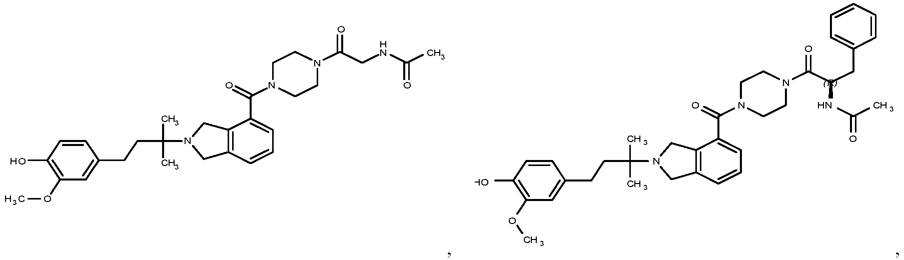

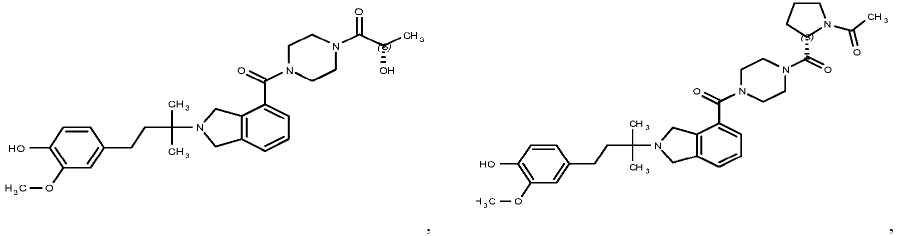

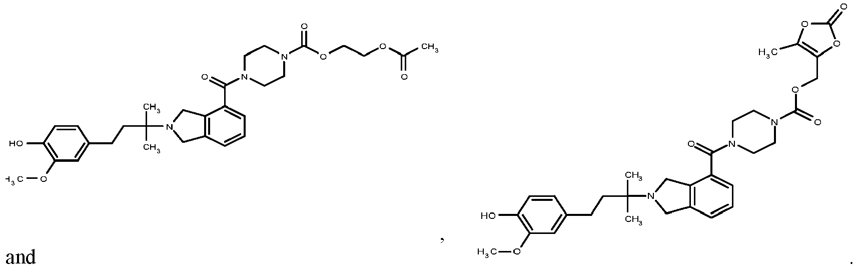

Some embodiments describe a method of treating a neurologic disease comprising administering to the subject a therapeutically effective amount of a pharmaceutical composition according to any embodiment described herein.

Some embodiments describe use of a compound according to any embodiment described herein, in the manufacture of a medicament for the treatment of a neurologic disease.

Some embodiments describe use of a composition comprising a compound according to any embodiment described herein, in the manufacture of a medicament for the treatment of a neurologic disease.

In some embodiments, the neurologic disease is selected from Age-Associated Memory Impairment (AAMI), Age-Related Cognitive Decline (ARCD), agitation synucleinopathies, Alzheimer's disease (AD), Amyotrophic lateral sclerosis (ALS) dementia, autosomal-dominant Parkinson's disease, chemotherapy-induced neuropathy (CIPN), Cognitive Impairment No Dementia (CIND), dementia, Creutzfeldt-Jakob disease (CJD), Diffuse Lewy Body Disease (DLBD) also known as Dementia with Lewy Bodies (DLB), disorders or conditions characterized by the presence of Lewy bodies, Down syndrome, dyskinesia, epilepsy, frontotemporal dementia (FTD), HIV-associated Neurocognitive Disorder (HAND), HIV dementia, Huntington's disease, Incidental LBD, Inherited LBD, Lewy body dysphagia, Mild Cognitive Impairment (MCI), multiple sclerosis, multiple system atrophy (MSA), Neuropathies (including but not limited to peripheral neuropathy, diabetic neuropathy and retinal neuropathy), Olivopontocerebellar Atrophy, Parkinson's disease (PD), preclinical Alzheimer's Disease (PCAD), psychiatric disorders (including but not limited to schizophrenia, bipolar disorders, depression, mania, anxiety disorders, post-traumatic stress disorders, delirium, eating disorders, autism, REM sleep behavior disorder, hallucinations, attention-deficit hyperactivity disorders, and psychosis), Pure Autonomic Failure, seizures, Shy-Drager Syndrome, Striatonigral Degeneration, synucleinopathies, traumatic brain injury (TBI), combined Alzheimer's and Parkinson disease and/or MSA, vascular dementia, diseases, disorders or conditions associated with abnormal expression, stability, activities and/or cellular processing of α-synuclein, diseases, disorders or conditions characterized by the presence of Lewy bodies, and combinations thereof.

### BRIEF DESCRIPTION OF THE DRAWINGS

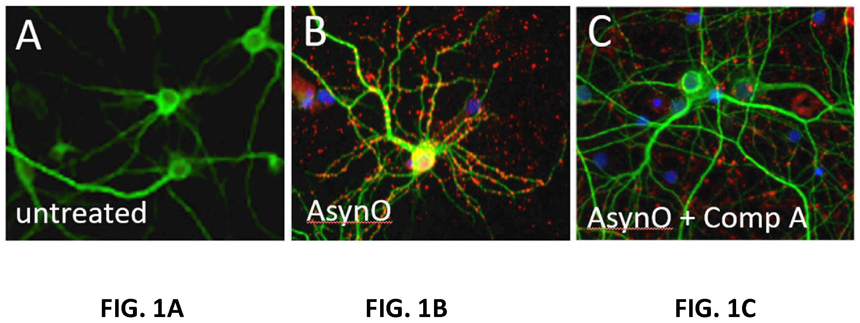

FIG. 1A is a representative image of a neuron culture with α seeding control (7.1nM) treated with a vehicle for 24 hours. Cultures were immunostained for the neurite marker microtubule associated protein 2 (MAP2; green) and for α-synuclein oligomers using an alpha-synuclein, oligomer-specific antibody (ASYO5 antibody, Agrisera; red). No α-synOs are present in the vehicle treated cultures.
FIG. 1B is a representative image of a neuron culture treated with synthetic α-synuclein oligomers (1µM) for 24 hours and immunostained for the neurite marker microtubule associated protein 2 (MAP2; green) and for α-synuclein oligomers using an alpha-synuclein, oligomer-specific antibody (ASYO5 antibody, Agrisera; red).
FIG. 1C is a representative image of a neuron culture treated with synthetic α-synuclein oligomers (1µM) and Compound A: 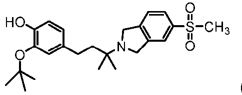 (10 µM) for 24 hours and immunostained for the neurite marker microtubule associated protein 2 (MAP2; green) and for α-synuclein oligomers using an alpha-synuclein, oligomer-specific antibody (ASYO5 antibody, Agrisera; red).

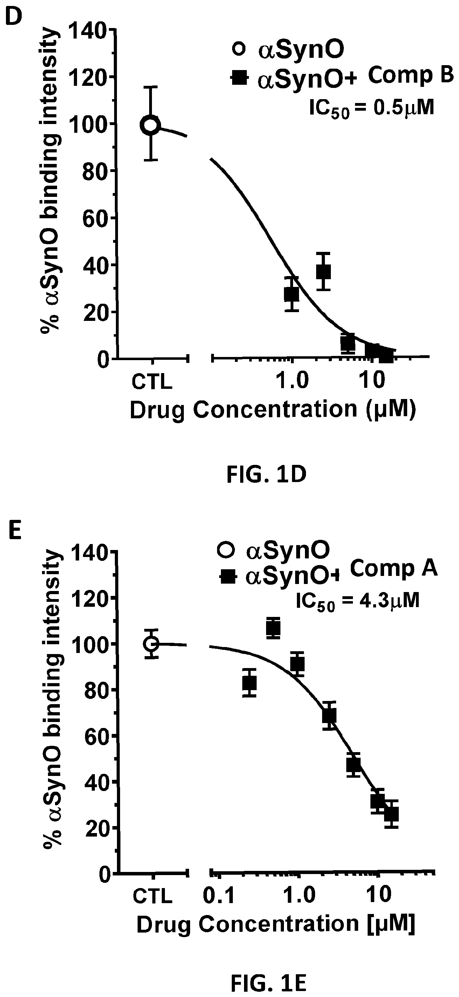

FIG. 1D is a graphical representation of binding affinity of α-synuclein oligomers (α-SynO) to neuronal synapses after treatment with vehicle (α-SynO) or treatment with Compound B

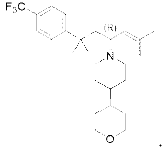

FIG. 1E is a graphical representation of binding affinity of α-synuclein oligomers (α-SynO) to neuronal synapses after treatment with vehicle (α-SynO) or treatment with Compound A 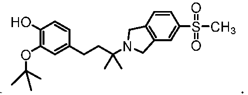

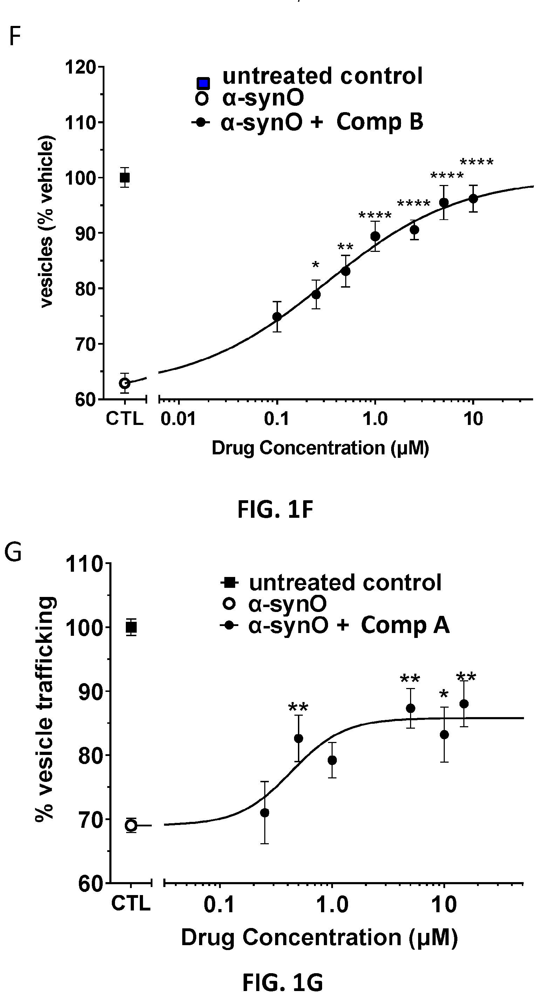

FIG. 1F is a graphical representation of vesicular trafficking in untreated control cultures, cultures treated with α-synuclein oligomers (α-SynO) for 24 hours, or cultures treated with α-synuclein oligomers (α-SynO) and Compound B 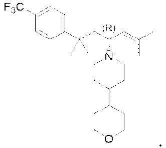
FIG. 1G is a graphical representation of vesicular trafficking in untreated control cultures, cultures treated with α-synuclein oligomers (α-SynO) for 24 hours, or cultures treated with α-synuclein oligomers (α-SynO) and Compound A

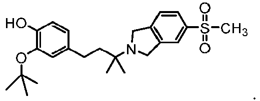

### DETAILED DESCRIPTION

This invention is not limited to the particular processes, compositions, or methodologies described, as these may vary. The terminology used in the description is for the purpose of describing the particular versions or embodiments only, and is not intended to limit the scope of the present invention. Unless defined otherwise, all technical and scientific terms used herein have the same meanings as commonly understood by one of ordinary skill in the art. All publications mentioned herein, are incorporated by reference in their entirety. Nothing herein is to be construed as an admission that the invention is not entitled to antedate such disclosure by virtue of prior invention.

### Definitions

Where a range of values is provided, it is intended that each intervening value between the upper and lower limit of that range and any other stated or intervening value in that stated range is encompassed within the disclosure. For example, if a range of 1 µm to 8 µm is stated, it is intended that 2 µm, 3 µm, 4 µm, 5 µm, 6 µm, and 7 µm are also explicitly disclosed.

At various places in the present specification, substituents of compounds of the disclosure are disclosed in groups or in ranges. It is specifically intended that embodiments of the disclosure include each and every individual subcombination of the members of such groups and ranges. For example, the term "C₁₋₆ alkyl" is specifically intended to individually disclose, e.g. methyl (C₁ alkyl), ethyl (C₂ alkyl), propyl (C₃ alkyl), butyl (C₄ alkyl), pentyl (C₅ alkyl), and hexyl (C₆ alkyl) as well as, *e.g.* C₁-C₂ alkyl, C₁-C₃ alkyl, C₁-C₄ alkyl, C₂-C₃ alkyl, C₂-C₄ alkyl, C₃-C₆ alkyl, C₄-C₅ alkyl, and C₅-C₆ alkyl.

The articles "a" and "an" as used herein, mean "one or more" or "at least one," unless otherwise indicated. That is, reference to any element of the present invention by the indefinite article "a" or "an" does not exclude the possibility that more than one of the element is present.

As used herein, the terms "Abeta" or "Aβ" shall include compositions comprising soluble amyloid peptide containing components such as Aβ monomers, Aβ oligomers, or complexes of Aβ peptide (in monomeric, dimeric, or polymeric form) with other soluble peptides or proteins as well as other soluble Aβ assemblies, including any processed product of amyloid precursor protein. Soluble Aβ oligomers are known to be neurotoxic. Even AP1-42 dimers are known to impair synaptic plasticity in mouse hippocampal slices. In one theory known in the art, native Aβ1-42 monomers are considered neuroprotective, self-association of Aβ monomers into soluble Aβ oligomers is required for neurotoxicity. However, certain Aβ mutant monomers (arctic mutations E22G) are reported to be associated with familial Alzheimer's Disease.

As used herein, the term "about" means plus or minus 10% of the numerical value of the number with which it is being used. Therefore, about 50 mL means in the range of 45 mL-55 mL.

Unless specifically indicated, the term "active ingredient" is to be understood as referring to a compound according to any embodiment describe herein.

"Administering," or "administration" and the like, when used in conjunction with the compounds of the disclosure refers to providing the compounds or pharmaceutical compositions according to any of the embodiments described herein, to a subject in need of treatment. Preferably the subject is a mammal, more preferably a human. The present invention comprises administering the pharmaceutical composition of the invention alone or in conjunction with another therapeutic agent. When a pharmaceutical composition of the invention is administered in conjunction with another therapeutic agent, the pharmaceutical composition of the invention and the other therapeutic agent. can be administered at the same time or different times.

The term "agonist" refers to a compound, the presence of which results in a biological activity of a receptor that is the same as the biological activity resulting from the presence of a naturally occurring ligand for the receptor.

The term "alkanoyl" or "alkylcarbonyl"as used herein, is meant to refer to an alkyl group attached to a carbonyl radical. An example of an alkanoyl is

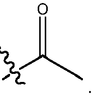

As used herein, the term "alkyl" is meant to refer to a saturated hydrocarbon group which is straight-chained or branched. Example alkyl groups include, but are not limited to, methyl (Me), ethyl (Et), propyl (*e.g.* n-propyl and isopropyl), butyl (*e.g. n-*butyl, isobutyl, t-butyl), pentyl (e.g. n-pentyl, isopentyl, neopentyl), and the like. An alkyl group can contain from 1 to about 20, from 2 to about 20, from 1 to about 10, from 1 to about 8, from 1 to about 6, from 1 to about 4, or from 1 to about 3 carbon atoms. "C₁-C₁₀ alkyl" or "C₁₋₁₀ alkyl", is intended to include C₁, C₂, C₃, C₄, C₅, C₆, C₇, C₈, C₉, and C₁₀ alkyl groups. Additionally, for example, "C₁-C₆ alkyl" or "C₁₋₆ alkyl" denotes alkyl having 1 to 6 carbon atoms. The term "alkylene" refers to a divalent alkyl linking group. An example of alkylene is methylene (CH₂).

As used herein, "alkenyl" is intended to include hydrocarbon chains of either straight or branched configuration with one or more, preferably one to three, carbon-carbon double bonds that may occur in any stable point along the chain. For example, "C₂-C₆ alkenyl" or "C₂₋₆ alkenyl" (or alkenylene), is intended to include C₂, C₃, C₄, C₅, and C₆ alkenyl groups. Examples of alkenyl include, but are not limited to, ethenyl, 1-propenyl, 2-propenyl, 2-butenyl, 3-butenyl, 2-pentenyl, 3-pentenyl, 4-pentenyl, 2-hexenyl, 3-hexenyl, 4-hexenyl, 5-hexenyl, 2-methyl-2-propenyl, and 4-methyl- 3 -pentenyl.

The term "alkoxy" or "alkyloxy" refers to an -O-alkyl group. "C₁-C₆ alkoxy" or "C₁₋₆ alkoxy" (or alkyloxy), is intended to include C₁, C₂, C₃, C₄, C₅, and C₆, alkoxy groups. Examples of alkoxy groups include, but are not limited to, methoxy, ethoxy, propoxy (e.g., n-propoxy and isopropoxy), and t-butoxy.

The term "alkoxyxlkoxy" refers to an alkoxy group attached to an alkoxy group. An example of an alkoxy group includes -O-(CH₂)₂-OCH₃.

As used herein, "alkynyl" is intended to include hydrocarbon chains of either straight or branched configuration having one or more, preferably one to three, carbon-carbon triple bonds that may occur in any stable point along the chain. For example, "C₂-C₆ alkynyl" is intended to include C₂, C₃, C₄, C₅, and C₆ alkynyl groups; such as ethynyl, propynyl, butynyl, pentynyl, and hexynyl.

As used herein, the terms "α-synuclein," "alpha-synuclein," or "αSyn" refers to protein that, in humans, is encoded by the SNCA gene. α-synuclein is a neuronal protein that regulates synaptic vesicle trafficking and subsequent neurotransmitter release. As used herein, the term "α-synuclein" shall include compositions comprising soluble α-synuclein peptide containing components such as α-synuclein monomers, α-synuclein oligomers, or complexes of α-synuclein peptide (in monomeric, dimeric, or polymeric form) with other soluble peptides or proteins as well as other soluble α-synuclein assemblies, including any processed product of α-synuclein. Soluble α-synuclein oligomers are known to be neurotoxic. α-synuclein oligomers are reported to be associated with familial Parkinson's disease and Dementia with Lewy Bodies (DLB).

The term "animal" as used herein, includes, but is not limited to, humans and non-human vertebrates such as wild, experimental, domestic and farm animals and pets.

The term "antagonist" refers to an entity, *e.g.* a compound, antibody or fragment, the presence of which results in a decrease in the magnitude of a biological activity of a receptor. In certain embodiments, the presence of an antagonist results in complete inhibition of a biological activity of a receptor. As used herein, the term "sigma-2 receptor antagonist" is used to describe a compound that acts as a "functional antagonist" at the sigma-2 receptor.

As used herein, "aryl" refers to monocyclic or polycyclic (e.g. having 2, 3 or 4 fused rings) aromatic hydrocarbons such as, for example, phenyl, naphthyl, anthracenyl, phenanthrenyl, indanyl, indenyl, and the like. In some embodiments, aryl groups have from 6 to about 20 carbon atoms. In some embodiments, aryl groups have from 5 to about 10 carbon atoms.

As used herein, "arylalkyl" refers to an aryl group attached to an alkyl radical. In preferred embodiments the alkyl is a C₁₋₆ alkyl.

The term "aroyl" or "arylcarbonyl" as used herein, refers to an aryl group attached to a carbonyl radical. Examples of aroyl include but are not limited to benzoyl.

As used herein the term "brain penetrability" refers to the ability of a drug, antibody or fragment, to cross the blood-brain barrier. In some embodiments, an animal pharmacokinetic (pK) study, for example, a mouse pharmacokinetic/blood-brain barrier study can be used to determine or predict brain penetrability. In some embodiments various concentrations of a compound or pharmaceutical composition according to any embodiment described herein, can be administered, for example at 3, 10 and 30 mg/kg, for example p.o. for 5 days and various pK properties are measured, e.g., in an animal model. In some embodiments, dose related plasma and brain levels are determined. In some embodiments, brain Cmax > 100, 300, 600, 1000, 1300, 1600, or 1900 ng/mL. In some embodiments good brain penetrability is defined as a brain/plasma ratio of > 0.1, > 0.3, > 0.5, > 0.7, > 0.8 , >0.9, preferably >1, and more preferably > 2, >5, or > 10. In other embodiments, good brain penetrability is defined as greater than about 0.1%, 1%, 5%, greater than about 10%, and preferably greater than about 15% of an administered dose crossing the BBB after a predetermined period of time. In certain embodiments, the dose is administered orally (p.o.). In other embodiments, the dose is administered intravenously (i.v.), prior to measuring pK properties.

As used herein, the term "contacting" refers to the bringing together or combining of molecules (or of a molecule with a higher order structure such as a cell or cell membrane) such that they are within a distance that allows for intermolecular interactions such as the non-covalent interaction between two peptides or one protein and another protein or other molecule, such as a small molecule. In some embodiments, contacting occurs in a solution in which the combined or contacted molecules are mixed in a common solvent and are allowed to freely associate. In some embodiments, the contacting can occur at or otherwise within a cell or in a cell-free environment. In some embodiments, the cell-free environment is the lysate produced from a cell. In some embodiments, a cell lysate may be a whole-cell lysate, nuclear lysate, cytoplasm lysate, and combinations thereof. In some embodiments, the cell-free lysate is lysate obtained from a nuclear extraction and isolation wherein the nuclei of a cell population are removed from the cells and then lysed. In some embodiments, the nuclei are not lysed, but are still considered to be a cell-free environment. The molecules can be brought together by mixing such as vortexing, shaking, and the like.

The term "cyclic amino" or "cyclic amino group" as used herein, is a heterocycloalkyl or heteroaryl group containing a nitrogen radical, thus allowing bonding through the nitrogen atom. The group can be represented by the formula: 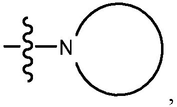 wherein 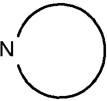 is any heterocyclic or heteroaromatic ring containing 0-3 additional heteroatoms selected from nitrogen, sulfur and oxygen.

The term "cycloalkanoyl" or "cycloalkylcarbonyl" as used herein, is meant to describe a cycloalkyl group attached to a carbonyl radical. Examples of cycloalkanoyl include but are not limited to,

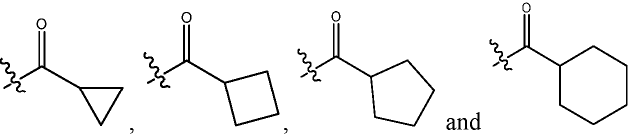

As used herein, "cycloalkyl" refers to non-aromatic cyclic hydrocarbons including cyclized alkyl, alkenyl, and alkynyl groups that contain up to 20 ring-forming carbon atoms. Cycloalkyl groups can include mono- or polycyclic (e.g. having 2, 3 or 4 fused rings) ring systems as well as spiro ring systems. A cycloalkyl group can contain from 3 to about 15, from 3 to about 10, from 3 to about 8, from 3 to about 6, from 4 to about 6, from 3 to about 5, or from 5 to about 6 ring-forming carbon atoms. Ring-forming carbon atoms of a cycloalkyl group can be optionally substituted by oxo or sulfido. Example of cycloalkyl groups include, but are not limited to, cyclopropyl, cyclobutyl, cyclopentyl, cyclohexyl, cycloheptyl, cyclopentenyl, cyclohexenyl, cyclohexadienyl, cycloheptatrienyl, norbornyl, norpinyl, norcarnyl, adamantyl, and the like. Also included in the definition of cycloalkyl are moieties that have one or more aromatic rings fused (*i.e.* having a bond in common with) to the cycloalkyl ring, for example, benzo or thienyl derivatives of cyclopentane, cyclopentene, cyclohexane, and the like (*e.g*. 2,3-dihydro-1H-indene-1-yl, or 1H-inden-2(3H)-one-1-yl). Preferably, "cycloalkyl" refers to cyclized alkyl groups that contain up to 20 ring-forming carbon atoms. Examples of cycloalkyl preferably include cyclopropyl, cyclobutyl, cyclopentyl, cyclohexyl, cycloheptyl, adamantyl, and the like

The term "cycloalkylalkyl" refers to a cycloalkyl group attached to an alkyl radical. In preferred embodiments the alkyl is a C₁₋₆ alkyl.

The term "drug-like properties" is used herein, to describe the pharmacokinetic and stability characteristics of a compound upon administration; including brain penetrability, metabolic stability and/or plasma stability.

As used herein, "halo" or "halogen" includes fluorine, chlorine, bromine, and iodine.

As used herein, "haloalkoxy" represents a haloalkyl group as defined herein, with the indicated number of carbon atoms, attached through an oxygen bridge. For example, "C₁-C₆ haloalkoxy" or "C₁₋₆ haloalkoxy", is intended to include C₁, C₂, C₃, C₄, C₅, and C₆ haloalkoxy groups. An example haloalkoxy group is OCF₃. As used herein, "trihalomethoxy" refers to a methoxy group having three halogen substituents. Examples of trihalomethoxy groups include, but are not limited to, -OCF₃, -OCClF₂, -OCCl₃, and the like.

As used herein, "haloalkyl" is intended to include both branched and straight-chain saturated aliphatic hydrocarbon groups having the specified number of carbon atoms, substituted with one or more halogens. Example haloalkyl groups include, but are not limited to, CF₃, C₂F₅, CHF₂, CCl₃, CHCl₂, C₂Cl₅, CH₂CF₃, and the like.

As used herein, "heteroaryl" groups refer to an aromatic heterocycle having up to 20 ring-forming atoms and having at least one heteroatom ring member (ring-forming atom) such as sulfur, oxygen, or nitrogen. In some embodiments, the heteroaryl group has at least one or more heteroatom ring-forming atoms each independently selected from sulfur, oxygen, and nitrogen. Heteroaryl groups include monocyclic and polycyclic (e.g. having 2, 3 or 4 fused rings) systems. Examples of heteroaryl groups include without limitation, pyridyl (a.k.a. pyridinyl), pyrimidinyl, pyrazinyl, pyridazinyl, triazinyl, furyl, quinolyl, isoquinolyl, thienyl, imidazolyl, thiazolyl, indolyl, pyrryl (a.k.a.pyrrolyl), oxazolyl, benzofuryl, benzothienyl, benzthiazolyl, isoxazolyl, pyrazolyl, triazolyl, tetrazolyl, indazolyl, 1,2,4-thiadiazolyl, isothiazolyl, benzothienyl, purinyl, carbazolyl, benzimidazolyl, indolinyl, and the like. In some embodiments, the heteroaryl group has from 1 to about 20 carbon atoms, and in further embodiments from about 1 to about 5, from about 1 to about 4, from about 1 to about 3, from about 1 to about 2, carbon atoms as ring-forming atoms. In some embodiments, the heteroaryl group contains 3 to about 14, 3 to about 7, or 5 to 6 ring-forming atoms. In some embodiments, the heteroaryl group has 1 to about 4, 1 to about 3, or 1 to 2 heteroatoms.

The term "heterocycloalkoxy" as used herein, refers to an -O-heterocycloalkyl group. An example of a heterocycloalkoxy group is

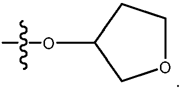

As used herein, "heterocycloalkyl" or "heterocyclyl" refers to a non-aromatic heterocyclyl group having up to 20 ring-forming atoms including cyclized alkyl, alkenyl, and alkynyl groups where one or more of the ring-forming carbon atoms is replaced by a heteroatom such as an O, N, or S atom. Heterocycloalkyl groups can be mono or polycyclic (e.g. both fused and spiro systems). For example, "heterocycloalkyl" groups include morpholino, thiomorpholino, piperazinyl, tetrahydrofuranyl, tetrahydrothienyl, 2,3-dihydrobenzofuryl, 1,3-benzodioxole, benzo-1,4-dioxane, piperidinyl, pyrrolidinyl, isoxazolidinyl, isothiazolidinyl, pyrazolidinyl, oxazolidinyl, thiazolidinyl, imidazolidinyl, pyrrolidin-2-one-3-yl, and the like. Ring-forming carbon atoms and heteroatoms of a heterocycloalkyl group can be optionally substituted by oxo or sulfido. For example, a ring-forming S atom can be substituted by 1 or 2 oxo (*i.e.* form a S(O) or S(O)₂). For example, a ring-forming C atom can be substituted by oxo (*i.e.* form carbonyl). Also included in the definition of heterocycloalkyl are moieties that have one or more aromatic rings fused (*i.e.* having a bond in common with) to the nonaromatic heterocyclic ring, for example pyridinyl, thiophenyl, phthalimidyl, naphthalimidyl, and benzo derivatives of heterocycles such as indoline, isoindoline, isoindolin-1-one-3-yl, 4,5,6,7-tetrahydrothieno[2,3-c]pyridine-5-yl, 5,6-dihydrothieno[2,3-c]pyridin-7(4H)-one-5-yl, and 3,4-dihydroisoquinolin-1(2H)-one-3yl groups. Ring-forming carbon atoms and heteroatoms of the heterocycloalkyl group can be optionally substituted by oxo or sulfido. In some embodiments, the heterocycloalkyl group has from 2 to about 20 carbon atoms or 3 to 20 carbon atoms. In some embodiments, the heterocycloalkyl group contains 3 to about 14, 3 to about 7, or 5 to 6 ring-forming atoms. In some embodiments, the heterocycloalkyl group has 1 to 4 heteroatoms. In some embodiments, the heterocycloalkyl group contains 0 to 3 double bonds. In some embodiments, the heterocycloalkyl group contains 0 to 2 triple bonds.

The terms "hydroxyl" and "hydroxy" are used interchangeably to mean an OH group.

The term "improves" is used to convey that the disclosure changes either the characteristics and/or the physical attributes of the tissue to which it is being provided, applied or administered. The term "improves" may also be used in conjunction with a disease state such that when a disease state is "improved" the symptoms or physical characteristics associated with the disease state. are diminished, reduced, eliminated, delayed or averted.

The term "inhibiting" includes the blockade, aversion of a certain result or process, or the restoration of the converse result or process. In terms of prophylaxis or treatment by administration of a compound of the disclosure, "inhibiting" includes protecting against (partially or wholly) or delaying the onset of symptoms, alleviating symptoms, or protecting against, diminishing or eliminating a disease, condition or disorder.

As used herein the term "metabolic stability" refers to the ability of a compound to survive first-pass metabolism (intestinal and hepatic degradation or conjugation of a drug administered orally). This can be assessed, for example, *in vitro* by exposure of the compounds to mouse or human hepatic microsomes. In some embodiments, good metabolic stability refers to a t_{1/2} > 5 min, > 10 min, > 15 minutes, > 20 minutes, and preferably > 30 min upon exposure of a compound to mouse or human hepatic microsomes. In some embodiments, good metabolic stability refers to an Intrinsic Clearance Rate (Clᵢₙₜ) of < 300 uL/min/mg, preferably < 200 uL/min/mg, and more preferably ≤ 100 uL/min/mg.

The term "n-membered" where n is an integer typically describes the number of ring-forming atoms in a moiety where the number of ring-forming atoms is n. For example, pyridine is an example of a 6-membered heteroaryl ring and thiophene is an example of a 5-membered heteroaryl group.

As used herein, the term "a neuronal cell" can be used to refer to a single cell or to a population of cells. In some embodiments, the neuronal cell is a primary neuronal cell. In some embodiments, the neuronal cell is an immortalized or transformed neuronal cell or a stem cell. A primary neuronal cell is a neuronal cell that cannot differentiate into other types of neuronal cells, such as glia cells. A stem cell is one that can differentiate into neurons and other types of neuronal cells such as glia. In some embodiments, assays utilize a composition comprising at least one neuronal cell is free of glia cells. In some embodiments, the composition comprises less than about 30%, 25%, 20%, 15%, 10%, 5%, or 1% of glia cells, which are known to internalize and accumulate Abeta. The primary neuronal cell can be derived from any area of the brain of an animal. In some embodiments, the neuronal cell is a hippocampal or cortical cell. The presence of glia cells can be determined by any method. In some embodiments, glia cells are detected by the presence of GFAP and neurons can be detected by staining positively with antibodies directed against MAP2.

As used herein, the term "optionally substituted" means that substitution is optional and therefore includes both unsubstituted and substituted atoms and moieties. A "substituted" atom or moiety indicates that any hydrogen on the designated atom or moiety can be replaced with a selection from the indicated substituent group, provided that the normal valence of the designated atom or moiety is not exceeded, and that the substitution results in a stable compound. For example, if a methyl group (*i.e.* CH₃) is optionally substituted, then up to 3 hydrogen atoms on the carbon atom can be replaced with substituent groups. Substituent groups include, but are not limited to, alkanoyl, alkoxy, alkoxyalkyl, (alkoxy)alkoxyalkyl, alkoxycarbonyl, alkyl, aryloxy, aryloyl, cycloalkanoyl, substituted or unsubstituted C₃-C₁₀ cycloalkyl, -OC(O)NCH(CH₃)₂, (N,N-dimethylamino)pyridinyl, (N,N-dimethylamino)sulfonyl, halo, heterocyclyl, (heterocyclyl)alkoxyalkyl, heterocycloalkyl, hydroxyl, hydroxyalkyl, methylpiperidinyl, methylsulfonyl, methylsulfonylphenyl, morpholinylpyridinyl, optionally substituted C₁-C₁₀ alkyl, optionally substituted C₅-C₁₀ aryl, optionally substituted C₃-C₁₀ heteroaryl, perfluoroalkyl, phenyl, piperidinyl, pyrrolidinylpyridinyl, tetrahydropyranyl, CF₃. A substituted alkyl group for example indicates that one or more hydrogen atoms on the alkyl group is replaced with a substituent group, selected from but not limited to, halo, hydroxyl, alkoxy, heterocycloalkoxy, alkoxyalkoxy, C(O)OMe, and C(O)OEt. A substituted aryl group for example, indicates that one or more hydrogen atoms on the aryl group is replaced with a substituent group, selected from but not limited to, -SO₂Me or phenyl group. A substituted heteroaryl group for example, indicates that one or more hydrogen atoms on the heteroaryl group is replaced with a substituent group, selected from, but not limited to, heterocycloalkyl, heteroaryl, N,N-dimethylamino. A substituted heterocycloalkyl group for example, indicates that one or more hydrogen atoms on the heterocycloalkyl group is replaced with a substituent group, selected from, but not limited to, heterocyclalkyl, heteroaryl, N,N-dimethylamino, hydroxyl, alkoxy, alkoxycarbonyl, alkyl, aryl, sulfonyl, dimethylaminosulfonyl, aroyl, cycloalkanoyl, alkanoyl and -OC(O)NCH(CH₃)₂. In some instances two hydrogen atoms on the same carbon of, for example, a heterocyclyl or alkyl group are replaced with a group to form a spiro compound selected from but not limited to, for example,

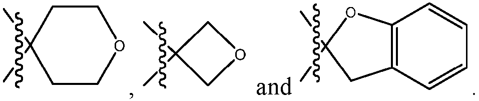

The term "partial agonist" refers to a compound the presence of which results in a biological activity of a receptor that is of the same type as that resulting from the presence of a naturally occurring ligand for the receptor, but of a lower magnitude.

The phrase "pharmaceutically acceptable" refers to molecular entities and compositions that are generally regarded as safe and nontoxic. In particular, pharmaceutically acceptable carriers, diluents or other excipients used in the pharmaceutical compositions of this disclosure are physiologically tolerable, compatible with other ingredients, and do not typically produce an allergic or similar untoward reaction (for example, gastric upset, dizziness and the like) when administered to a patient. Preferably, as used herein, the term "pharmaceutically acceptable" means approved by a regulatory agency of the Federal or a state government or listed in the U.S. Pharmacopoeia or other generally recognized pharmacopoeia for use in animals, and more particularly in humans.

The phrase "pharmaceutically acceptable salt(s)", as used herein, includes those salts of compounds of the disclosure that are safe and effective for use in mammals and that possess the desired biological activity. Pharmaceutically acceptable salts include salts of acidic or basic groups present in compounds of the disclosure or in compounds identified pursuant to the methods of the disclosure. Pharmaceutically acceptable acid addition salts include, but are not limited to, hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate and pamoate (i.e., 1,1'-methylene-bis-(2-hydroxy-3-naphthoate)) salts. Certain compounds of the disclosure can form pharmaceutically acceptable salts with various amino acids. Suitable base salts include, but are not limited to, aluminum, calcium, lithium, magnesium, potassium, sodium, zinc, iron and diethanolamine salts. Pharmaceutically acceptable base addition salts are also formed with amines, such as organic amines. Examples of suitable amines are N,N'-dibenzylethylenediamine, chloroprocaine, choline, diethanolamine, dicyclohexylamine, ethylenediamine, N-methylglucamine, and procaine.

As used herein, the term "pharmaceutically acceptable carrier" includes any of the standard pharmaceutical carriers, such as a phosphate buffered saline solution, water, emulsions such as an oil/water or water/oil emulsion, and various types of wetting agents. The term also encompasses any of the agents approved by a regulatory agency of the US Federal government or listed in the US Pharmacopeia for use in animals, including humans.

The term "selectivity" or "selective" refers to a difference in the binding affinity of a compound (Kᵢ) for a sigma receptor, for example, a sigma-2 receptor, compared to a non-sigma receptor. The compound possess high selectivity for a sigma receptor in synaptic neurons. The Kᵢ for a sigma-2 receptor or both a sigma-2 and a sigma-1 receptor is compared to the Kᵢ for a non-sigma receptor. In some embodiments, the compound is a selective sigma-2 receptor antagonist, or sigma-1 receptor ligand, and has at least 10-fold, 20-fold, 30-fold, 50-fold, 70-fold, 100-fold, or 500-fold higher affinity, or more, for binding to a sigma receptor compared to a non-sigma receptor as assessed by a comparison of binding dissociation constant Kᵢ values, or IC₅₀ values, or binding constant, at different receptors. Any known assay protocol can be used to assess the Kᵢ or IC₅₀ values at different receptors, for example, by monitoring the competitive displacement from receptors of a radiolabeled compound with a known dissociation constant, for example, by the method of Cheng and Prusoff (1973) (Biochem. Pharmacol. 22, 3099-3108), or specifically as provided herein.

As used herein the term "plasma stability" refers to the degradation of compounds in plasma, for example, by enzymes such as hydrolases and esterases. Any of a variety of *in vitro* assays can be employed. Compounds are incubated in plasma over various time periods. The percent parent compound (analyte) remaining at each time point reflects plasma stability. Poor stability characteristics can tend to have low bioavailability. Good plasma stability can be defined as greater than 50% analyte remaining after 30 min, greater than 50% analyte remaining after 45 minutes, and preferably greater than 50% analyte remaining after 60 minutes.

"Sigma-2 ligand" refers to a compound that binds to a sigma-2 receptor and includes agonists, antagonists, partial agonists, inverse agonists and simply competitors for other ligands of this receptor or protein.

The term "sigma-2 receptor antagonist compound" refers to a compound that binds to a sigma-2 receptor in a measurable amount and acts as a functional antagonist with respect to Abeta effects oligomer induced synaptic dysfunction resultant from sigma-2 receptor binding.

The terms "subject," "individual" or "patient" are used interchangeably and as used herein, are intended to include human and non-human animals. Non-human animals includes all vertebrates, e.g. mammals and non-mammals, such as non-human primates, sheep, dogs, cats, cows, horses, chickens, amphibians, and reptiles, although mammals are preferred, such as non-human primates, sheep, dogs, cats, cows and horses. Preferred subjects include human patients. The methods are particularly suitable for treating human patients having a disease or disorder described herein.

As used herein, the term "therapeutic" means an agent utilized to treat, combat, ameliorate, protect against or improve an unwanted condition or disease of a subject.

A "therapeutically effective amount" of a compound, pharmaceutically acceptable salt thereof or pharmaceutical composition according to any embodiment described herein, is an amount sufficient to produce a selected effect on at least one symptom or parameter of a specific disease or disorder or pathological process. The therapeutic effect may be objective (i.e., measurable by some test or marker) or subjective (i.e., subject gives an indication of or feels an effect or physician observes a change). A therapeutically effective amount of a compound, according to any embodiment described herein, may broadly range from 0.01 mg/kg to about 500 mg/kg, about 0.01 to about 250 mg/kg, about 0.01 to about 25 mg/kg, about 0.05 mg/kg to about 20 mg/kg, about 0.1 mg/kg to about 400 mg/kg, about 0.1 mg/kg to about 200 mg/kg, about 0.1 mg/kg to about 25 mg/kg, about 0.1 to about 10 mg/kg, about 0.2 to about 5 mg/kg, about 1 mg/kg to about 300 mg/kg, about 10 mg/kg to about 100 mg/kg, body weight. The effect contemplated herein, includes both medical therapeutic and/or prophylactic treatment, as appropriate. The specific dose of a compound administered according to this disclosure to obtain therapeutic and/or prophylactic effects is determined by the particular circumstances surrounding the case, including, for example, the compound administered, the route of administration, the co-administration of other active ingredients, the condition being treated, the activity of the specific compound employed, the specific composition employed, the age, body weight, general health, sex and diet of the patient; the time of administration, route of administration, and rate of excretion of the specific compound employed and the duration of the treatment;. The therapeutically effective amount administered will be determined by the physician in the light of the foregoing relevant circumstances and the exercise of sound medical judgment. A therapeutically effective amount of a compound, according to any embodiment described herein, is typically an amount such that when it is administered in a physiologically tolerable excipient composition, it is sufficient to achieve an effective systemic concentration or local concentration in the tissue. The total daily dose of the compounds according to any embodiment described herein administered to a human or other animal in single or in divided doses can be in amounts, for example, from about 0.01 mg/kg to about 500 mg/kg, about 0.01 to about 250 mg/kg, about 0.01 to about 25 mg/kg, about 0.05 mg/kg to about 20 mg/kg, about 0.1 mg/kg to about 400 mg/kg, about 0.1 mg/kg to about 200 mg/kg, about 0.1 mg/kg to about 25 mg/kg, about 0.1 to about 10 mg/kg, about 0.2 to about 5 mg/kg, about 1 mg/kg to about 300 mg/kg, about 10 mg/kg to about 100 mg/kg, body weight per day. Single dose pharmaceutical compositions of any embodiment described herein, may contain such amounts or submultiples thereof to make up the daily dose. For example, the compounds according to any embodiment described herein, may be administered on a regimen of 1 to 4 times per day, such as once, twice, three times or four times per day. In some embodiments, the therapeutically effective amount of a compound according to any embodiment disclosed herein, can range between about 0.01 and about 25 mg/kg/day. In some embodiments the therapeutically effective amount is between a lower limit of about 0.01 mg/kg of body weight, about 0.1 mg/kg of body weight, about 0.2 mg/kg of body weight, about 0.3 mg/kg of body weight, about 0.4 mg/kg of body weight, about 0.5 mg/kg of body weight, about 0.60 mg/kg of body weight, about 0.70 mg/kg of body weight, about 0.80 mg/kg of body weight, about 0.90 mg/kg of body weight, about 1 mg/kg of body weight, about 2.5 mg/kg of body weight, about 5 mg/kg of body weight, about 7.5 mg/kg of body weight, about 10 mg/kg of body weight, about 12.5 mg/kg of body weight, about 15 mg/kg of body weight, about 17.5 mg/kg of body weight, about 20 mg/kg of body weight, about 22.5 mg/kg of body weight, and about 25 mg/kg of body weight; and an upper limit of 25 mg/kg of body weight, about 22.5 mg/kg of body weight, about 20 mg/kg of body weight, about 17.5 mg/kg of body weight, about 15 mg/kg of body weight, about 12.5 mg/kg of body weight, about 10 mg/kg of body weight, about 7.5 mg/kg of body weight, about 5 mg/kg of body weight, about 2.5 mg/kg of body weight, about 1 mg/kg of body weight, about 0.9 mg/kg of body weight, about 0.8 mg/kg of body weight, about 0.7 mg/kg of body weight, about 0.6 mg/kg of body weight, about 0.5 mg/kg of body weight, about 0.4 mg/kg of body weight, about 0.3 mg/kg of body weight, about 0.2 mg/kg of body weight, about 0.1 mg/kg of body weight, and about 0.01 mg/kg of body weight. In some embodiments, the therapeutically effective amount is about 0.1 mg/kg/day to about 10 mg/kg/day; in some embodiments the therapeutically effective amount is about 0.2 and about 5 mg/kg/day. In some embodiments, treatment regimens according to the disclosure comprise administration to a patient in need of such treatment will usually include from about 1 mg to about 5000 mg, about 10 mg to about 2000 mg, about 10 mg to about 200 mg, about 20 to about 1000 mg, about 20 to about 500 mg, about 20 to about 400 mg, about 40 to about 800 mg, about 50 mg to about 500 mg, about 80 to about 1600 mg and about 50 mg, of a compound according to any embodiment disclosed herein, or a pharmaceutically acceptable salt thereof, per day in single or multiple doses. In some embodiments the therapeutically effective amount is a total daily dose of 50 mg to 500 mg. In some embodiments, the daily dose is between a lower limit of about 50 mg, about 55 mg, about 60 mg, about 65 mg, about 70 mg, about 75 mg, about 80 mg, about 85 mg, about 90 mg, about 95 mg, about 100 mg, about 105 mg, about 110 mg, about 115 mg; about 120 mg, about 125 mg, about 130 mg, about 135 mg, about 140 mg, about 145 mg, about 150 mg, about 155 mg, about 160 mg, about 165 mg, about 170 mg, about 175 mg, about 180 mg, about 185 mg, about 190 mg, about 195 mg, about 200 mg, about 205 mg, about 210 mg, about 215 mg; about 220 mg, about 225 mg, about 230 mg, about 235 mg, about 240 mg, about 245 mg, about 250 mg, about 255 mg, about 260 mg, about 265 mg, about 270 mg, about 275 mg, about 280 mg, about 285 mg, about 290 mg, about 295 mg, 300 mg, about 305 mg, about 310 mg, about 315 mg; about 320 mg, about 325 mg, about 330 mg, about 335 mg, about 340 mg, about 345 mg, about 350 mg, about 355 mg, about 360 mg, about 365 mg, about 370 mg, about 375 mg, about 380 mg, about 385 mg, about 390 mg, about 395, about 400 mg, about 405 mg, about 410 mg, about 415 mg; about 420 mg, about 425 mg, about 430 mg, about 435 mg, about 440 mg, about 445 mg, about 450 mg, about 455 mg, about 460 mg, about 465 mg, about 470 mg, about 475 mg, about 480 mg, about 485 mg, about 490 mg, about 495 mg, and about 500 mg and an upper limit of about 500 mg, about 495 mg, about 490 mg, about 485 mg, about 480 mg, about 475 mg, about 470 mg, about 465 mg, about 460 mg, about 455 mg, about 450 mg, about 445 mg, about 440 mg, about 435 mg, about 430 mg, about 425 mg, about 420 mg, about 415 mg, about 410 mg, about 405 mg, about 400 mg, about 395 mg, about 390 mg, about 385 mg, about 380 mg, about 375 mg, about 370 mg, about 365 mg, about 360 mg, about 355 mg, about 350 mg, about 345 mg, about 340 mg, about 335 mg, about 330 mg, about 325 mg, about 320 mg, about 315 mg, about 310 mg, about 305 mg about 300 mg, about 295 mg, about 290 mg, about 285 mg, about 280 mg, about 275 mg, about 270 mg, about 265 mg, about 260 mg, about 255 mg, about 250 mg, about 245 mg, about 240 mg, about 235 mg, about 230 mg, about 225 mg, about 220 mg, about 215 mg, about 210 mg, about 205 mg 200 mg, about 195 mg, about 190 mg, about 185 mg, about 180 mg, about 175 mg, about 170 mg, about 165 mg, about 160 mg, about 155 mg, about 150 mg, about 145 mg, about 140 mg, about 135 mg, about 130 mg, about 125 mg, about 120 mg, about 115 mg, about 110 mg, about 105 mg, about 100 mg, about 95 mg, about 90 mg; about 85 mg, about 80 mg, about 75 mg, about 70 mg, about 65 mg, about 60 mg, about 55 mg, and about 50 mg of a compound according to any embodiment herein. In some embodiments, the total daily dose is about 50 mg to 150 mg. In some embodiments, the total daily dose is about 50 mg to 250 mg. In some embodiments, the total daily dose is about 50 mg to 350 mg. In some embodiments, the total daily dose is about 50 mg to 450 mg. In some embodiments, the total daily dose is about 50 mg. It will be understood that the pharmaceutical formulations of the disclosure need not necessarily contain the entire amount of the compound that is effective in treating the disorder, as such effective amounts can be reached by administration of a plurality of divided doses of such pharmaceutical formulations. The compounds may be administered on a regimen of 1 to 4 times per day, such as once, twice, three times or four times per day.

The term "tissue" refers to any aggregation of similarly specialized cells which are united in the performance of a particular function.

The terms "treat," "treated," or "treating" as used herein, refers to both therapeutic treatment and prophylactic or preventative measures, wherein the object is to protect against (partially or wholly) or slow down (e.g., lessen or postpone the onset of) an undesired physiological condition, disorder or disease, or to obtain beneficial or desired clinical results such as partial or total restoration or inhibition in decline of a parameter, value, function or result that had or would become abnormal. For the purposes of this disclosure, beneficial or desired clinical results include, but are not limited to, alleviation of symptoms; diminishment of the extent or vigor or rate of development of the condition, disorder or disease; stabilization (i.e., not worsening) of the state of the condition, disorder or disease; delay in onset or slowing of the progression of the condition, disorder or disease; amelioration of the condition, disorder or disease state; and remission (whether partial or total), whether or not it translates to immediate lessening of actual clinical symptoms, or enhancement or improvement of the condition, disorder or disease. Treatment seeks to elicit a clinically significant response without excessive levels of side effects. Treatment also includes prolonging survival as compared to expected survival if not receiving treatment. Five million people are afflicted with Parkinson's disease worldwide. Symptoms of Parkinson's disease are debilitating and adversely affect patient's quality of life, yet no disease modifying therapies exist to treat Parkinson's disease; thus there is a huge unmet need to discovery and develop promising disease modifying therapeutics for Parkinson's disease. Sigma-2 modulators prevent alpha-Synuclein oligomer-induced neuronal dysfunction.

Alpha-synuclein (α-syn) pathology, lewy bodies, comprised of α-syn aggregates, was very early on identified as a chief hallmark of Parkinson's disease. Since then, human genetic evidence has mounted indicating that both familial and sporadic forms of Parkinson's disease are linked to SCNA, the gene encoding α-syn. More recently, accumulating evidence indicates that the soluble oligomeric form of α-syn, α-syn oligomers (α-synOs), are a toxic species present in Parkinson's disease that can contribute to neurodegeneration through a variety of mechanisms including disruptions in intracellular trafficking, disrupting normal autophagy, and eliciting synaptic dysfunction and loss. α-synOs can interact with synapses, and be spread transsynaptically to anatomically connected regions, spread which correlates with Braak staging of Parkinson's disease as well as Lewy body and synaptic pathology in neurons. The majority of α-syn targeted approaches in the clinic, have neglected to target the oligomeric species, and thus, therapeutic approaches effectively targeting α-synOs are urgently needed. Screening assays have identified compounds disclosed herein capable of blocking recombinant α-synO-induced deficits in lipid vesicle trafficking and in chaperone-mediated autophagy (increased expression of LAMP-2A). These compounds were identified as sigma-2 receptor modulators. Following confirmation of concentration-dependent activity in the trafficking assay, these compounds were tested in similar but more physiologically relevant trafficking assay using Parkinson's disease brain-derived α-synOs, and indeed, the sigma-2 modulators restored trafficking to normal. The sigma-2 receptor complex is comprised of transmembrane protein 97 (TMEM97) and progesterone receptor membrane component 1 (PGRMC1). Molecular interactions between the sigma-2 receptor component proteins PGRMC1 and TMEM97, α-synOs, and proteins that control vesicular tracking and autophagy (such as LC3B) may form the basis for these observations. Importantly, and for the first time, these data indicated that small molecule selective sigma-2 receptor modulators can prevent at least some toxic effects of α-synOs on neurons. In vitro proof of concept studies that sigma-2 receptor modulators may alleviate αSynO-induced neurotoxicity through multiple mechanisms supports that sigma-2 receptor modulators may be a promising therapeutic avenue to pursue for Parkinson's disease.

### Methods of Use

Various embodiments are directed to a method of treating neurological diseases, comprising administering to a subject in need thereof, a therapeutically effective amount of a compound as described herein.

In some embodiments the neurological disease is selected from the group consisting Age-Associated Memory Impairment (AAMI), Age-Related Cognitive Decline (ARCD), agitation synucleinopathies, Alzheimer's disease (AD), Amyotrophic lateral sclerosis (ALS) dementia, autosomal-dominant Parkinson's disease, chemotherapy-induced neuropathy (CIPN), Cognitive Impairment No Dementia (CIND), dementia, Creutzfeldt-Jakob disease (CJD), Diffuse Lewy Body Disease (DLBD) also known as Dementia with Lewy Bodies (DLB), disorders or conditions characterized by the presence of Lewy bodies, Down syndrome, dyskinesia, epilepsy, frontotemporal dementia (FTD), HIV-associated Neurocognitive Disorder (HAND), HIV dementia, Huntington's disease, Incidental LBD, Inherited LBD, Lewy body dysphagia, Mild Cognitive Impairment (MCI), multiple sclerosis, multiple system atrophy (MSA), Neuropathies (including but not limited to peripheral neuropathy, diabetic neuropathy and retinal neuropathy), Olivopontocerebellar Atrophy, Parkinson's disease (PD), preclinical Alzheimer's Disease (PCAD), psychiatric disorders (including but not limited to schizophrenia, bipolar disorders, depression, mania, anxiety disorders, post-traumatic stress disorders, delirium, eating disorders, autism, REM sleep behavior disorder, halucinations, attention-deficit hyperactivity disorders, and psychosis), Pure Autonomic Failure, seizures, Shy-Drager Syndrome, Striatonigral Degeneration, synucleinopathies, traumatic brain injury (TBI), combined Alzheimer's and Parkinson disease and/or MSA, vascular dementia, diseases, disorders or conditions associated with abnormal expression, stability, activities and/or cellular processing of α-synuclein, diseases, disorders or conditions characterized by the presence of Lewy bodies, and combinations thereof.

Various embodiments are directed to a method of treating a neurologic disease, comprising administering to a subject in need thereof, a therapeutically effective amount of a compound described herein.

Some embodiments are directed to a method of treating Parkinson's disease, comprising administering to a subject in need thereof, a therapeutically effective amount of a compound as described herein.

Some embodiments are directed to a method of treating Huntington's disease, comprising administering to a subject in need thereof, a therapeutically effective amount of a compound as described herein.

Some embodiments are directed to a method of treating Diffuse Lewy Body Disease (DLBD) also known as Dementia with Lewy Bodies (DLB), comprising administering to a subject in need thereof, a therapeutically effective amount of a compound as described herein.

Some embodiments are directed to a method of preventing cell death in a neuronal cell, comprising administering to a subject in need thereof, a therapeutically effective amount of a compound as described herein.

In some embodiments, a compound according to any embodiment described herein, may be protective against cellular dysfunction in a neurologic disease. In some embodiments, a compound according to any embodiment described herein, may prevent cellular dysfunction associated with a neurological disease. In some embodiments, a compound according to any embodiment described herein, may prevent cellular dysfunction associated a neurological disease wherein the cellular dysfunction may be caused by exposure to oligomeric Abeta, oxidative stress, and activities of complement C3. In some embodiments, the neurological disease is selected from Age-Associated Memory Impairment (AAMI), Age-Related Cognitive Decline (ARCD), agitation synucleinopathies, Alzheimer's disease (AD), Amyotrophic lateral sclerosis (ALS) dementia, autosomal-dominant Parkinson's disease, chemotherapy-induced neuropathy (CIPN), Cognitive Impairment No Dementia (CIND), dementia, Creutzfeldt-Jakob disease (CJD), Diffuse Lewy Body Disease (DLBD) also known as Dementia with Lewy Bodies (DLB), disorders or conditions characterized by the presence of Lewy bodies, Down syndrome, dyskinesia, epilepsy, frontotemporal dementia (FTD), HIV-associated Neurocognitive Disorder (HAND), HIV dementia, Huntington's disease, Incidental LBD, Inherited LBD, Lewy body dysphagia, Mild Cognitive Impairment (MCI), multiple sclerosis, multiple system atrophy (MSA), Neuropathies (including but not limited to peripheral neuropathy, diabetic neuropathy and retinal neuropathy), Olivopontocerebellar Atrophy, Parkinson's disease (PD), preclinical Alzheimer's Disease (PCAD), psychiatric disorders (including but not limited to schizophrenia, bipolar disorders, depression, mania, anxiety disorders, post-traumatic stress disorders, delirium, eating disorders, autism, REM sleep behavior disorder, halucinations, attention-deficit hyperactivity disorders, and psychosis), Pure Autonomic Failure, seizures, Shy-Drager Syndrome, Striatonigral Degeneration, synucleinopathies, traumatic brain injury (TBI), combined Alzheimer's and Parkinson disease and/or MSA, vascular dementia, diseases, disorders or conditions associated with abnormal expression, stability, activities and/or cellular processing of α-synuclein, diseases, disorders or conditions characterized by the presence of Lewy bodies, and combinations thereof.

In some embodiments, the oxidative stress results in cellular damage. In some embodiments cellular damage is selected from the group consisting of cytotoxicity, lipid peroxidation, carbonyl formation, formation of reactive oxygen species, changes in mitochondrial membrane potential, changes in mitochondrial mass, changes in mitochondrial function, changes in autophagic flux, loss of lysosomal integrity, changes in lysosomal activity, defects in photoreceptor outer segment (POS) trafficking, accumulation of toxic macromolecules, axonal injury, cell senescence, apoptosis, and cell death.

Some embodiments are directed to a method of preventing cytotoxicity, comprising administering to a subject in need thereof, a therapeutically effective amount of a compound as described herein.

Some embodiments are directed to a method of preventing changes in lysosomal activity, comprising administering to a subject in need thereof, a therapeutically effective amount of a compound as described herein.

Some embodiments are directed to a method of preventing changes in autophagic flux, comprising administering to a subject in need thereof, a therapeutically effective amount of a compound as described herein.

Some embodiments are directed to a method of preventing cell death, comprising administering to a subject in need thereof, a therapeutically effective amount of a compound as described herein.

Some embodiments are directed to a method of preventing apoptosis, comprising administering to a subject in need thereof, a therapeutically effective amount of a compound as described herein.

Some embodiments are directed to a method of preventing complement C3 dysfunction, comprising administering to a subject in need thereof, a therapeutically effective amount of a compound as described herein.

In some embodiments, the complement C3 dysfunction results in cellular damage. In some embodiments cellular damage is selected from the group consisting of cell death, deficits in trans-epithelial electrical resistance (TEER), and deficits in RPE barriers.

Some embodiments are directed to a method of preventing inflammation, comprising administering to a subject in need thereof, a therapeutically effective amount of a compound as described herein.

Some embodiments are directed to a methods of slowing the progression of a neurologic disease, comprising administering to a subject in need thereof, a therapeutically effective amount of a compound as described herein.

Some embodiments are directed to a methods of preventing a neurologic disease, comprising administering to a subject in need thereof, a therapeutically effective amount of a compound as described herein.

Some embodiments are directed to a methods of slowing the progression of a symptom associated with a neurologic disease, comprising administering to a subject in need thereof, a therapeutically effective amount of a compound as described herein.

### Compounds for use in the Invention

In some embodiments the compound for use in the invention is a compound selected from the group consisting of:
A. a compound of Formula I, 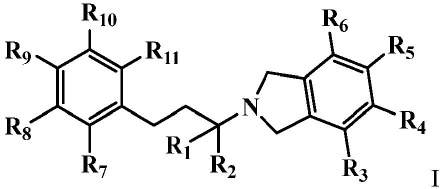 or a pharmaceutically acceptable salt thereof:
   wherein:
   each of R₁ and R₂ is independently selected from H, C₁-C₆ alkyl, or CH₂OR'; wherein each R' if present in R₁, and R₂ is independently H or C₁-C₆ alkyl;
   each of R₃, R₄, R₅, and R₆ is independently selected from the group consisting of H, C₁-C₆ alkyl, OH, OCH₃, OCH(CH₃)₂, OCH₂CH(CH₃)₂, OC(CH₃)₃, O(C₁-C₆ alkyl), OCF₃, OCH₂CH₂OH, O(C₁-C₆ alkyl)OH, O(C₁-C₆ haloalkyl), F, Cl, Br, I, CF₃, CN, NO₂, NH₂, C₁-C₆ haloalkyl, C₁-C₆ hydroxyalkyl, C₁-C₆ alkoxy C₁-C₆alkyl, aryl, heteroaryl, C₃-C₇ cycloalkyl, heterocycloalkyl, alkylaryl, CO₂R', C(O)R', NH(C₁-C₄ alkyl), N(C₁-C₄ alkyl)₂, NH(C₃-C₇ cycloalkyl), NHC(O)(C₁-C₄ alkyl), CONR'₂, NC(O)R', NS(O)ₙR', S(O)ₙNR'₂, S(O)ₙR', C(O)O(C₁-C₄ alkyl), OC(O)N(R')₂, C(O)(C₁-C₄ alkyl), and C(O)NH(C₁-C₄ alkyl), wherein each R' if present in R₃, R₄, R₅, and R₆ is independently selected from the group consisting of H, CH₃, CH₂CH₃, C₃-C₆ alkyl, C₁-C₆ haloalkyl, or optionally substituted aryl, alkylaryl, piperazin-1-yl, piperidin-1-yl, morpholinyl, heterocycloalkyl, heteroaryl, C₁-C₆ alkoxy, NH(C₁-C₄ alkyl), and N(C₁-C₄ alkyl)₂, wherein the optionally substituted group is selected from C₁-C₆ alkyl or C₂-C₇ acyl;
   or R₃ and R₄, together with the C atom to which they are attached form a 4-, 5-, 6- 7-or 8- membered cycloalkyl, aryl, heteroaryl, or heterocycloalkyl that is optionally substituted with 1, 2, 3, 4, or 5 substituents independently selected from OH, amino, halo, C₁₋C₆ alkyl, C₁-C₆ haloalkyl, C₁-C₆ alkoxy, C₁-C₆ haloalkoxy, aryl, arylalkyl, heteroaryl, heteroarylalkyl, cycloalkyl, and heterocycloalkyl or R₃ and R₄ are linked together to form a - O-C₁-C₂ methylene-O- group;
   or R₄ and R₅, together with the C atom to which they are attached form a 4-, 5-, 6- 7-or 8- membered cycloalkyl, aryl, heteroaryl, or heterocycloalkyl that is optionally substituted with 1, 2, 3, 4, or 5 substituents independently selected from OH, amino, halo, C₁₋C₆ alkyl, C₁-C₆ haloalkyl, C₁-C₆ alkoxy, C₁-C₆ haloalkoxy, aryl, arylalkyl, heteroaryl, heteroarylalkyl, cycloalkyl, and heterocycloalkyl or R₄ and R₅ are linked together to form a - O-C₁₋₂ methylene-O- group;
   each of R₇, R₈, R₉, R₁₀, and R₁₁ is independently selected from the group consisting of H, C₁-C₆ alkyl, OH, OCH₃, OCH(CH₃)₂, OCH₂CH(CH₃)₂, OC(CH₃)₃, O(C₁-C₆ alkyl), OCF₃, OCH₂CH₂OH, O(C₁-C₆ alkyl)OH, O(C₁-C₆ haloalkyl), O(CO)R', F, Cl, Br, I, CF₃, CN, NO₂, NH₂, C₁-C₆ haloalkyl, C₁-C₆ hydroxyalkyl, C₁-C₆ alkoxy C₁-C₆ alkyl, aryl, heteroaryl, C₃-C₇ cycloalkyl, heterocycloalkyl, alkylaryl, heteroaryl, CO₂R', C(O)R', NH(C₁-C₄ alkyl), N(C₁-C₄ alkyl)₂, NH(C₃-C₇ cycloalkyl), NHC(O)(C₁-C₄ alkyl), CONR'₂, NC(O)R', NS(O)ₙR', S(O)ₙNR'₂, S(O)ₙR', C(O)O(C₁-C₄ alkyl), OC(O)N(R')₂, C(O)(C₁-C₄ alkyl), and C(O)NH(C₁-C₄ alkyl); wherein each R' if present in R₇, R₈, R₉, R₁₀, and R₁₁ is independently selected from the group consisting of H, CH₃, CH₂CH₃, C₃-C₆ alkyl, C₁-C₆ haloalkyl, aryl, alkylaryl, piperazin-1-yl, piperidin-1-yl, morpholinyl, heterocycloalkyl, heteroaryl, C₁-C₆ alkoxy, NH(C₁-C₄ alkyl), and N(C₁-C₄ alkyl)₂;
   or R₇ and R₈, together with the N or C atoms to which they are attached form a 4-, 5-, 6- 7- or 8- membered cycloalkyl, aryl, heterocycloalkyl or heteroaryl group that is optionally substituted with 1, 2, 3, 4, or 5 substituents independently selected from OH, amino, halo, C₁-C₆ alkyl, C₁-C₆ haloalkyl, C₁-C₆ alkoxy, C₁-C₆ haloalkoxy, aryl, arylalkyl, heteroaryl, heteroarylalkyl, cycloalkyl, and heterocycloalkyl or R₇ and R₈ are linked together to form a -O-C₁₋₂ methylene-O- group;
   or R₈ and R₉, together with the N or C atoms to which they are attached form a 4-, 5-, 6- 7-or 8- membered cycloalkyl, aryl, heterocycloalkyl or heteroaryl group that is optionally substituted with 1, 2, 3, 4, or 5 substituents independently selected from OH, amino, halo, C₁-C₆ alkyl, C₁-C₆ haloalkyl, C₁-C₆ alkoxy, C₁-C₆ haloalkoxy, aryl, arylalkyl, heteroaryl, heteroarylalkyl, cycloalkyl, and heterocycloalkyl or R₈ and R₉ are linked together to form a -O-C₁₋₂ methylene-O- group;
   each n is independently 0, 1, or 2;
   with the proviso that R₇, R₈, R₉, R₁₀, and R₁₁ are not all H; and
   with the proviso that the following compounds, or pharmaceutically acceptable salts thereof are excluded: 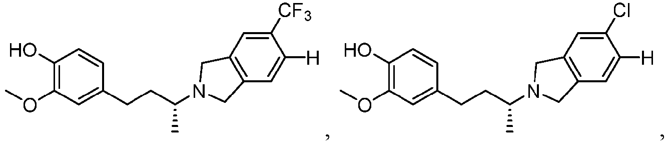 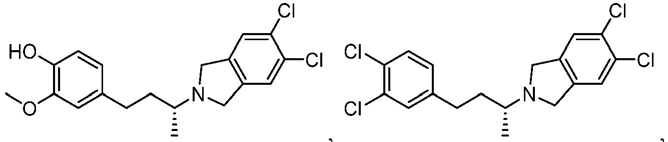 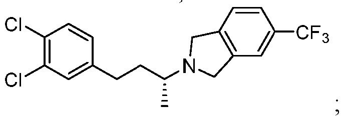 or
B. a compound of Formula IA 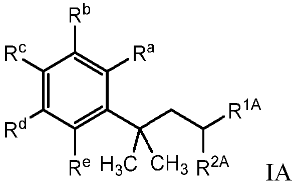 or pharmaceutically acceptable salt thereof:
   wherein:
   each of R^{a}, R^{b}, R^{c}, R^{d} and R^{e} is independently selected from the group consisting of, H, hydroxyl, Cl, F, methyl, -OCH₃, -OC(CH₃)₃, O-CH(CH₃)₂, CF₃, SO₂CH₃, and morpholino;
   R^{1A} is selected from the group consisting of hydrogen, alkyl, phenyl, or - CH=C(CH₃)₂; and
   R^{2A} is an optionally substituted cyclic amino group.

### Compounds of Formula I

In some embodiments a compound for use in the invention is selected from a compound of Formula I 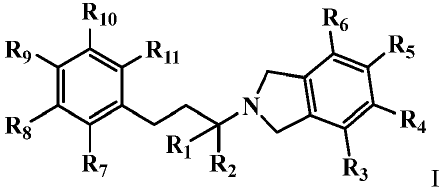 or a pharmaceutically acceptable salt thereof:
wherein:
each of R₁ and R₂ is independently selected from H, C₁-C₆ alkyl, or CH₂OR'; wherein each R' if present in R₁, and R₂ is independently H or C₁-C₆ alkyl;
each of R₃, R₄, R₅, and R₆ is independently selected from the group consisting of H, C₁-C₆ alkyl, OH, OCH₃, OCH(CH₃)₂, OCH₂CH(CH₃)₂, OC(CH₃)₃, O(C₁-C₆ alkyl), OCF₃, OCH₂CH₂OH, O(C₁-C₆ alkyl)OH, O(C₁-C₆ haloalkyl), F, Cl, Br, I, CF₃, CN, NO₂, NH₂, C₁-C₆ haloalkyl, C₁-C₆ hydroxyalkyl, C₁-C₆ alkoxy C₁-C₆alkyl, aryl, heteroaryl, C₃-C₇ cycloalkyl, heterocycloalkyl, alkylaryl, CO₂R', C(O)R', NH(C₁-C₄ alkyl), N(C₁-C₄ alkyl)₂, NH(C₃-C₇ cycloalkyl), NHC(O)(C₁-C₄ alkyl), CONR'₂, NC(O)R', NS(O)ₙR', S(O)ₙNR'₂, S(O)ₙR', C(O)O(C₁-C₄ alkyl), OC(O)N(R')₂, C(O)(C₁-C₄ alkyl), and C(O)NH(C₁-C₄ alkyl), wherein each R' if present in R₃, R₄, R₅, and R₆ is independently selected from the group consisting of H, CH₃, CH₂CH₃, C₃-C₆ alkyl, C₁-C₆ haloalkyl, or optionally substituted aryl, alkylaryl, piperazin-1-yl, piperidin-1-yl, morpholinyl, heterocycloalkyl, heteroaryl, C₁-C₆ alkoxy, NH(C₁-C₄ alkyl), and N(C₁-C₄ alkyl)₂, wherein the optionally substituted group is selected from C₁-C₆ alkyl or C₂-C₇ acyl;
or R₃ and R₄, together with the C atom to which they are attached form a 4-, 5-, 6- 7-or 8- membered cycloalkyl, aryl, heteroaryl, or heterocycloalkyl that is optionally substituted with 1, 2, 3, 4, or 5 substituents independently selected from OH, amino, halo, C₁₋C₆ alkyl, C₁-C₆ haloalkyl, C₁-C₆ alkoxy, C₁-C₆ haloalkoxy, aryl, arylalkyl, heteroaryl, heteroarylalkyl, cycloalkyl, and heterocycloalkyl or R₃ and R₄ are linked together to form a - O-C₁-C₂ methylene-O- group;
or R₄ and R₅, together with the C atom to which they are attached form a 4-, 5-, 6- 7-or 8- membered cycloalkyl, aryl, heteroaryl, or heterocycloalkyl that is optionally substituted with 1, 2, 3, 4, or 5 substituents independently selected from OH, amino, halo, C₁₋C₆ alkyl, C₁-C₆ haloalkyl, C₁-C₆ alkoxy, C₁-C₆ haloalkoxy, aryl, arylalkyl, heteroaryl, heteroarylalkyl, cycloalkyl, and heterocycloalkyl or R₄ and R₅ are linked together to form a - O-C₁₋₂ methylene-O- group;
each of R₇, R₈, R₉, R₁₀, and R₁₁ is independently selected from the group consisting of H, C₁-C₆ alkyl, OH, OCH₃, OCH(CH₃)₂, OCH₂CH(CH₃)₂, OC(CH₃)₃, O(C₁-C₆ alkyl), OCF₃, OCH₂CH₂OH, O(C₁-C₆ alkyl)OH, O(C₁-C₆ haloalkyl), O(CO)R', F, Cl, Br, I, CF₃, CN, NO₂, NH₂, C₁-C₆ haloalkyl, C₁-C₆ hydroxyalkyl, C₁-C₆ alkoxy C₁-C₆ alkyl, aryl, heteroaryl, C₃-C₇ cycloalkyl, heterocycloalkyl, alkylaryl, heteroaryl, CO₂R', C(O)R', NH(C₁-C₄ alkyl), N(C₁-C₄ alkyl)₂, NH(C₃-C₇ cycloalkyl), NHC(O)(C₁-C₄ alkyl), CONR'₂, NC(O)R', NS(O)ₙR', S(O)ₙNR'₂, S(O)ₙR', C(O)O(C₁-C₄ alkyl), OC(O)N(R')₂, C(O)(C₁-C₄ alkyl), and C(O)NH(C₁-C₄ alkyl); wherein each R' if present in R₇, R₈, R₉, R₁₀, and R₁₁ is independently selected from the group consisting of H, CH₃, CH₂CH₃, C₃-C₆ alkyl, C₁-C₆ haloalkyl, aryl, alkylaryl, piperazin-1-yl, piperidin-1-yl, morpholinyl, heterocycloalkyl, heteroaryl, C₁-C₆ alkoxy, NH(C₁-C₄ alkyl), and N(C₁-C₄ alkyl)₂;
or R₇ and R₈, together with the N or C atoms to which they are attached form a 4-, 5-, 6- 7- or 8- membered cycloalkyl, aryl, heterocycloalkyl or heteroaryl group that is optionally substituted with 1, 2, 3, 4, or 5 substituents independently selected from OH, amino, halo, C₁-C₆ alkyl, C₁-C₆ haloalkyl, C₁-C₆ alkoxy, C₁-C₆ haloalkoxy, aryl, arylalkyl, heteroaryl, heteroarylalkyl, cycloalkyl, and heterocycloalkyl or R₇ and R₈ are linked together to form a -O-C₁₋₂ methylene-O- group;
or R₈ and R₉, together with the N or C atoms to which they are attached form a 4-, 5-, 6- 7-or 8- membered cycloalkyl, aryl, heterocycloalkyl or heteroaryl group that is optionally substituted with 1, 2, 3, 4, or 5 substituents independently selected from OH, amino, halo, C₁-C₆ alkyl, C₁-C₆ haloalkyl, C₁-C₆ alkoxy, C₁-C₆ haloalkoxy, aryl, arylalkyl, heteroaryl, heteroarylalkyl, cycloalkyl, and heterocycloalkyl or R₈ and R₉ are linked together to form a -O-C₁₋₂ methylene-O- group;
each n is independently 0, 1, or 2;
with the proviso that R₇, R₈, R₉, R₁₀, and R₁₁ are not all H; and
with the proviso that the following compounds, or pharmaceutically acceptable salts thereof are excluded:

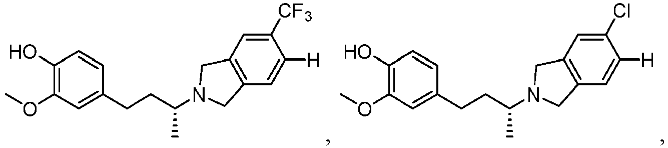

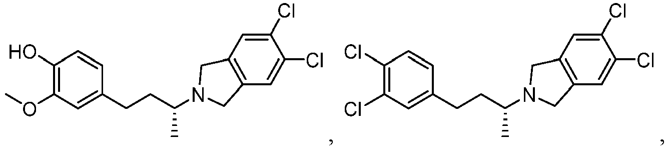

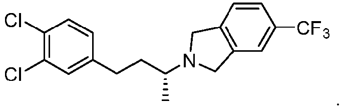

In some embodiments, a compound for use in the invention, is a compound of Formula I, or pharmaceutically acceptable salt thereof, wherein R₁ and R₂ are each independently selected from H or CH₃; R₃, R₄, R₅, and R₆ are each independently selected from H, C₁-C₆ alkyl, OH, OCH₃, O(C₁-C₆ alkyl), O(C₁-C₆ haloalkyl), F, Cl, CF₃, aryl, heteroaryl, C₃₋₇ cycloalkyl, CO₂R', C(O)R', OC(O)N(R')₂, CONR'₂, NC(O)R', NS(O)ₙR', S(O)ₙNR'₂, S(O)ₙR'; where n= 0, 1, or 2; R' are each independently H, CH₃, CH₂CH₃, C₃-C₆ alkyl, C₁-C₆ haloalkyl; or optionally substituted piperazin-1-yl, piperidin-1-yl, morpholinyl, heterocycloalkyl, or aryl, wherein optionally substituted group is selected from C₁-C₆ alkyl or C₂-C₇ acyl; or R₃ and R₄, together with the C atom to which they are attached, form a 5-, or 6-membered C₃₋₇cycloalkyl, or aryl; or R₄ and R₅, together with the C atom to which they are attached, form a C₃₋₇cycloalkyl, or a 5- or 6- membered aryl; or R₃ and R₄ are linked together to form a -O-C₁₋₂ methylene-O- group; or R₄ and R₅ are linked together to form a -O-C₁₋₂ methylene-O- group; and R₇, R₈, R₉, R₁₀, and R₁₁ are each independently selected from H, OH, CH₃, CH₂CH₃, F, Cl, CF₃, OCF₃, C₁-C₆ haloalkyl, OCH₃, O(C₁-C₆ alkyl), OCH₂CH₂OH, O(C₁-C₆ alkyl)OH, aryl, heteroaryl, C₃₋₇ cycloalkyl, alkylaryl, CO₂R', CONR'₂, S(O)ₙNR'₂, S(O)ₙR', C(O)O(C₁₋₄ alkyl), OC(O)N(R')₂, and C(O)NH(C₁₋₄ alkyl); where n= 0, 1, or 2; R' are each independently H, C₁-C₆ alkyl, C₁-C₆ haloalkyl, aryl, alkylaryl, or C₁₋₆ alkoxy.

In some embodiments, a compound for use in the invention, a compound for use in the invention, is a compound of Formula I, or pharmaceutically acceptable salt thereof, wherein R₇, R₁₀, R₁₁ are each H; R₃ and R₄ are each independently selected from H, F, Cl, S(O)ₙR', C(O)R', wherein n= 2, and R' is selected from CH₃, piperazin-1-yl, piperidin-1-yl, morpholinyl; R₈ is selected from OH, OCH₃, OCH(CH₃)₂, OCH₂CH(CH₃)₂, or OC(CH₃)₃; and R₉ is OH.

In some embodiments, a compound for use in the invention a compound for use in the invention, is a compound of Formula I, or pharmaceutically acceptable salt thereof, selected from the group consisting of: 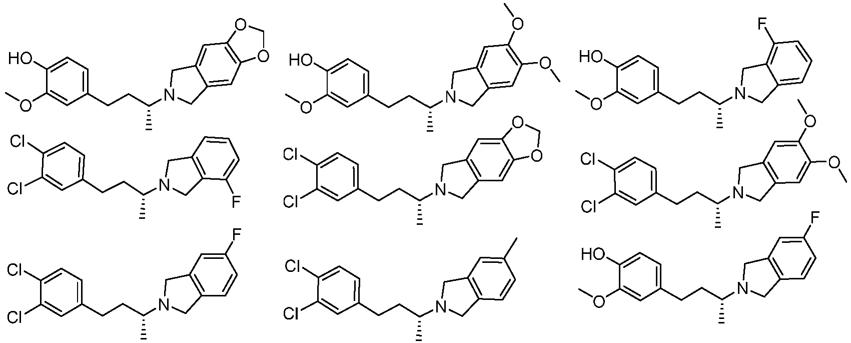 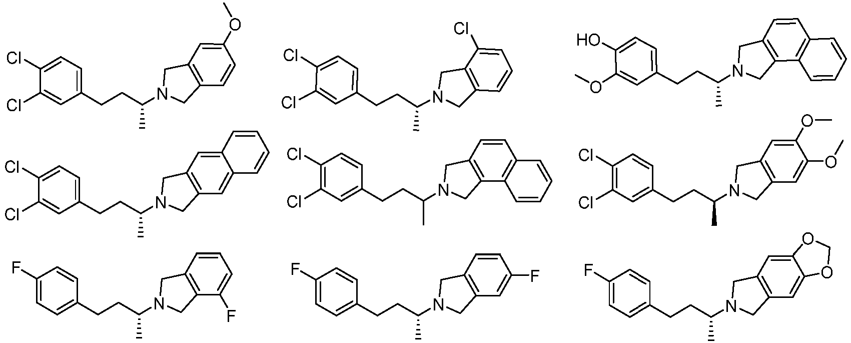 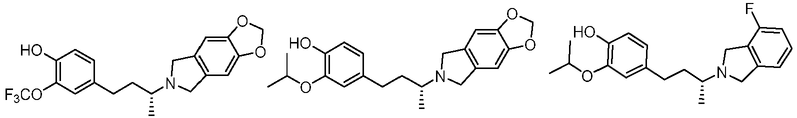 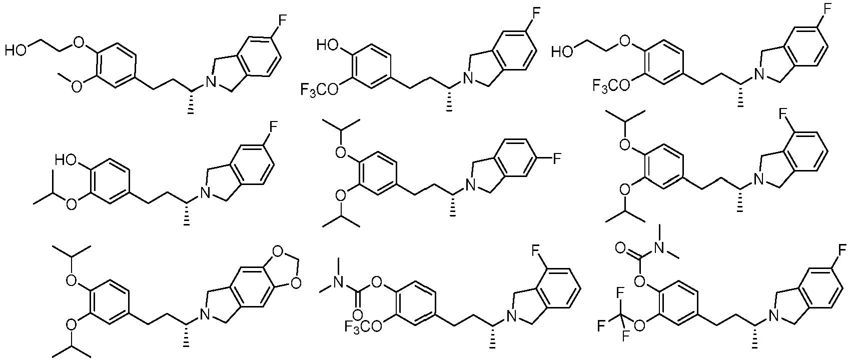 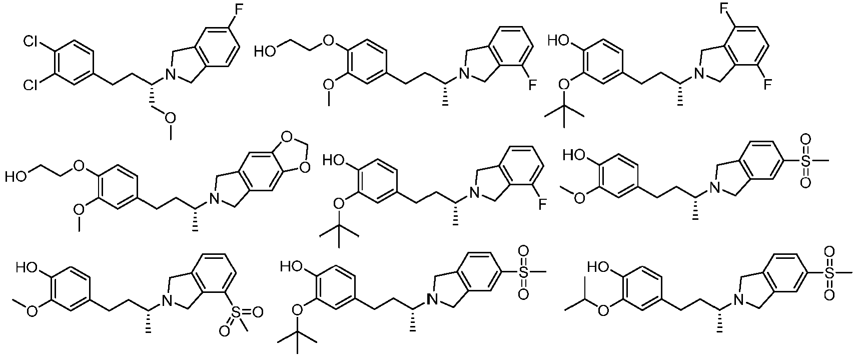 and

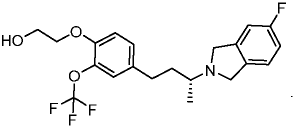

In further embodiments, a compound for use in the invention a compound of Formula II, or pharmaceutically acceptable salt thereof: 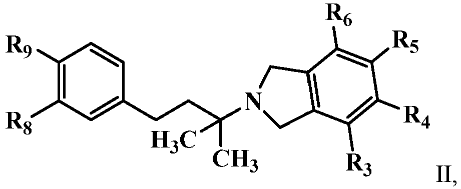
wherein R₃, R₄, R₅, and R₆ are each independently selected from H, Cl, F, OH, CH₃, C₁₋₆ alkyl, OCH₃, OCH(CH₃)₂, OCH₂CH(CH₃)₂, OC(CH₃)₃, OC₁₋₆ alkyl, aryl, heteroaryl, heterocycloalkyl, CO₂R', CONR'₂, NC(O)R', NS(O)ₙR', S(O)ₙNR'₂, S(O)ₙR', C(O)R', OC(O)N(R')₂, or C(O)NH(C₁₋₄ alkyl), wherein n= 0, 1, or 2; and R' are each independently H, C₁-C₆ alkyl, C₁-C₆ haloalkyl; or optionally substituted aryl, alkylaryl, piperazin-1-yl, piperidin-1-yl, morpholinyl, heterocycloalkyl, heteroaryl, C₁₋₆ alkoxy, NH(C₁₋₄ alkyl), or NH(C₁₋₄ alkyl)₂, wherein optionally substituted group is selected from C₁-C₆ alkyl or C₂-C₇ acyl;
or R₃ and R₄, together with the C atom to which they are attached, form a 6-membered aryl; or R₃ and R₄ are linked together to form a -O-C₁₋₂ methylene-O- group; or R₄ and R₅, together with the C atom to which they are attached, form a 6- membered aryl; or R₄ and R₅ are linked together to form a -O-C₁₋₂ methylene-O- group; and
R₈ and R₉ are each independently selected from H, Cl, F, OH, CH₃, C₁₋₆ alkyl, OCH₃, OCH(CH₃)₂, OCH₂CH(CH₃)₂, OC(CH₃)₃, O(CO)R', OC₁₋₆ alkyl, aryl, heteroaryl, heterocycloalkyl, CO₂R', CONR'₂, NC(O)R', NS(O)ₙR', S(O)ₙNR'₂, S(O)ₙR', OC(O)N(R')₂, or C(O)NH(C₁₋₄ alkyl);
or R₈ and R₉, together with the N or C atoms to which they are attached form a form a 4-, 5-, 6- 7-or 8- membered cycloalkyl, aryl, heterocycloalkyl or heteroaryl group that is optionally substituted with 1, 2, 3, 4, or 5 substituents independently selected from OH, amino, halo, C₁₋₆ alkyl, C₁₋₆ haloalkyl, C₁₋₆ alkoxy, C₁₋₆ haloalkoxy, aryl, arylalkyl, heteroaryl, heteroarylalkyl, cycloalkyl, and heterocycloalkyl and R⁹ and R¹⁰ are each independently selected from a bond, C, N, S, and O; or R₈ and R₉ are linked together to form a -O-C₁₋₂ methylene-O- group.

In further embodiments, a compound for use in the invention, is a compound of Formula II, or pharmaceutically acceptable salt thereof, wherein at least one of R₃, R₄, R₅ and R₆ is not H; and at least one of R₈ and R₉ is not H.

In other embodiments, a compound for use in the invention, is a compound of Formula II, or pharmaceutically acceptable salt thereof, wherein R₇, R₁₀, R₁₁ are each H; R₃ and R₄ are each independently selected from H, F, Cl, S(O)ₙR', C(O)R', wherein n= 2, and R' is selected from CH₃, or optionally substituted piperazin-1-yl, piperidin-1-yl, or morpholinyl, wherein optionally substituted group is selected from C₁-C₆ alkyl or C₂-C₇ acyl; R₈ is selected from OH, Cl, OCH₃, OCH(CH₃)₂, OCH₂CH(CH₃)₂, or OC(CH₃)₃; and R₉ is OH or Cl.

In further embodiments, a compound for use in the invention, is a compound of Formula II, or pharmaceutically acceptable salt thereof, wherein R₃ and R₄ are each independently selected from H, F, Cl, S(O)ₙR', C(O)R', wherein n= 2, and R' is selected from CH₃, piperazin-1-yl, piperidin-1-yl, or morpholinyl; R₅ and R₆ are each H; R₈ is selected from OH, OCH₃, OCH(CH₃)₂, OCH₂CH(CH₃)₂, or OC(CH₃)₃; and R₉ is OH.

In further embodiments, a compound for use in the invention, or pharmaceutically acceptable salt thereof is selected from the group consisting of

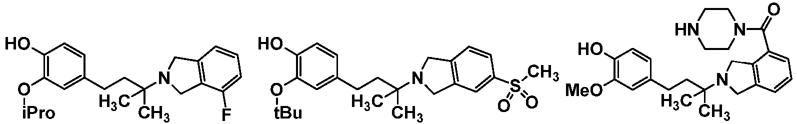

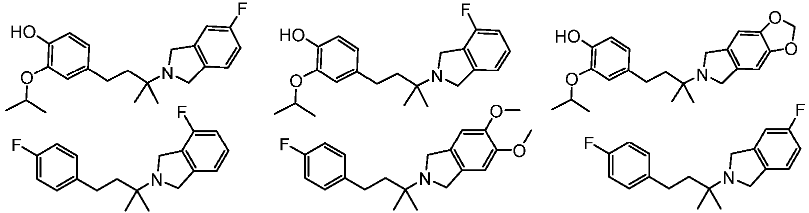

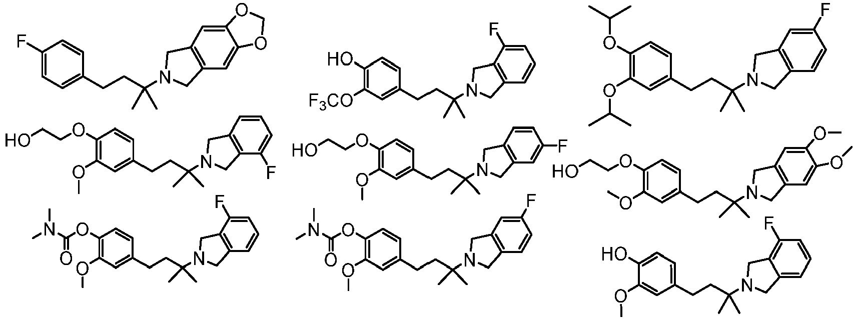

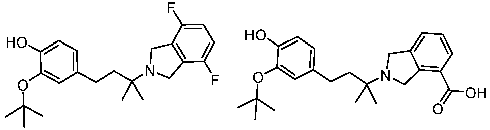

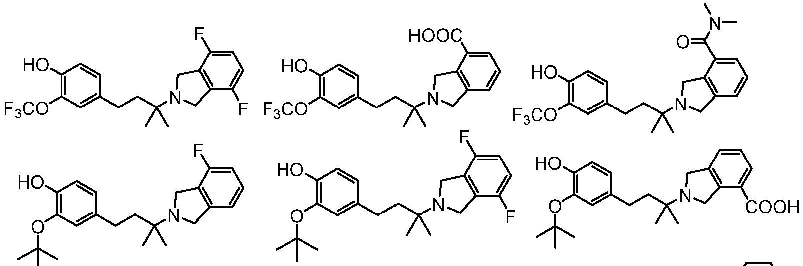

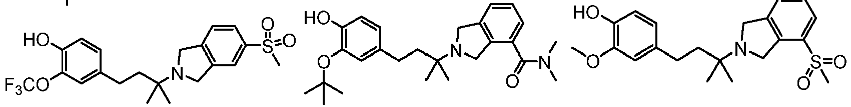

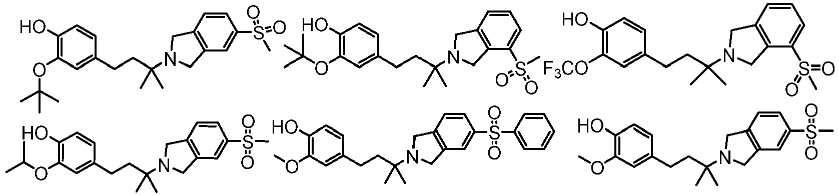

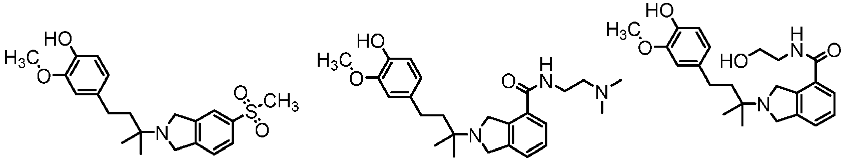

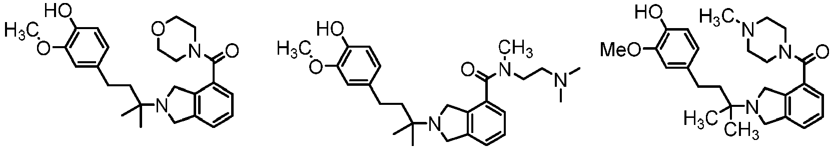

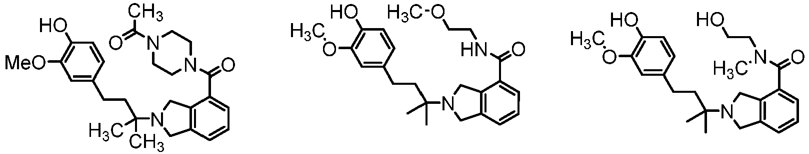

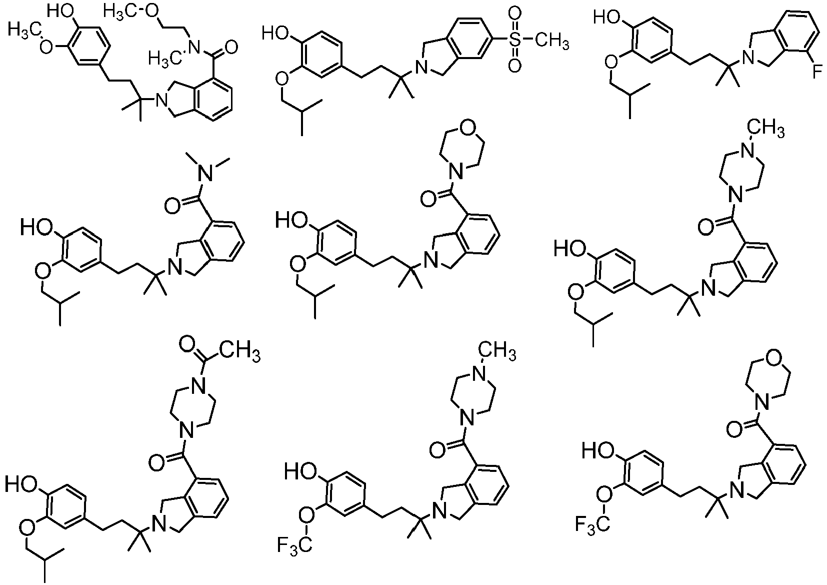

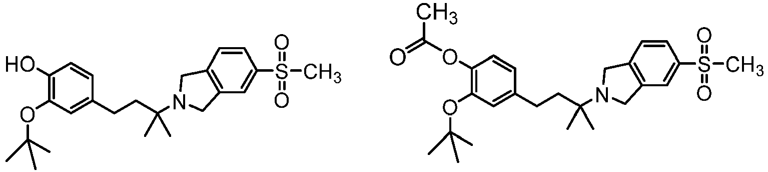

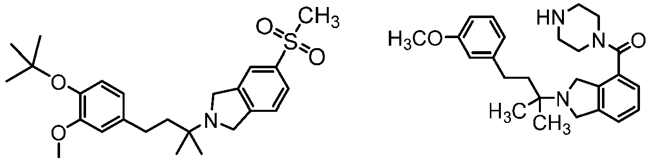

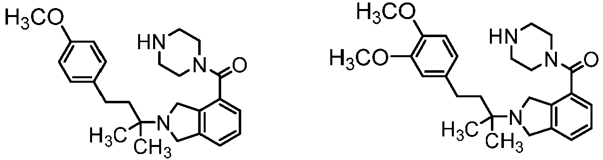

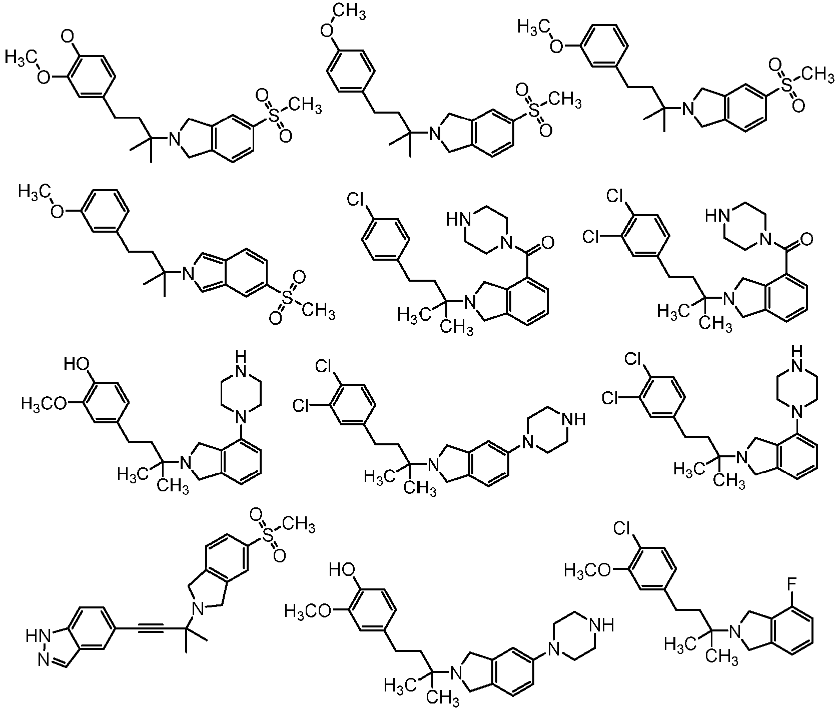

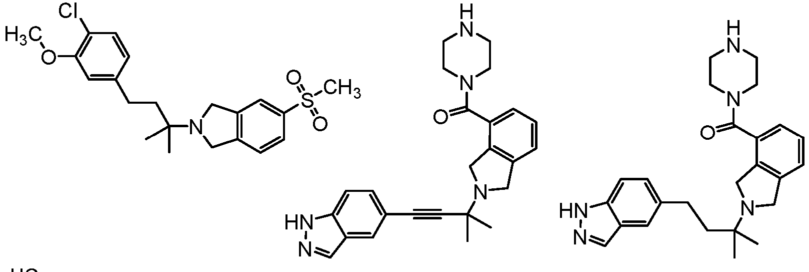

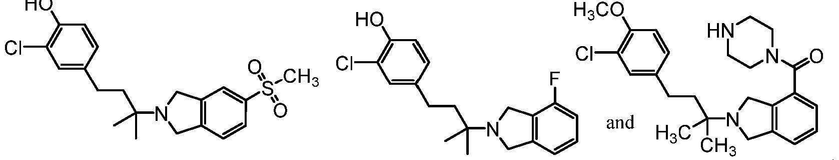

In further embodiments, a compound for use in the invention, or pharmaceutically acceptable salt thereof, is a compound selected from the group consisting of:

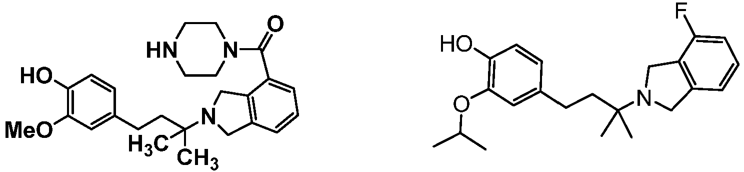

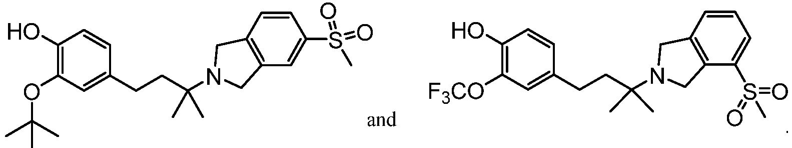

In further embodiments, a compound for use in the invention, or pharmaceutically acceptable salt thereof, is a compound selected from the group consisting of:

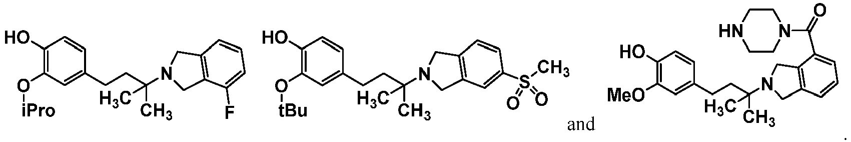

In some embodiments, a compound for use in the invention, is: 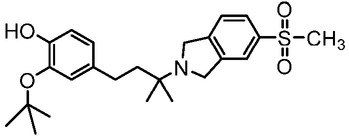 or a pharmaceutically acceptable salt thereof.

In some embodiments, a compound for use in the invention, is:

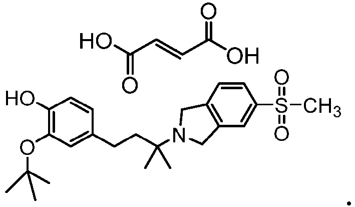

In some embodiments a compound for use in the invention, or pharmaceutically acceptable salt thereof, is a compound wherein each of R₁, R₂, R₃, R₄, R₅, R₆, R₇, R₈, R₉, R₁₀, and R₁₁ of Formula I is as defined herein, with the proviso that when R₁, R₃, R₆, R₇, R₁₀ and R₁₁ are each H; R₂ is CH₃; R₈ is OCH₃ or Cl; and R₉ is OH or Cl; then R₄ is not Cl or CF₃, and R₅ is not Cl or CF₃.

In some embodiments, a compound for use in the invention, is a compound of Formula II: 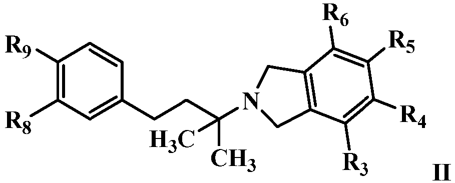
or a pharmaceutically acceptable salt thereof
wherein R₃, R₄, R₅, R₆, R₈, and R₉ are as described herein.

In another embodiment, a compound for use in the invention, is a compound of Formula III: 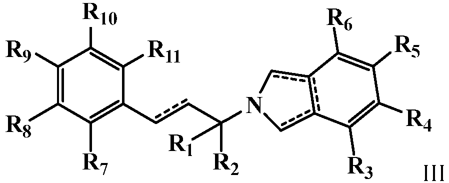 or pharmaceutically acceptable salt thereof, wherein R₃, R₄, R₅, R₆, R₇, R₈, R₉, R₁₀ and R₁₁ are as provided herein and wherein each ----- is independently selected from a single, double or triple bond.

In some aspects, a compound for use in the invention, is a compound according to Formula III selected from the group consisting of: 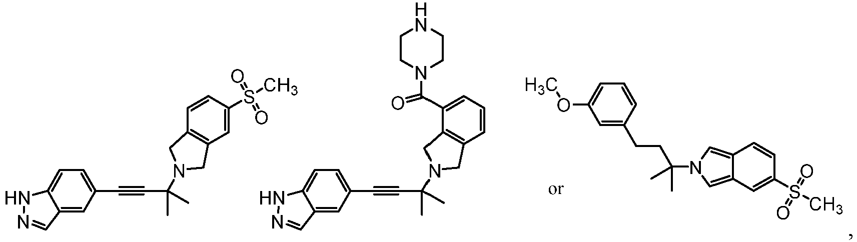 or a pharmaceutically acceptable salt thereof.

In some embodiments, a compound for use in the invention comprises a racemic mixture or an enantiomer of a compound of Formula I, wherein R₃, R₄, R₅, R₆, R₈, and R₉ are as described herein.

In some embodiments, a compound for use in the invention comprises a compound of Formula I, or a pharmaceutically acceptable salt thereof, wherein R₈ and R₉ are independently selected from OH, C₁₋₆ alkoxy, and hydroxy C₁₋₆ alkoxy.

In some embodiments, a compound for use in the invention comprises a compound of Formula I, or a pharmaceutically acceptable salt thereof, wherein R₈ and R₉ are independently selected from OH and NH(C₁₋₄ alkyl).

In some embodiments, a compound for use in the invention comprises a compound of Formula I, or a pharmaceutically acceptable salt thereof, wherein R₈ and R₉ are independently selected from H, halo, C₁₋₆ haloalkyl, and C₁₋₆ haloalkoxy.

In some embodiments, a compound for use in the invention comprises a compound of Formula I, or a pharmaceutically acceptable salt thereof, wherein R₈ and R₉ are each independently selected from OH, halo, C₁₋₆ alkoxy and C₁₋₆ haloalkoxy and R₁ and R₂ are each independently C₁₋₆ alkyl.

In some embodiments, a compound for use in the invention comprises a compound of Formula I, or a pharmaceutically acceptable salt thereof, wherein R₁ and R₂ are each methyl.

In some embodiments, a compound for use in the invention comprises a compound of Formula I, or a pharmaceutically acceptable salt thereof, wherein one of R₁ and R₂ is methyl and the other is H.

In some embodiments, a compound for use in the invention comprises a compound of Formula I, or a pharmaceutically acceptable salt thereof, wherein R₈ and R₉ are each independently selected from OH and C₁₋₆ alkoxy and R₁ and R₂ are each independently methyl.

In some embodiments, a compound for use in the invention comprises a compound of Formula I, or a pharmaceutically acceptable salt thereof, wherein R₈ and R₉ are independently selected from H, halo, and C₁₋₆ haloalkyl, and R₁ and R₂ are each methyl.

In some embodiments, a compound for use in the invention comprises a compound of Formula I, or a pharmaceutically acceptable salt thereof, wherein R⁸ and R⁹ are each independently selected from H, halo and C_{1- 6} haloalkyl.

In some embodiments, a compound for use in the invention comprises a compound of Formula I, or a pharmaceutically acceptable salt thereof, wherein R₇ and R₁₁ are each H.

In some embodiments, a compound for use in the invention comprises a compound of Formula I, or a pharmaceutically acceptable salt thereof, wherein R₃, R₄, R₅, and R₆ are each independently selected from H, halo, C_{1- 6} alkyl, C₁₋₆ haloalkyl and C₁₋₆ alkoxy.

In some embodiments, a compound for use in the invention comprises a compound of Formula I, or a pharmaceutically acceptable salt thereof, wherein R₃, R₄ and R₅ are each independently selected from H, halo, C₁₋₆ alkyl, C₁₋₆ haloalkyl and C₁₋₆ alkoxy.

In some embodiments, a compound for use in the invention comprises a compound of Formula I, or a pharmaceutically acceptable salt thereof, wherein R₃, R₄, R₅, and R₆ are each independently selected from H, halo, S(O)ₙR', C(O)OR', C(O)N(R')₂, and C(O)R'; where n= 2; R' are each independently H, CH₃, CH₂CH₃, C₃-C₆ alkyl, C₁-C₆ haloalkyl, or optionally C₁-C₆ alkyl or C₂-C₇ acyl substituted aryl, alkylaryl, piperazinyl, piperidinyl, morpholinyl, heterocycloalkyl, and heteroaryl.

In some embodiments, a compound for use in the invention comprises a compound of Formula I, or a pharmaceutically acceptable salt thereof, wherein R₃, R₄ and R₅ are each independently selected from H, halo, S(O)ₙR', and C(O)R'; where n= 2; R' are each independently CH₃, CH₂CH₃, C₃-C₆ alkyl, aryl, piperazin-1-yl, piperidin-1-yl, and morpholinyl-4-yl.

In some embodiments, a compound for use in the invention comprises a compound of Formula I, or a pharmaceutically acceptable salt thereof, wherein R₃, R₄ and R₅ are each independently selected from H, halo, S(O)ₙR', and C(O)R'; where n= 2; R' are each independently CH₃, CH₂CH₃, C₃-C₆ alkyl, aryl, piperazin-1-yl, piperidin-1-yl, and morpholinyl-4-yl; R₈ and R₉ are each independently selected from OH, halo, C₁₋₆ alkoxy and C₁₋₆ haloalkoxy; and R₁ and R₂ are each methyl.

In some embodiments, a compound for use in the invention comprises a compound of Formula I, or a pharmaceutically acceptable salt thereof, wherein R₃ and R₄ or R₄ and R₅ together with the C atom to which they are attached form a 6-membered cycloalkyl, or a heterocycloalkyl, aryl or heteroaryl ring.

In some embodiments, a compound for use in the invention comprises a compound of Formula I, or a pharmaceutically acceptable salt thereof, wherein R₃ and R₄ or R₄ and R₅ are O, and are linked together to form a -O-C₁₋₂ methylene-O- group.

In some embodiments, a compound for use in the invention comprises a compound of Formula I, or a pharmaceutically acceptable salt thereof, wherein R² and R³ are independently selected from H, OH, halo, C₁₋₆ alkoxy and C₁₋₆ haloalkyl.

In some embodiments, a compound for use in the invention comprises a compound of Formula II, or a pharmaceutically acceptable salt thereof, wherein R₃ and R₄ are independently selected from H, Cl, F, -OMe, -CF₃, S(O)ₙR', and C(O)R'; where n= 2; R' are each independently H, CH₃, CH₂CH₃, C₃-C₆ alkyl, aryl, piperazin-1-yl, piperidin-1-yl, and morpholinyl-4-yl; R₈ and R₉ are each independently selected from OH and C₁₋₆ alkoxy.

In some embodiments, a compound for use in the invention comprises a compound of Formula I, or a pharmaceutically acceptable salt thereof, wherein R² and R³ are independently selected from H, OH, Cl, F, -OMe, and -CF₃, wherein R⁷ and R⁸ are each independently selected from H and C₁₋₆ alkyl, wherein R⁹ is H, and wherein R⁵ and R⁶ are each independently selected from H and C₁₋₆ haloalkyl.

In some embodiments, a compound of any of Formulas I-III according to any embodiment described herein, may contain a proviso to remove one or more of the following compounds:

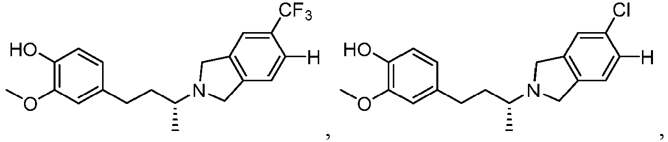

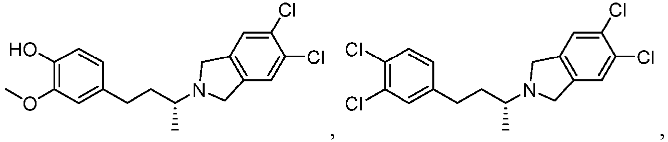

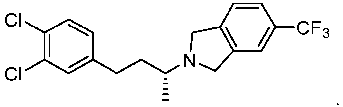

### Compounds of Formula IA

In some embodiments a compound for use in the invention comprises a compound of Formula IA 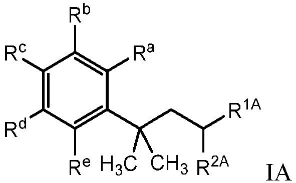 or pharmaceutically acceptable salt thereof:
wherein:
each of R^{a}, R^{b}, R^{c}, R^{d} and R^{e} is independently selected from the group consisting of, H, hydroxyl, Cl, F, methyl, -OCH₃, -OC(CH₃)₃, O-CH(CH₃)₂, CF₃, SO₂CH₃, and morpholino;
R^{1A} is selected from the group consisting of hydrogen, alkyl, phenyl, or - CH=C(CH₃)₂; and
R^{2A} is an optionally substituted cyclic amino group.

In some embodiments, a compound for use in the invention comprises a compound of Formula IA wherein each of substituents R^{a}, R^{b}, R^{c}, R^{d} and R^{e} is independently selected from the group consisting of, H, hydroxyl, Cl, F, methyl, -OCH₃, -OC(CH₃)₃, O-CH(CH₃)₂, CF₃, SO₂CH₃, and morpholino.

In some embodiments, a compound for use in the invention comprises a compound of Formula IA wherein each of substituents R^{a}, R^{b}, R^{c}, R^{d} and R^{e} is independently selected from the group consisting of, H, Cl, F, and CF₃.

In some embodiments, a compound for use in the invention comprises a compound of Formula IA wherein each of substituents R^{a}, R^{b}, R^{d} and R^{e} is independently H; and R^{c} is selected from the group consisting of H, hydroxyl, halo, alkyl, alkoxy, CF₃, SO₂CH₃, and morpholino.

In some embodiments, a compound for use in the invention comprises a compound of Formula IA wherein each of substituents R^{a}, R^{b}, R^{d} and R^{e} is independently H; and R^{c} is selected from the group consisting of H, hydroxyl, Cl, F, methyl, -OCH₃, - OC(CH₃)₃, O-CH(CH₃)₂, CF₃, SO₂CH₃, and morpholino.

In some embodiments, a compound for use in the invention comprises a compound of Formula IA wherein each of substituents R^{a}, R^{b}, R^{d} and R^{e} is independently H; and R^{c} is selected from the group consisting of H, Cl, F, and CF₃.

In various embodiments, a compound for use in the invention comprises a compound of Formula IA wherein R^{2A} is any heterocycloalkyl or heteroaryl containing a nitrogen in the ring that is bound to the aliphatic chain of Formula IA through the nitrogen atom. In some embodiments, for example, R^{2A} is an optionally substituted cyclic amino group selected from: 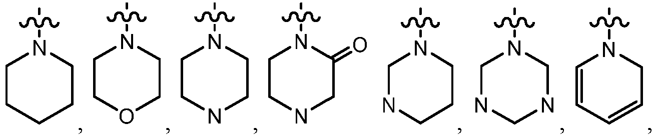 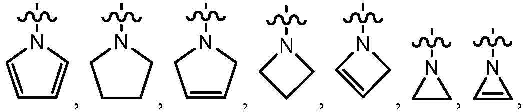 and the like, wherein each nitrogen containing heterocycloalkyl or heteroaryl can be optionally substituted with one or more substituents selected from, hydroxyl, halo, CF₃, alkoxy, aryloxy, optionally substituted C₁-C₁₀ alkyl, optionally substituted C₅-C₁₀ aryl, optionally substituted C₃-C₁₀ heteroaryl, substituted or unsubstituted C₃-C₁₀ cycloalkyl or heterocycloalkyl.

In various embodiments, a compound for use in the invention comprises a compound of Formula IA wherein R^{2A} is selected from the group consisting of optionally substituted aziridinyl, optionally substituted pyrrolidinyl, optionally substituted imidizolidinyl, optionally substituted piperidinyl, optionally substituted piperazinyl, optionally substituted oxopiperazinyl, and optionally substituted morpholinyl.

In some embodiments, a compound for use in the invention comprises a compound of Formula IA wherein when R^{2A} is a substituted cyclic amino, one or more of the hydrogen atoms in the cyclic amino group is replaced with a group selected from alkanoyl, alkoxy, alkoxyalkyl, (alkoxy)alkoxyalkyl, alkoxycarbonyl, alkyl, aryloxy, aryloyl, cycloalkanoyl, -OC(O)NCH(CH₃)₂, (N,N-dimethylamino)pyridinyl, (N,N-dimethylamino)sulfonyl, halo, heterocyclyl, (heterocyclyl)alkoxyalkyl, hydroxyl, hydroxyalkyl, methylpiperidinyl, methylsulfonyl, methylsulfonylphenyl, morpholinylpyridinyl, perfluoroalkyl, phenyl, piperidinyl, pyrrolidinylpyridinyl, tetrahydropyranyl, and CF₃. In some embodiments two hydrogen atoms on the same carbon of the cyclic amino group are replaced with a compound selected from 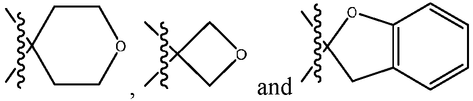 to form a spiro compound.

In some embodiments, a compound for use in the invention comprises a compound of Formula IA wherein R^{2A} is a pyrrolidinyl or a substituted pyrrolidinyl substituted with one or more substituents selected from the group consisting of alkoxyalkyl, alkoxycarbonyl, alkyl, hydroxyl, and hydroxyalkyl. In some embodiments R^{2A} is a substituted pyrrolidinyl substituted with a single substituent selected from the group consisting of alkoxyalkyl, alkoxycarbonyl, alkyl, hydroxyl, and hydroxyalkyl. In some embodiments R^{2A} is a substituted pyrrolidinyl substituted with a single substituent selected from the group consisting of hydroxyl, hydroxymethyl, methoxymethyl, methoxycarbonyl and methyl.

In some embodiments, a compound for use in the invention comprises a compound of Formula IA wherein R^{2A} is a piperidinyl or a substituted piperidinyl substituted with one or more substituents selected from the group consisting of alkoxy, alkoxyalkyl, (alkoxy)alkoxyalkyl, alkoxycarbonyl, alkyl, aryloxy, -OC(O)NCH(CH₃)₂, (N,N-dimethylamino)pyridinyl, halo, heterocyclyl, (heterocyclyl)alkoxyalkyl, hydroxy, hydroxyalkyl, methylpiperidinyl, methylsulfonylphenyl, morpholinylpyridinyl, perfluoroalkyl, phenyl, piperidinyl, pyrrolidinylpyridinyl, tetrahydropyranyl, and CF₃. In some embodiments, R^{2A} is a piperidinyl or a substituted piperidinyl substituted with a single substituent selected from the group consisting of alkoxy, alkoxyalkyl, (alkoxy)alkoxyalkyl, alkoxycarbonyl, alkyl, aryloxy, -OC(O)NCH(CH₃)₂, (N,N-dimethylamino)pyridinyl, halo, heterocyclyl, (heterocyclyl)alkoxyalkyl, hydroxyl, hydroxyalkyl, methylpiperidinyl, methylsulfonylphenyl, morpholinylpyridinyl, perfluoroalkyl, phenyl, piperidinyl, pyrrolidinylpyridinyl, tetrahydropyranyl, and CF₃. In some embodiments, R^{2A} is a piperidinyl or a substituted piperidinyl substituted with a single substituent selected from the group consisting of alkoxy, alkoxyalkyl, (alkoxy)alkoxyalkyl, alkoxycarbonyl, alkyl, aryloxy, - OC(O)NCH(CH₃)₂, (N,N-dimethylamino)pyridinyl, halo, heterocyclyl, (heterocyclyl)alkoxyalkyl, hydroxyl, hydroxyalkyl, methylpiperidinyl, methylsulfonylphenyl, morpholinylpyridinyl, perfluoroalkyl, phenyl, piperidinyl, pyrrolidinylpyridinyl, tetrahydropyranyl, and CF₃. In some embodiments, R^{2A} is a piperidinyl or a substituted piperidinyl substituted with a single substituent selected from the group consisting of methyl, isopropyl, isobutyl, CF₃, hydroxymethyl, hydroxyethyl, (isopropyloxy)ethyl, - (CH₂)₂O(CH₂)₂OCH₃, -(CH₂)₃OCH₃, -C(O)OMe, -C(O)OEt, hydroxyl, methoxy, isopropyloxy, phenyloxy, F, ethoxy, phenyl, 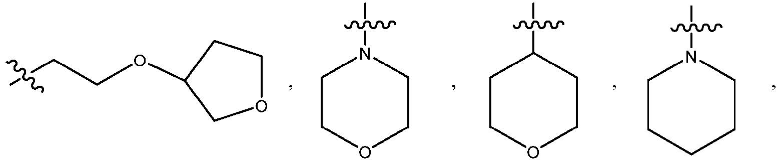 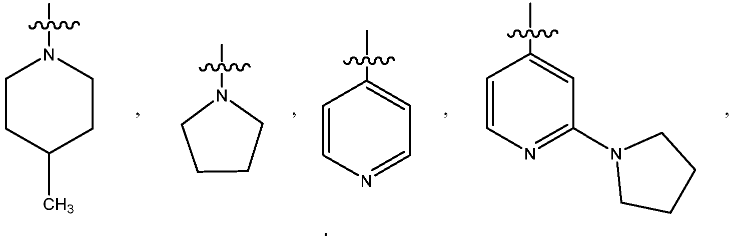 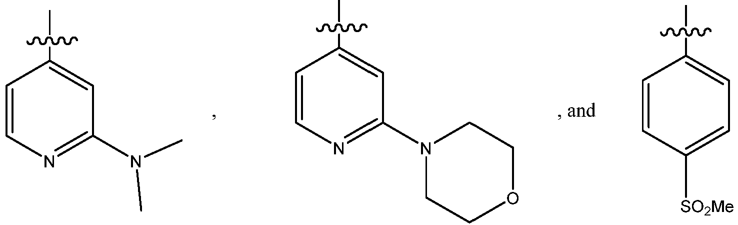 In some embodiments, R^{2A} is a piperidinyl or a substituted piperidinyl substituted at the 4 position of the piperidinyl with a single substituent selected from the group consisting of alkoxy, alkoxyalkyl, (alkoxy)alkoxyalkyl, alkoxycarbonyl, alkyl, aryloxy, - OC(O)NCH(CH₃)₂, (N,N-dimethylamino)pyridinyl, halo, heterocyclyl, (heterocyclyl)alkoxyalkyl, hydroxyl, hydroxyalkyl, methylpiperidinyl, methylsulfonylphenyl, morpholinylpyridinyl, perfluoroalkyl, phenyl, piperidinyl, pyrrolidinylpyridinyl, tetrahydropyranyl, and CF₃. In some embodiments, R^{2A} is a piperidinyl or a substituted piperidinyl substituted at the 4 position of the piperidinyl with a single substituent selected from the group consisting of methyl, isopropyl, isobutyl, CF₃, hydroxymethyl, hydroxyethyl, (isopropyloxy)ethyl, -(CH₂)₂O(CH₂)₂OCH₃, -(CH₂)₃OCH₃, -C(O)OMe, -C(O)OEt, hydroxyl, methoxy, isopropyloxy, phenyloxy, F, ethoxy, phenyl,

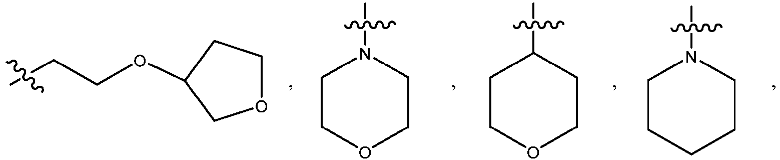

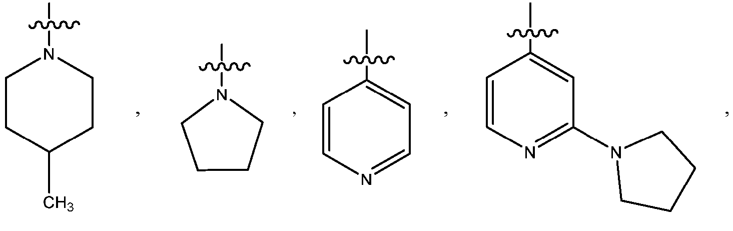

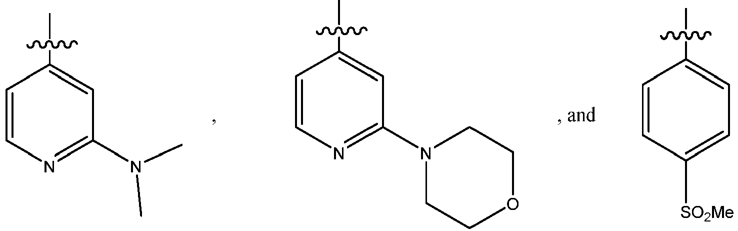

In some embodiments, a compound for use in the invention comprises a compound of Formula IA wherein R^{2A} is a piperidinyl or a substituted piperidinyl substituted with two substituent groups on the same carbon of the piperidinyl independently selected from the group consisting of alkoxyalkyl, alkyl, -OC(O)NCH(CH₃)₂, hydroxyl, and phenyl. In some embodiments, R^{2A} is a piperidinyl or a substituted piperidinyl substituted with two substituent groups at the 4 position of the piperidinyl independently selected from the group consisting of alkoxyalkyl, alkyl, -OC(O)NCH(CH₃)₂, hydroxyl, and phenyl. In some embodiments R^{2A} is a piperidinyl or a substituted piperidinyl substituted with two substituent groups at the 4 position selected from the group consisting of hydroxyl and methyl; hydroxyl and ethyl; hydroxyl and -(CH₂)₂OCH₃; hydroxyl and phenyl; methyl and phenyl; methyl and -OC(O)NCH(CH₃)₂; and butyl and -OC(O)NCH(CH₃)₂. In some embodiments two hydrogen atoms on the same carbon of the piperidinyl are replaced with a compound selected from 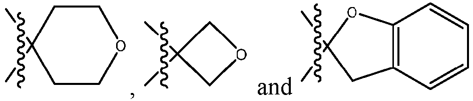 to form a spiro compound. In some embodiments two hydrogen atoms at the 4 position of the piperidinyl are replaced with a compound selected from 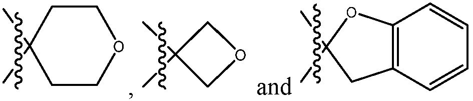 to form a spiro compound.

In some embodiments, a compound for use in the invention comprises a compound of Formula IA wherein R^{2A} is a piperazinyl or a substituted piperazinyl substituted with one or more substituents selected from the group consisting of alkanoyl, alkoxycarbonyl, aryloyl, cycloalkanoyl, (N,N-dimethylamino)sulfonyl, heterocyclyl, methylsulfonyl, and phenyl. In some embodiments, R^{2A} is a substituted piperazinyl substituted with a single substituent selected from the group consisting of alkanoyl, alkoxycarbonyl, aryloyl, cycloalkanoyl, (N,N-dimethylamino)sulfonyl, heterocyclyl, methylsulfonyl, and phenyl. In some embodiments, R^{2A} is a substituted piperazinyl substituted with a single substituent selected from the group consisting of -C(O)OC(CH₃)₃, -C(O)OCH₂CH(CH₃)₂, - C(O)OCH₂CH₃, -C(O)OCH₃, phenyl, -C(O)CH₃, -C(O)Ph, -SO₂Me, -SO₂N(CH₃)₂, 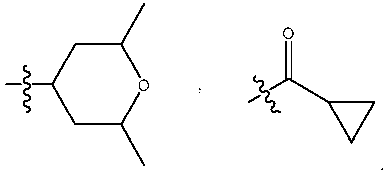 In some embodiments, R^{2A} is a substituted piperazinyl substituted with a single substituent at the 4 position selected from the group consisting of - C(O)OC(CH₃)₃, -C(O)OCH₂CH(CH₃)₂, -C(O)OCH₂CH₃, -C(O)OCH₃, phenyl, -C(O)CH₃, - C(O)Ph, -SO₂Me, -SO₂N(CH₃)₂,

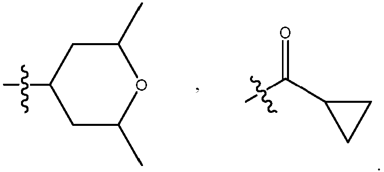

In certain embodiments, a compound for use in the invention comprises a compound of Formula IA wherein R^{2A} is a substituted piperdinyl of formula: 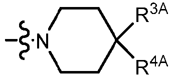 wherein, R^{3A} is hydrogen or C₁-C₈ alkyl, and R^{4A} is hydrogen, hydroxyl, halogen, CF₃, alkoxy, aryloxy, optionally substituted C₁-C₁₀ alkyl, optionally substituted C₅-C₁₀ aryl, optionally substituted C₃-C₁₀ heteroaryl, optionally substituted C₃-C₁₀ cycloalkyl or optionally substituted C₃-C₁₀ heterocycloalkyl.

In some embodiments, a compound for use in the invention comprises a compound of Formula IA wherein R^{2A} is 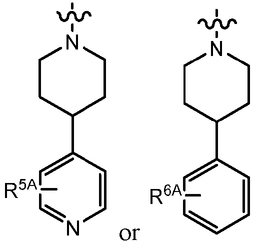 wherein each of R^{5A} and R^{6A} is independently, hydrogen, hydroxyl, sulfonyl, dialkylamino, optionally substituted C₁-C₁₀ alkyl, optionally substituted C₅-C₁₀ aryl optionally substituted C₃-C₁₀ heteroaryl, optionally substituted C₃-C₁₀ cycloalkyl or optionally substituted C₃-C₁₀ heterocycloalkyl. In some embodiments R^{5A} is hydrogen, dialkylamino, or C₃-C₁₀ heterocycloalkyl. In some embodiments R^{5A} is hydrogen, dialkylamino, pyrrolidinyl or morpholinyl. In some embodiments, R^{6A} is sulfonyl. In some embodiments, R^{6A} is methylsulfonyl.

In some embodiments, a compound for use in the invention comprises a compound of Formula IA wherein R^{2A} is: 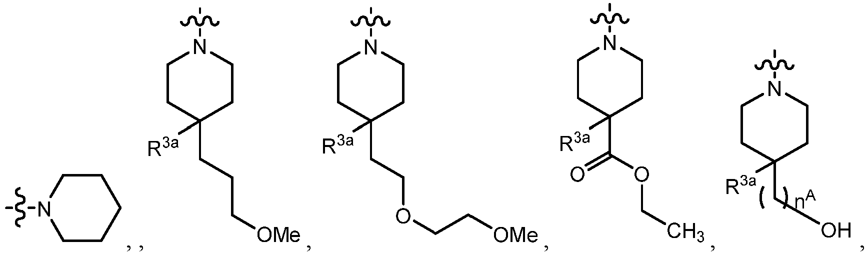 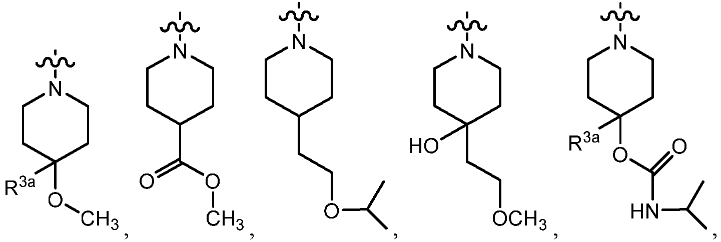 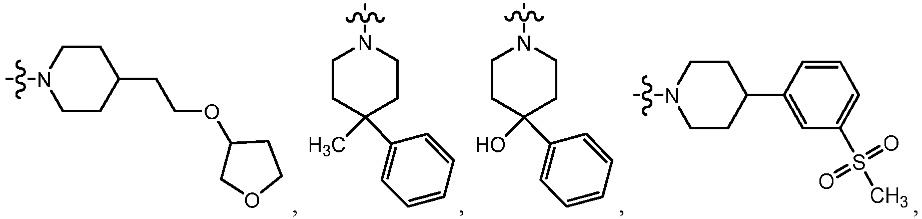 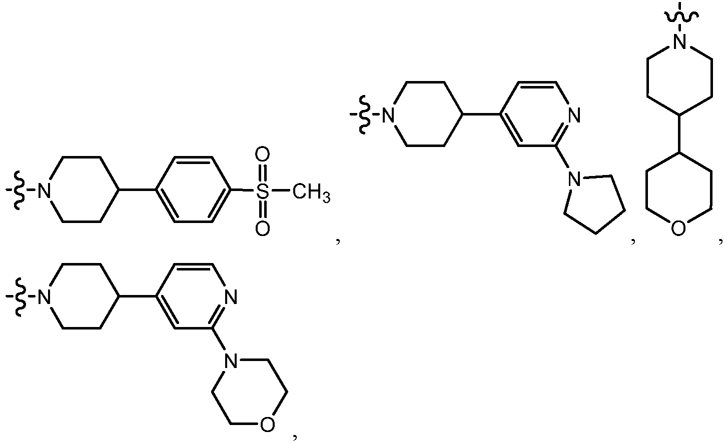 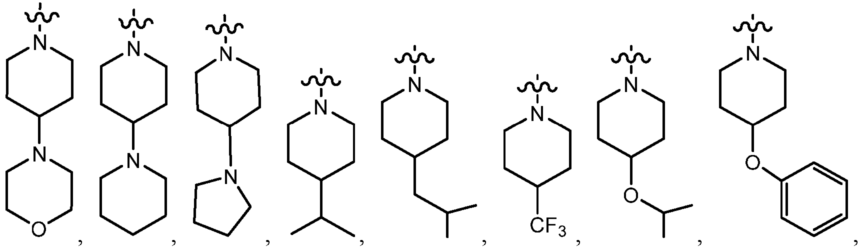 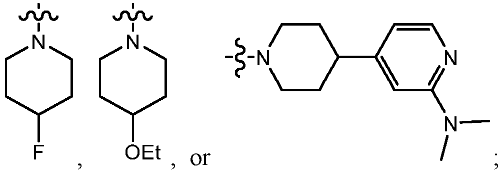 wherein R^{3a} selected from the group consisting of hydrogen and C₁-C₈ alkyl; and n^{A} is an integer selected from 0, 1 and 2.

In some embodiments a compound for use in the invention comprises a compound of Formula IA wherein R^{2A} is

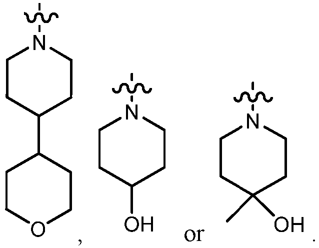

In some embodiments, a compound for use in the invention comprises a compound of Formula IA wherein R^{2A} is optionally substituted morpholinyl. In some embodiments, R^{2A} is morpholinyl.

In some embodiments a compound for use in the invention comprises a compound of Formula IA wherein R^{2A} or is optionally substituted piperazinyl of the formula 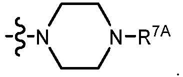 wherein R⁷ is hydrogen, hydroxyl, sulfonyl, dialkylaminosulfonyl, alkoxycarbonyl, acyl, benzoyl, cycloalkylcarbonyl, optionally substituted C₁-C₁₀ alkyl, optionally substituted C₅-C₁₀ aryl optionally substituted C₃-C₁₀ heteroaryl, optionally substituted C₃-C₁₀ cycloalkyl or optionally substituted C₃-C₁₀ heterocycloalkyl. In some embodiments R^{7A} is sulfonyl, dialkylaminosulfonyl, alkoxycarbonyl, acyl, benzoyl, cycloalkylcarbonyl, C₅-C₁₀ aryl or optionally substituted C₃-C₁₀ heterocycloalkyl.

In some embodiments a compound for use in the invention comprises a compound of Formula IA wherein R^{2A} is

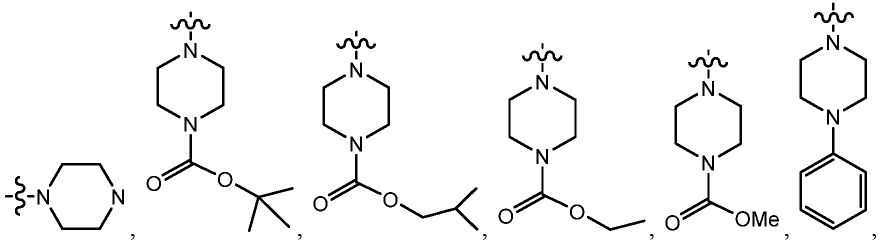

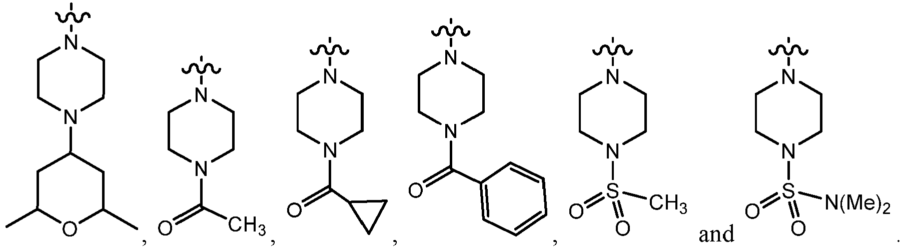

In various embodiments, a compound for use in the invention comprises a compound of Formula IA wherein R^{2A} is optionally substituted pyrrolidinyl: 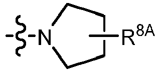 where R^{8A} is hydrogen, hydroxyl, sulfonyl, optionally substituted C₁-C₁₀ alkyl, optionally substituted C₅-C₁₀ aryl, optionally substituted C₃-C₁₀ heteroaryl, optionally substituted C₃-C₁₀ cycloalkyl or optionally substituted C₃-C₁₀ heterocycloalkyl. In some embodiments, R^{8A} is hydrogen, hydroxyl or optionally substituted C₁-C₁₀ alkyl.

In some embodiments, a compound for use in the invention comprises a compound of Formula IA wherein R^{2A} is:

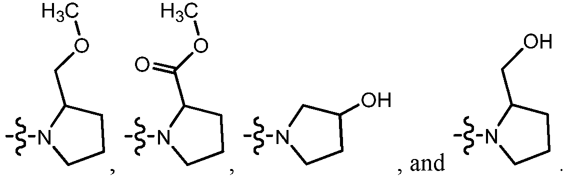

In some embodiments, a compound for use in the invention comprises a compound of Formula IA wherein R^{2A} is an optionally substituted bicyclic ring or an optionally substituted fused ring. For example, in some embodiments, R^{2A} is selected from the group consisting of: 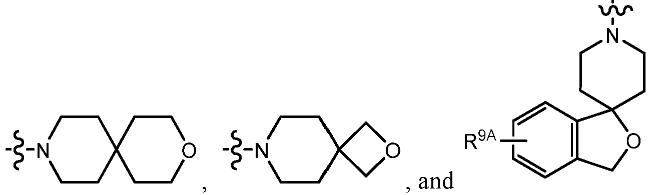 where R^{9A} is hydrogen, hydroxyl, sulfonyl, optionally substituted C₁-C₁₀ alkyl, optionally substituted C₅-C₁₀ aryl, optionally substituted C₃-C₁₀ heteroaryl, optionally substituted C₃-C₁₀ cycloalkyl or optionally substituted C₃-C₁₀ heterocycloalkyl.

In some embodiments, a compound for use in the invention comprises a compound of Formula IA wherein R^{2A} is 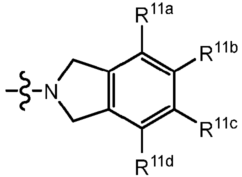 wherein each of R^{11a}, R^{11b}, R^{11c}, and R^{11d}, is, independently selected from, hydrogen, hydroxy, sulfonyl, optionally substituted C₁-C₁₀ alkyl, optionally substituted C₅-C₁₀ aryl, optionally substituted C₃-C₁₀ heteroaryl, optionally substituted C₃-C₁₀ cycloalkyl or optionally substituted C₃-C₁₀ heterocycloalkyl. In particular embodiments, R^{2A} is

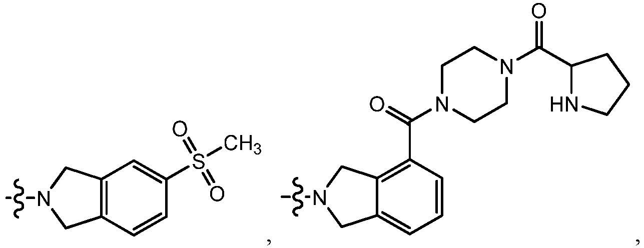

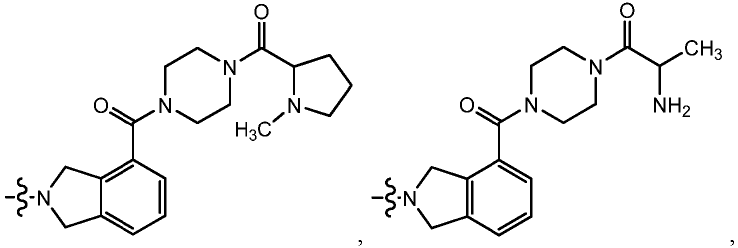

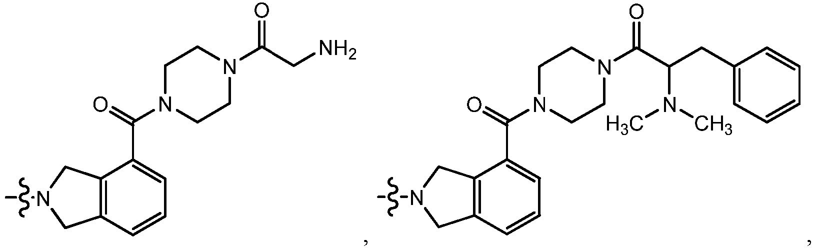

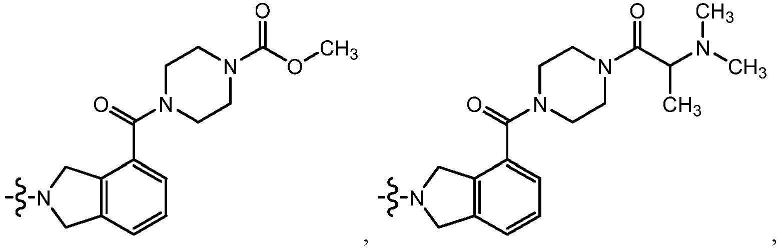

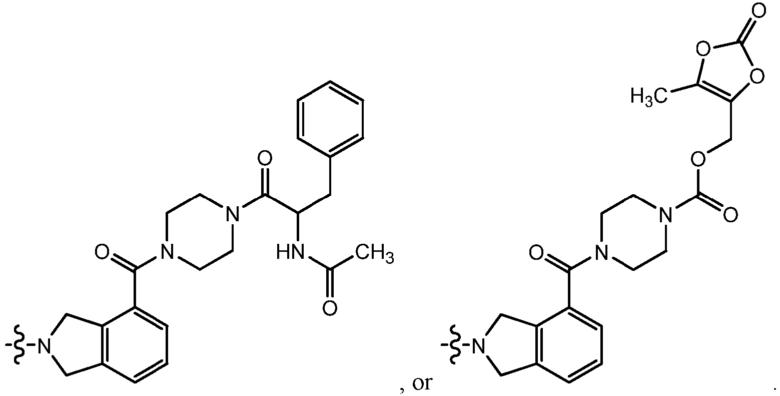

In some embodiments a compound for use in the invention is a compound of Formula IA wherein each R^{a}, R^{b}, R^{c}, R^{d} and R^{e} is selected from any embodiment disclosed herein for each of R^{a}, R^{b}, R^{c}, R^{d} and R^{e}; R^{1A} is selected from any embodiment disclosed herein for R^{1A}; and R^{2A} is selected from any embodiment disclosed herein for R^{2A}.

In some embodiments a compound for use in the invention is a compound selected from the group consisting of:

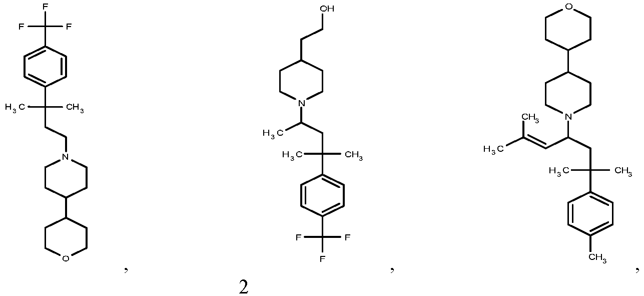

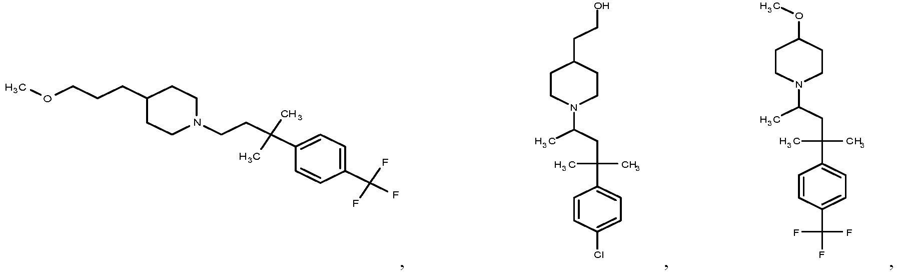

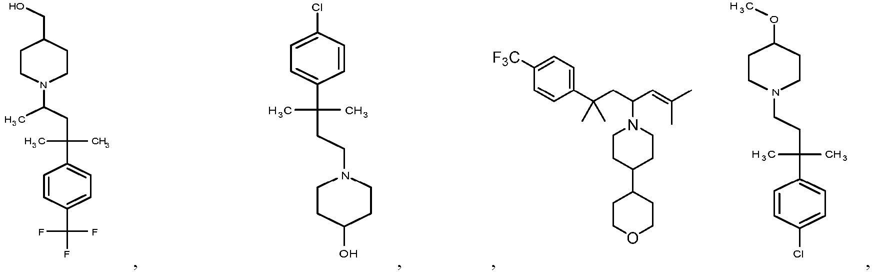

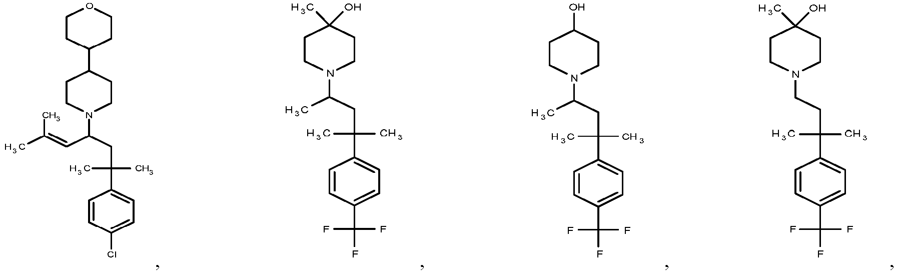

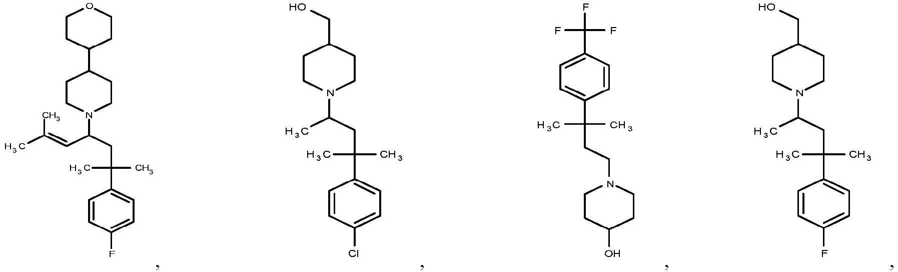

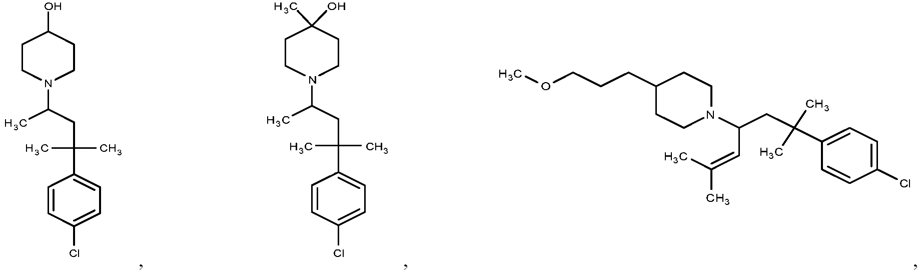

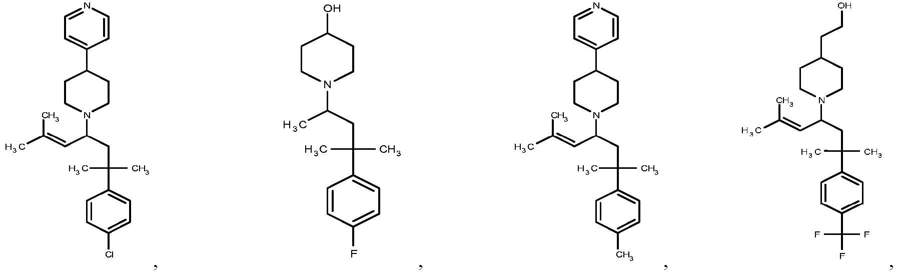

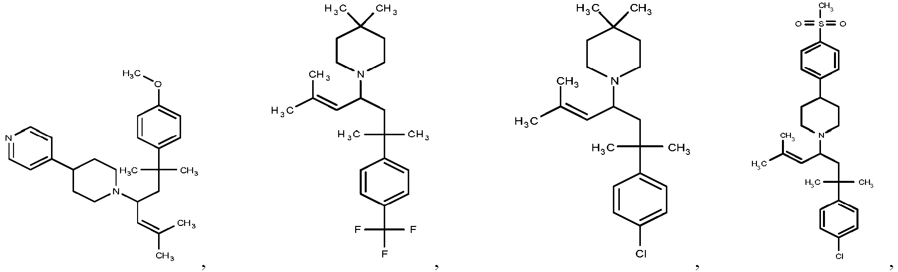

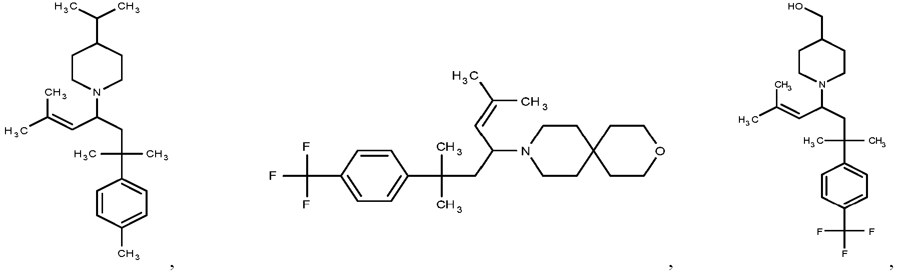

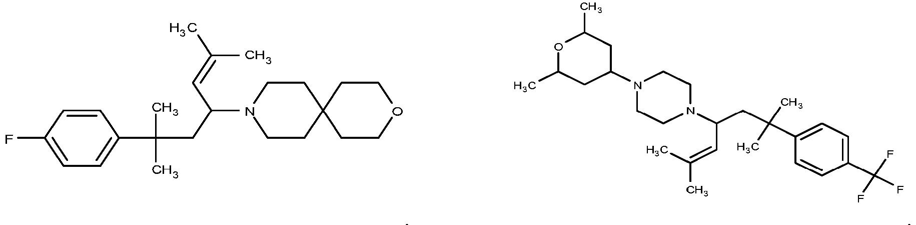

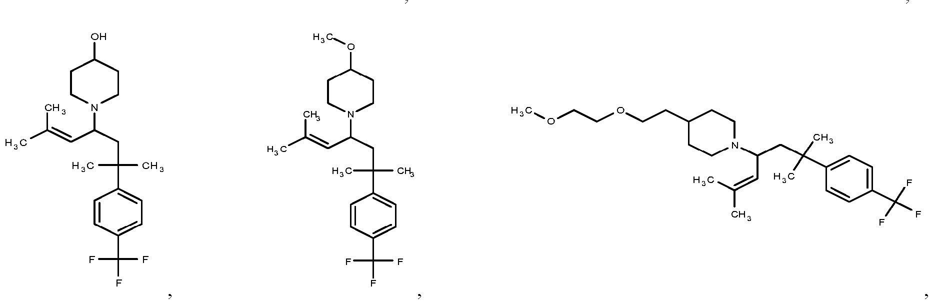

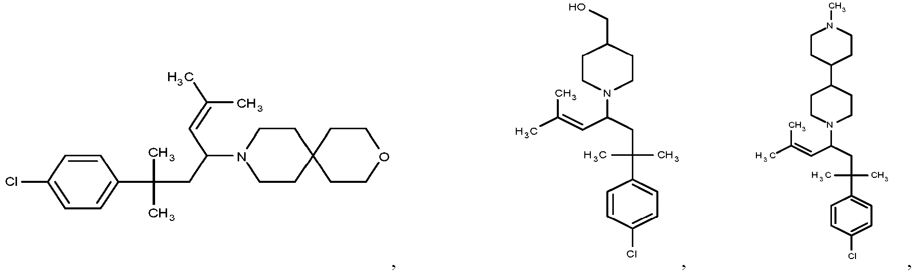

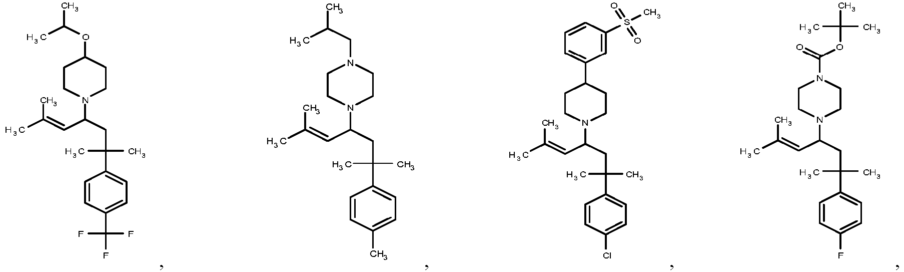

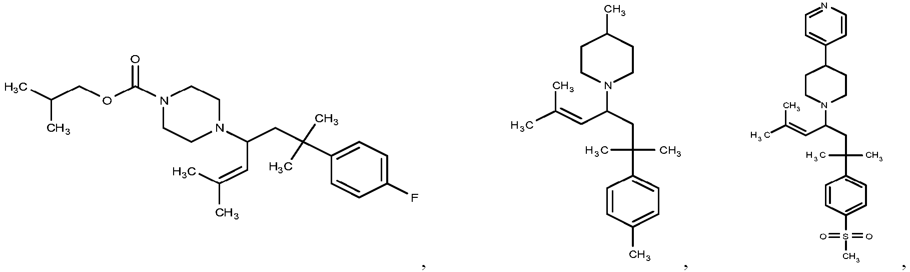

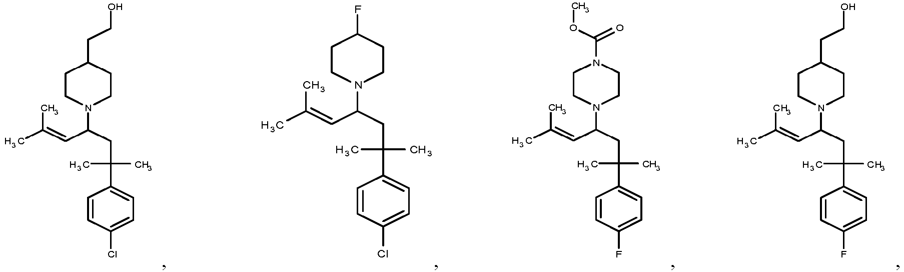

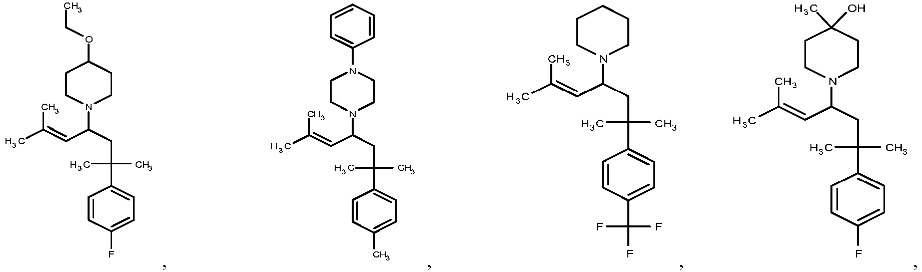

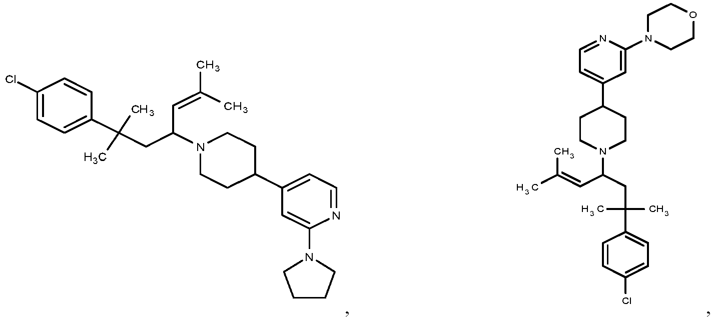

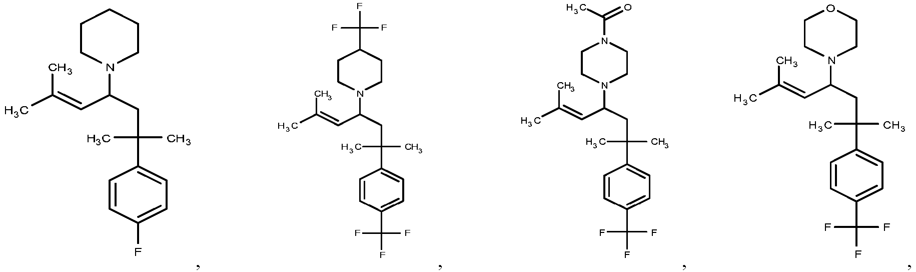

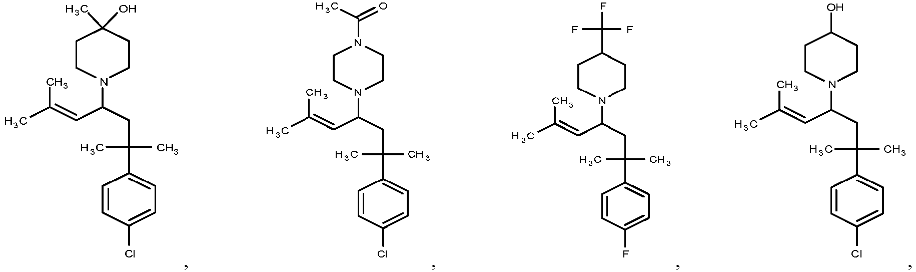

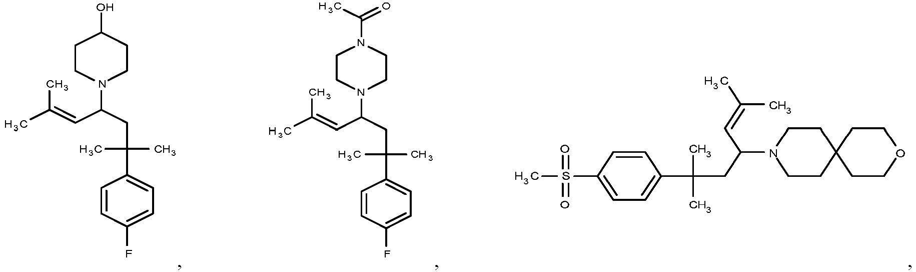

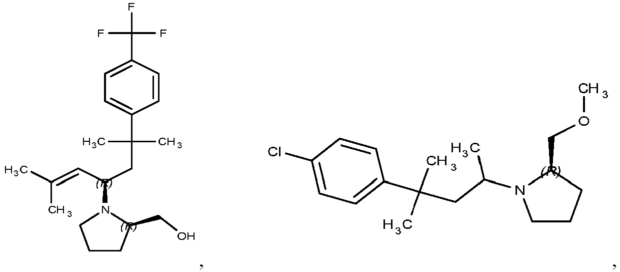

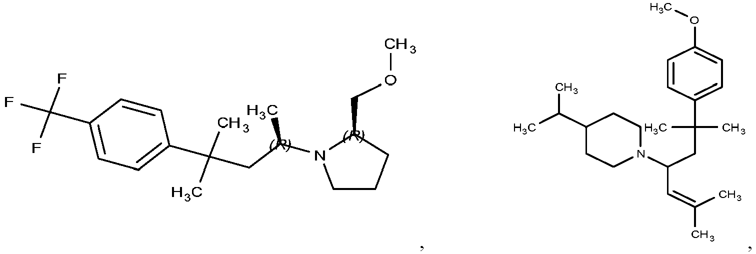

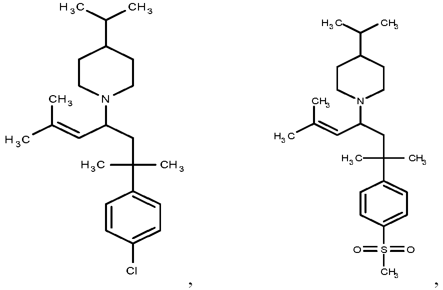

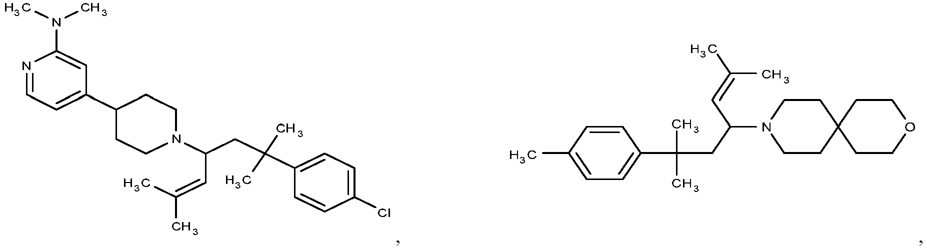

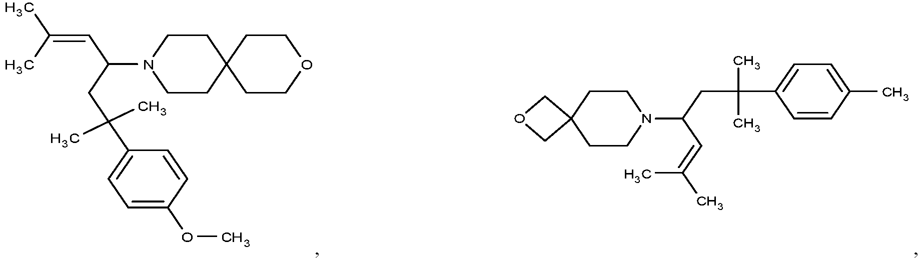

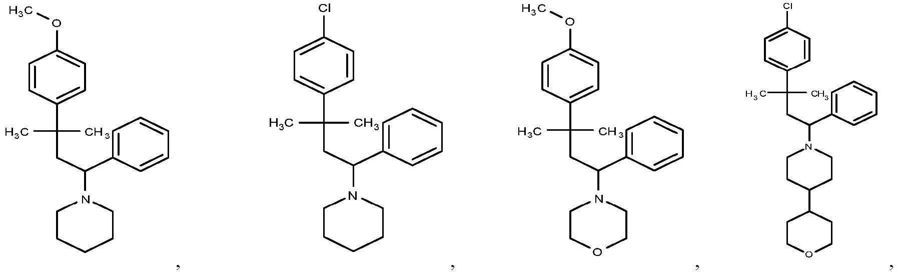

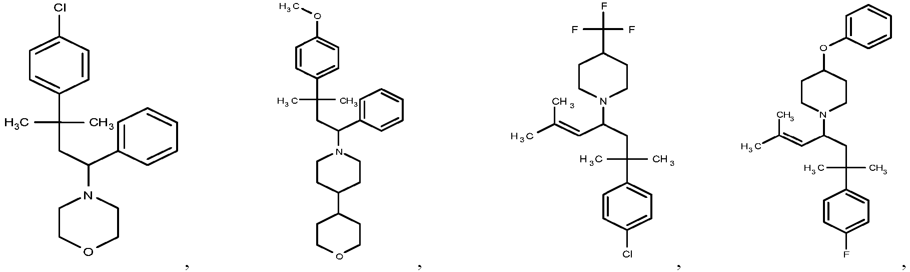

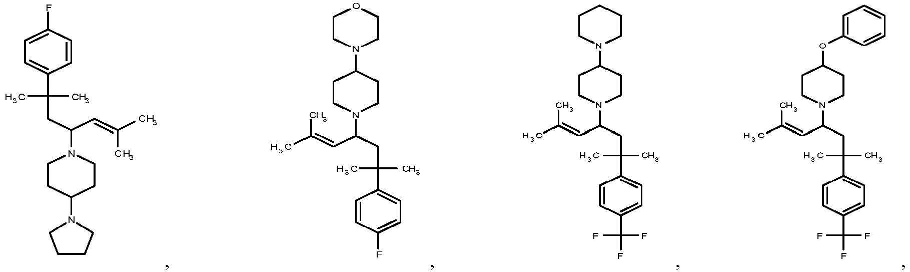

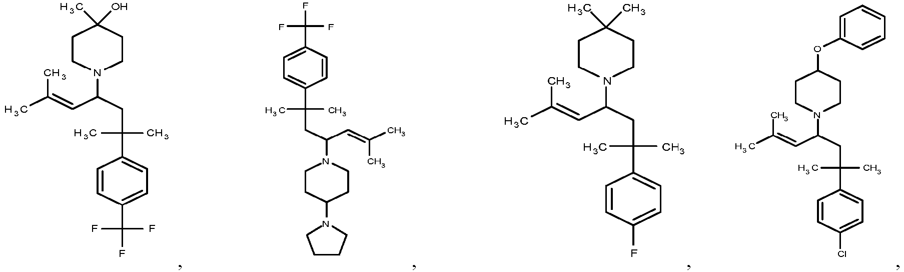

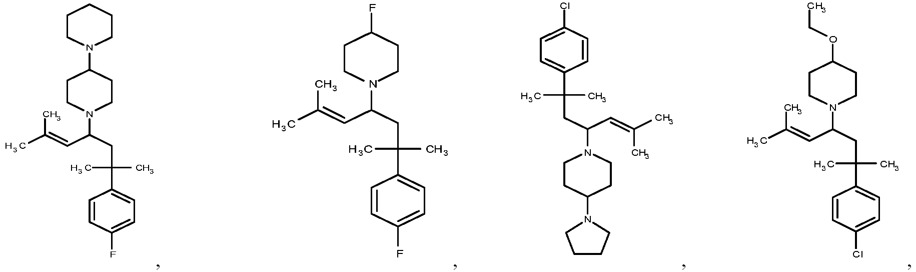

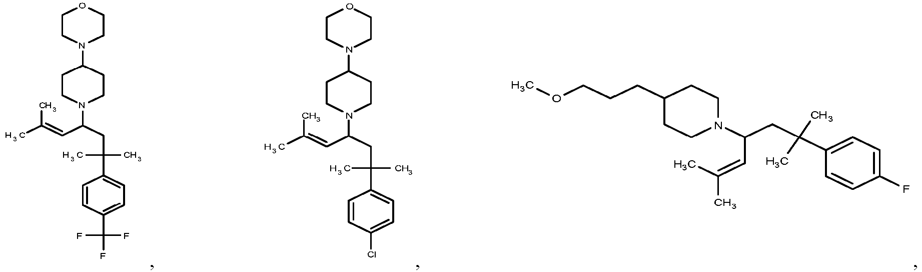

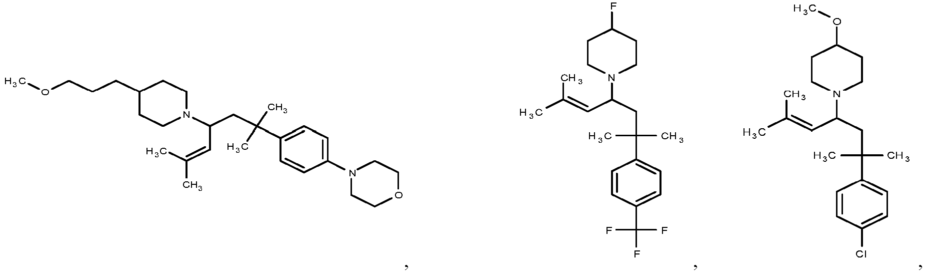

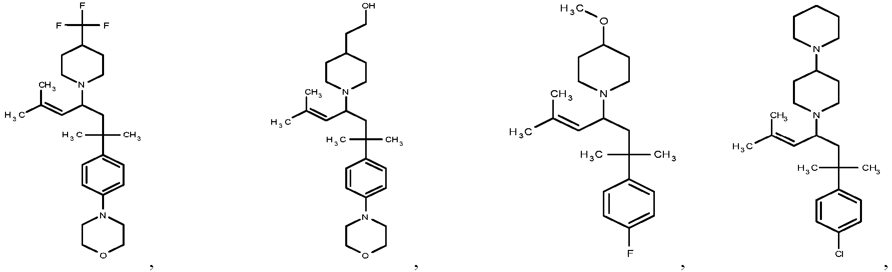

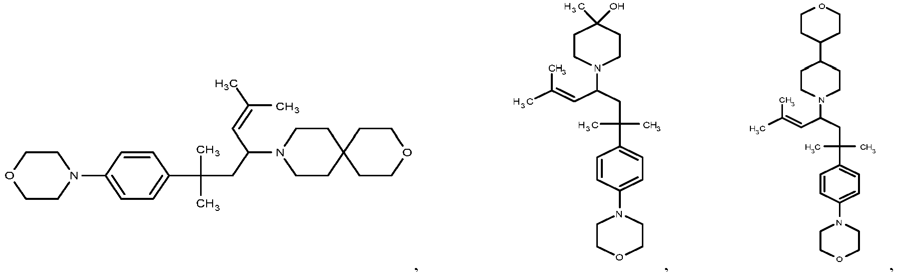

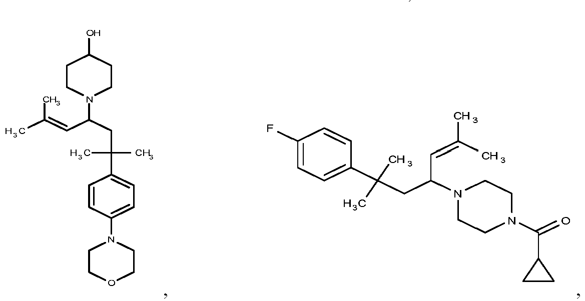

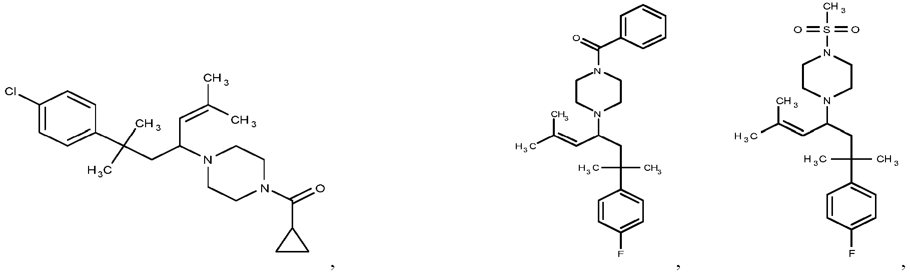

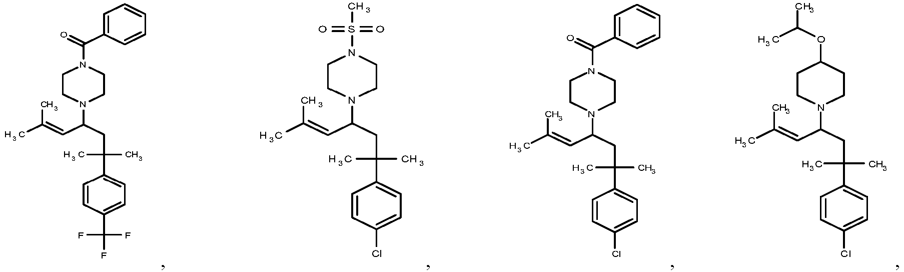

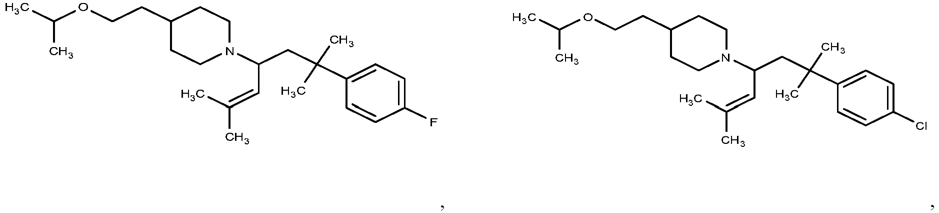

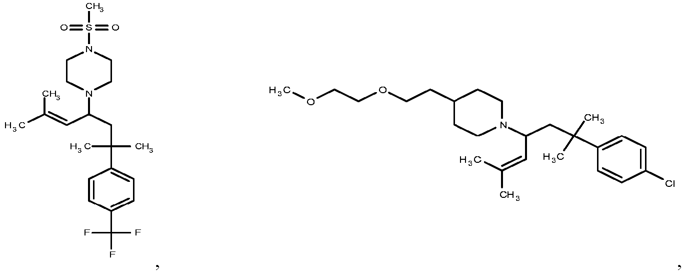

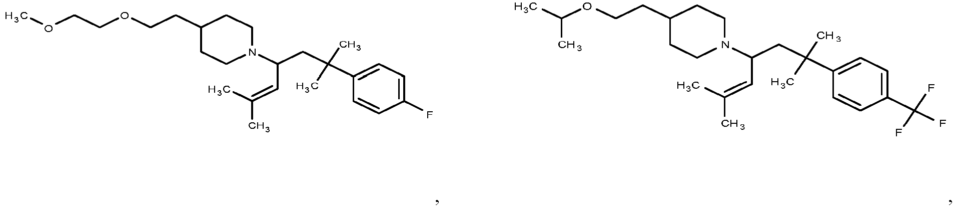

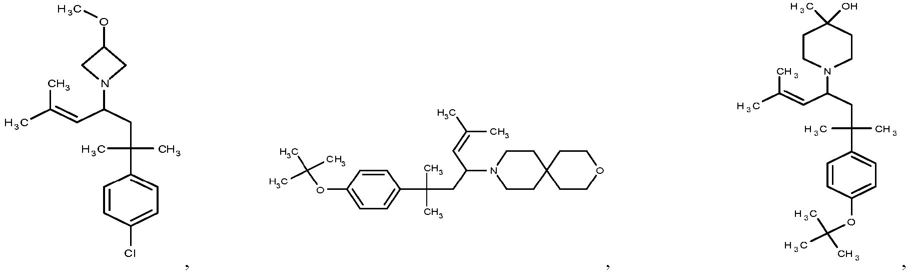

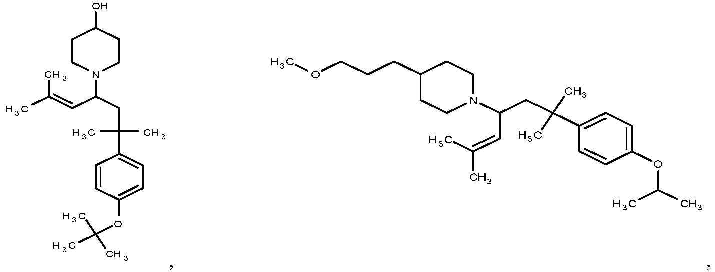

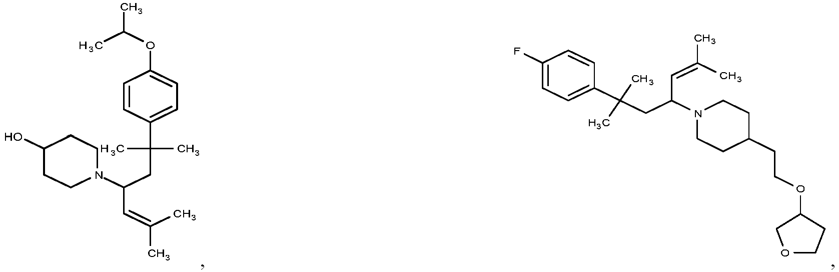

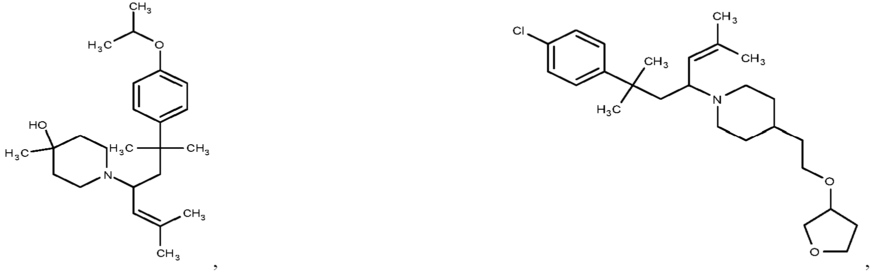

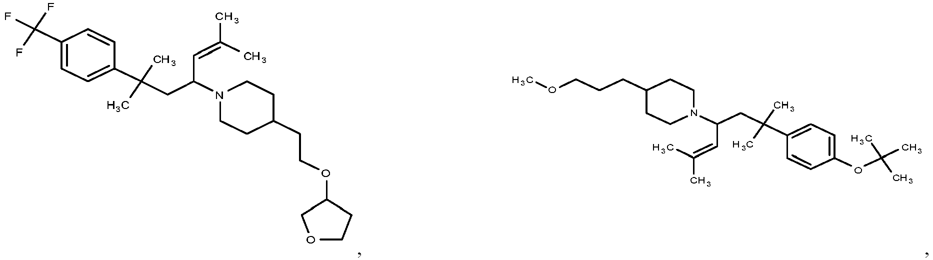

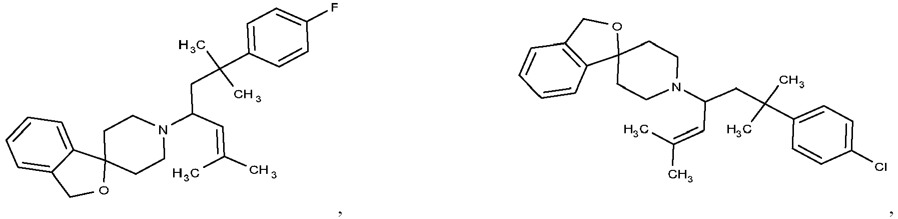

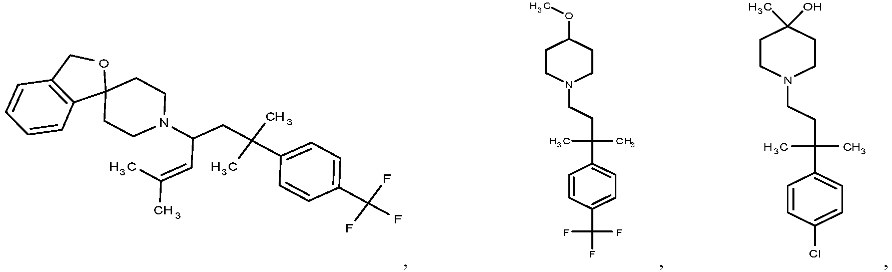

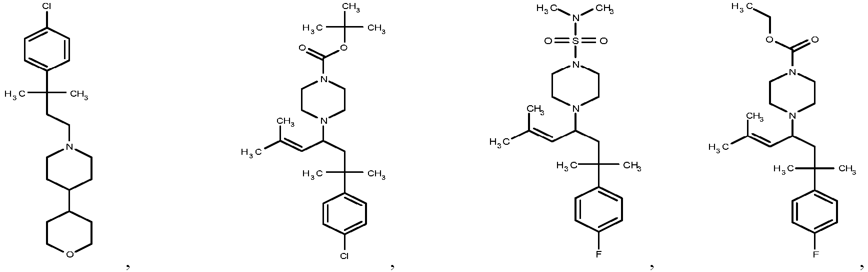

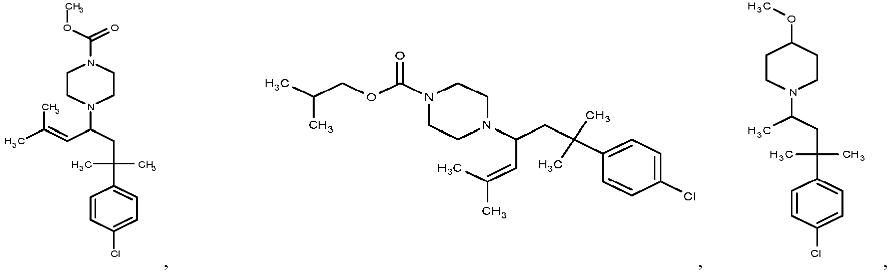

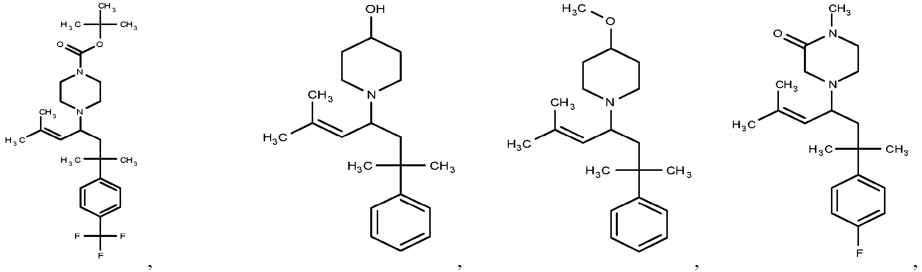

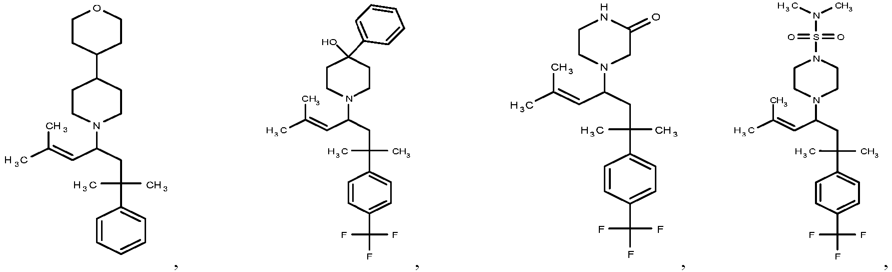

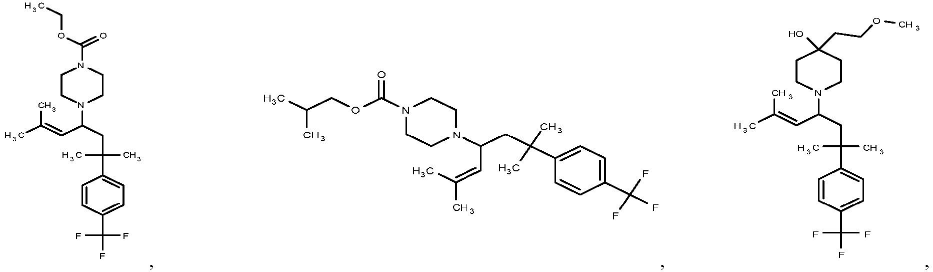

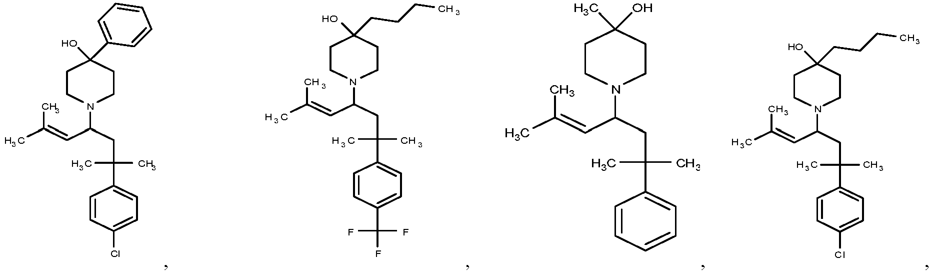

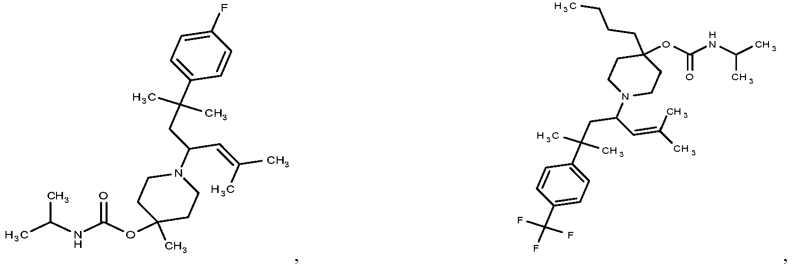

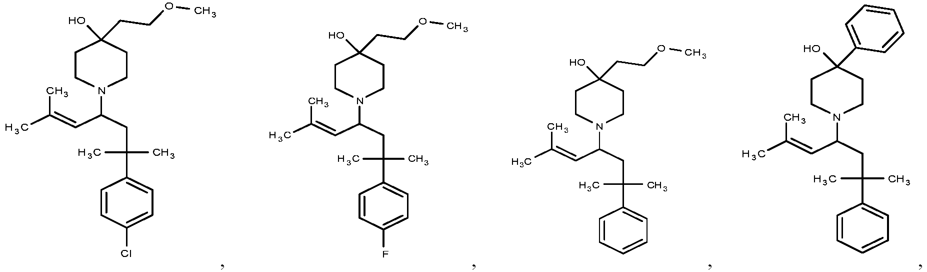

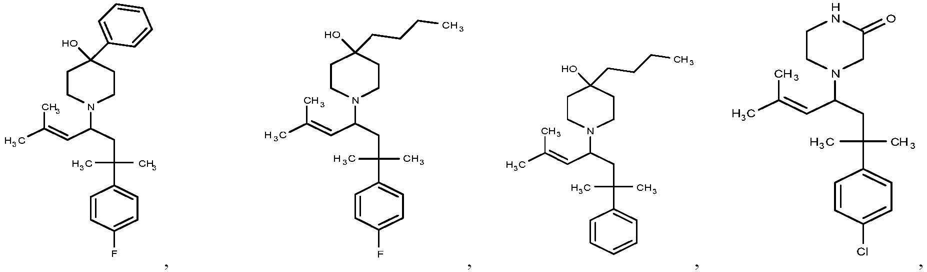

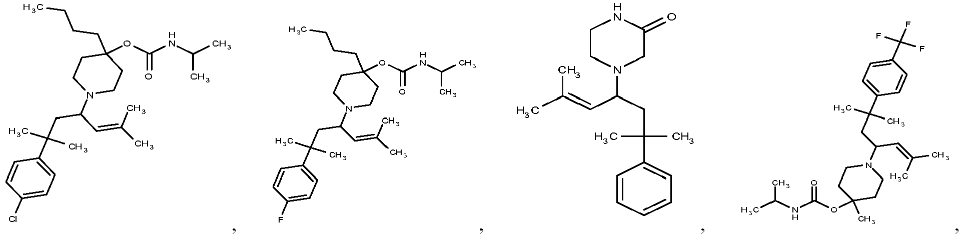

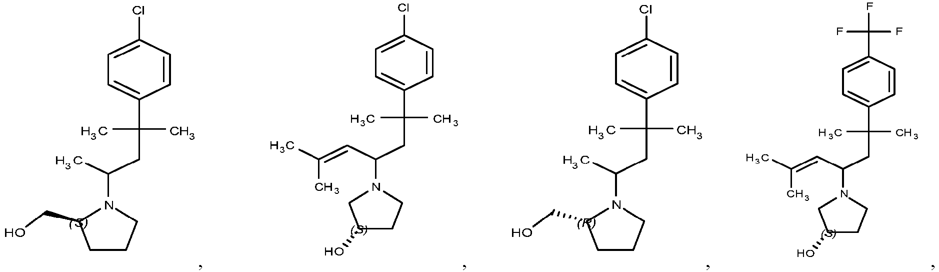

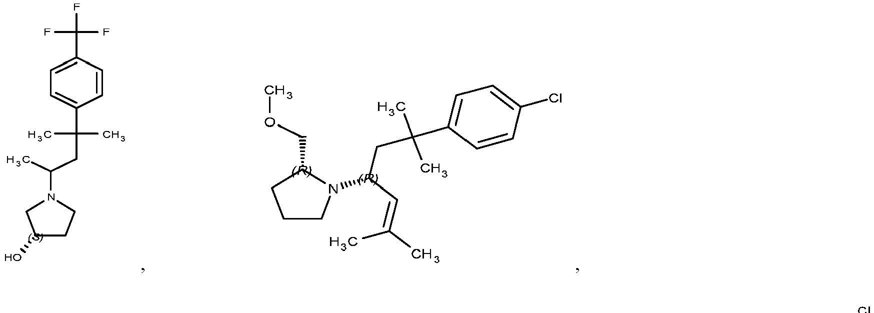

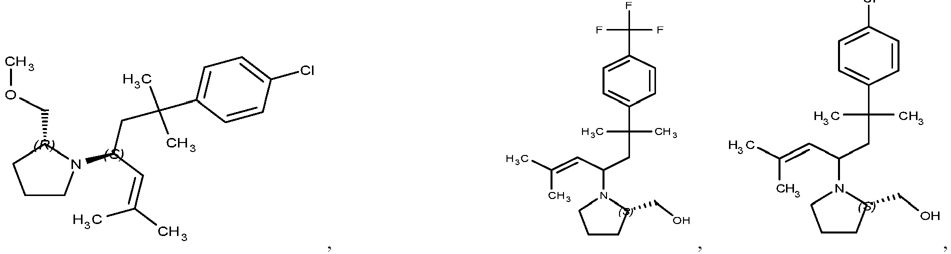

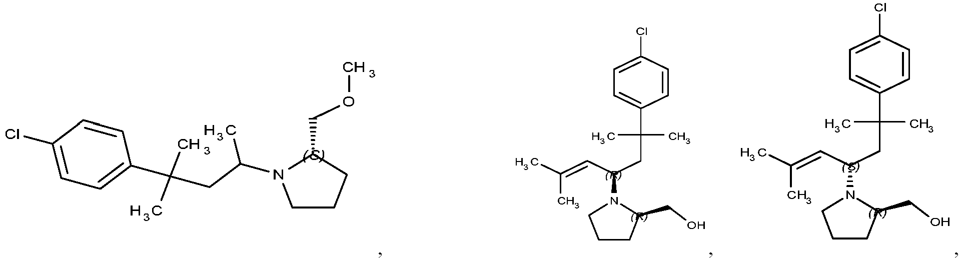

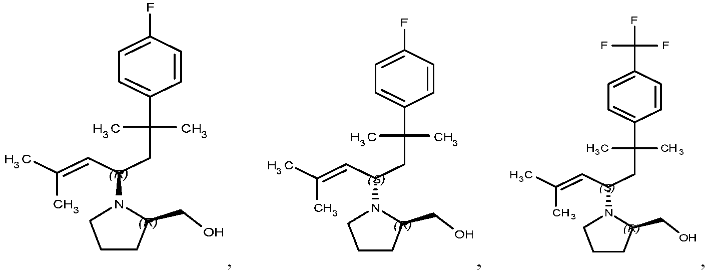

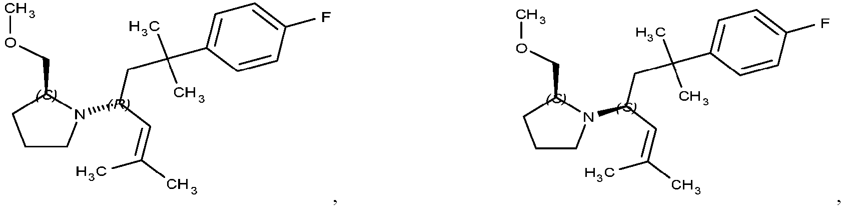

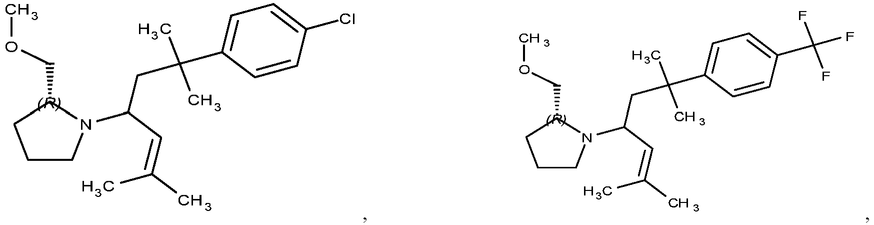

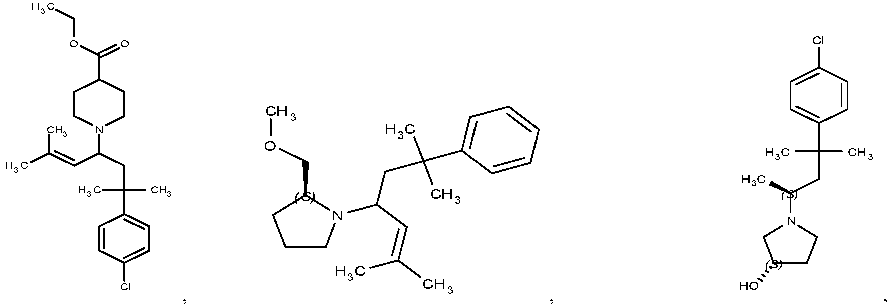

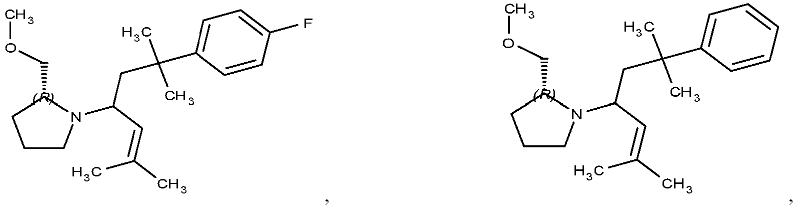

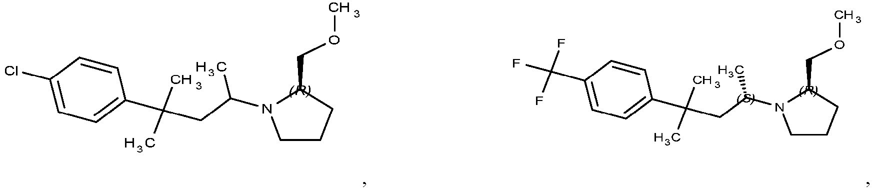

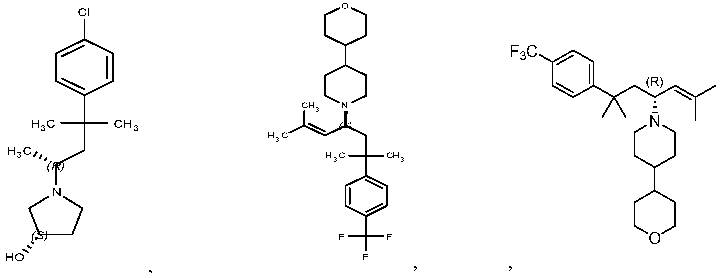

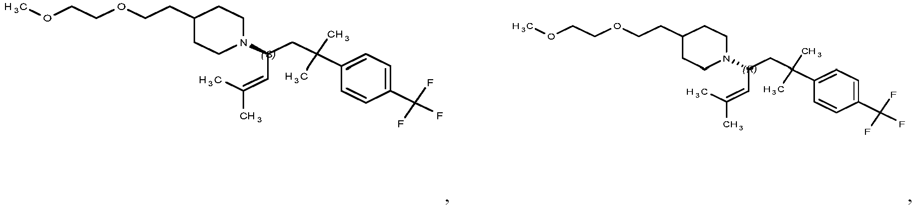

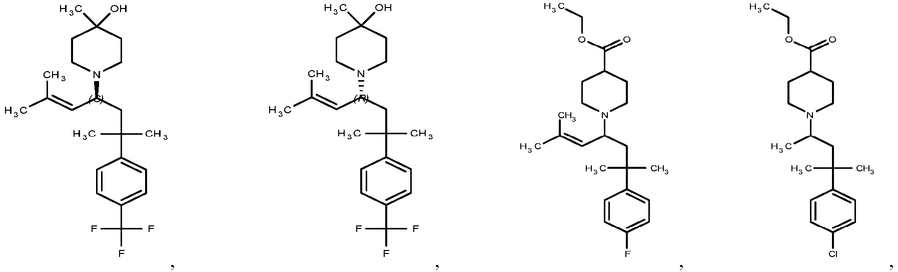

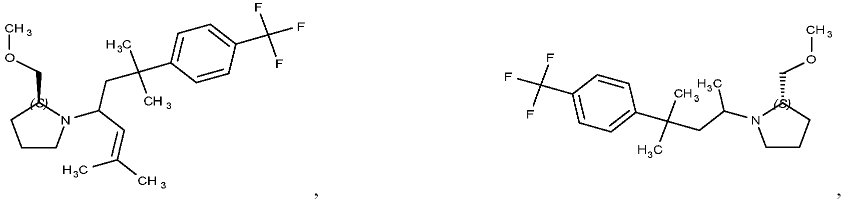

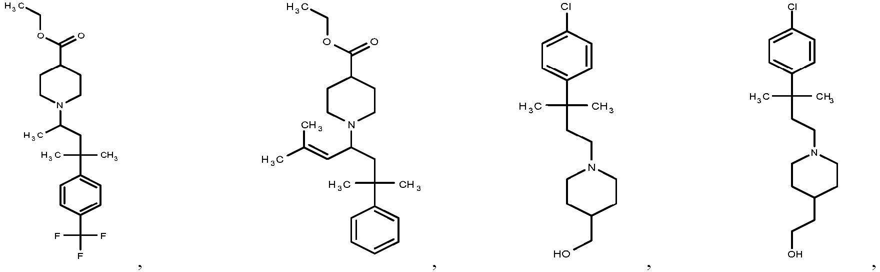

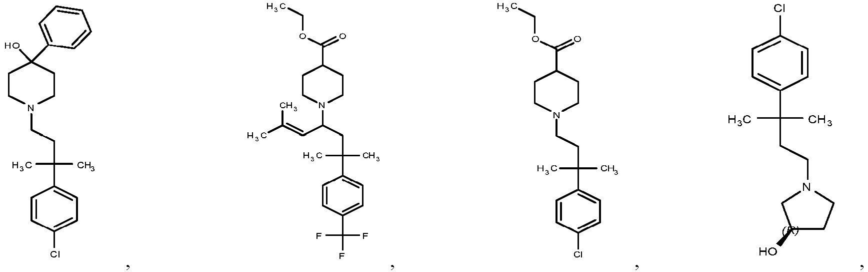

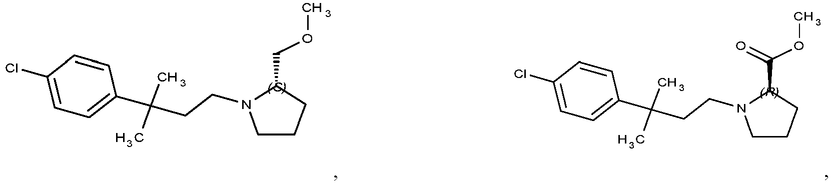

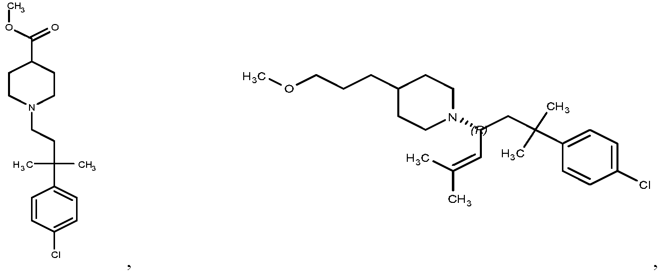

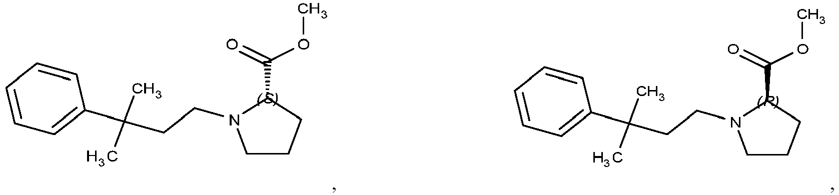

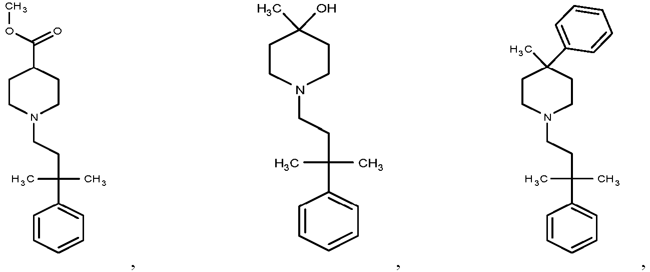

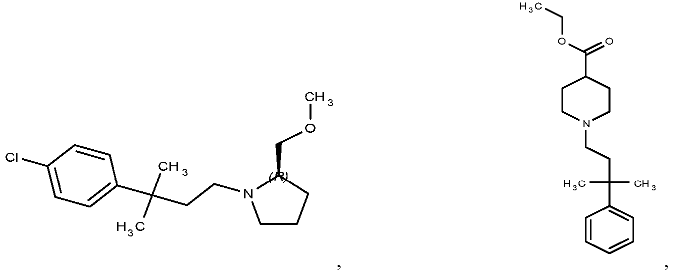

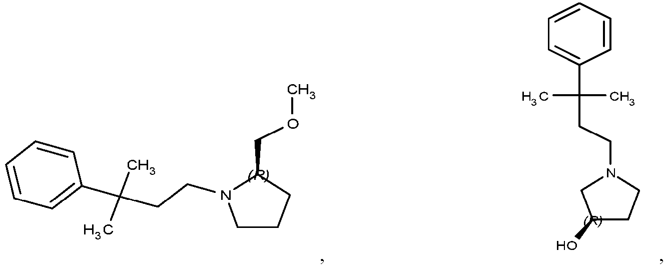

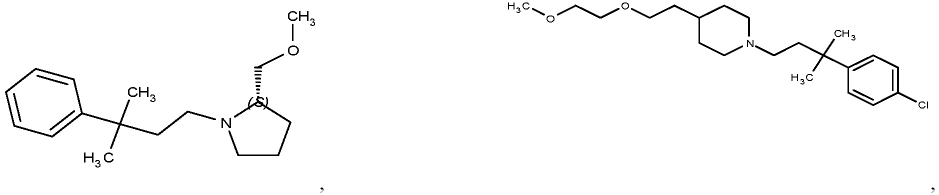

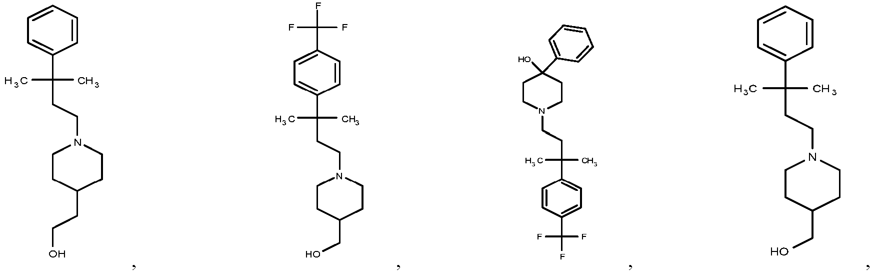

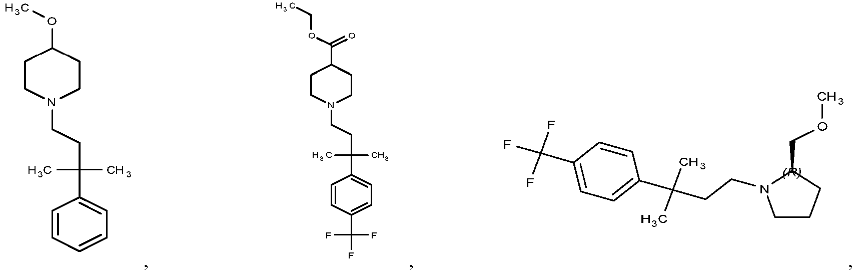

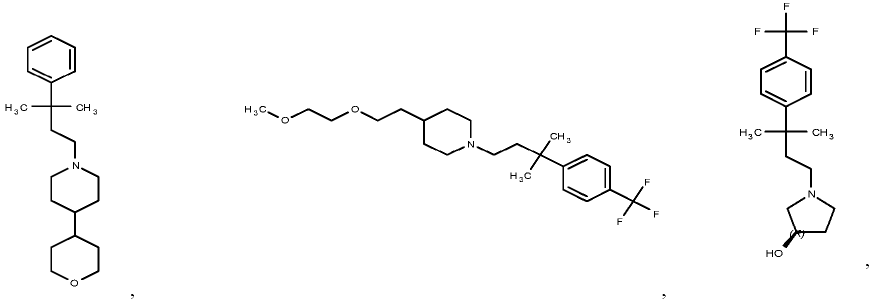

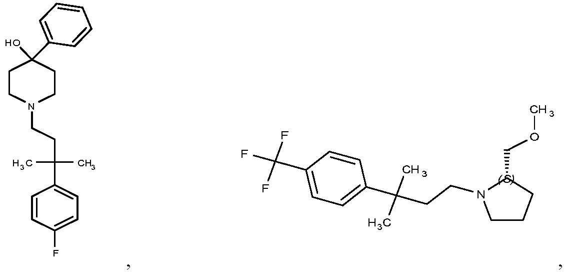

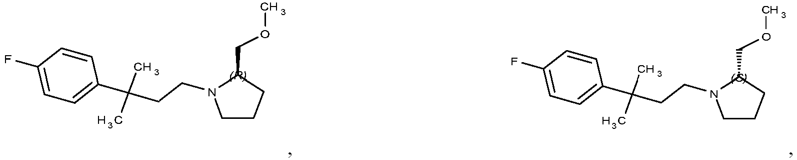

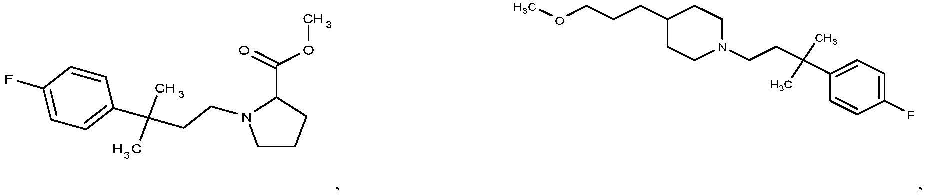

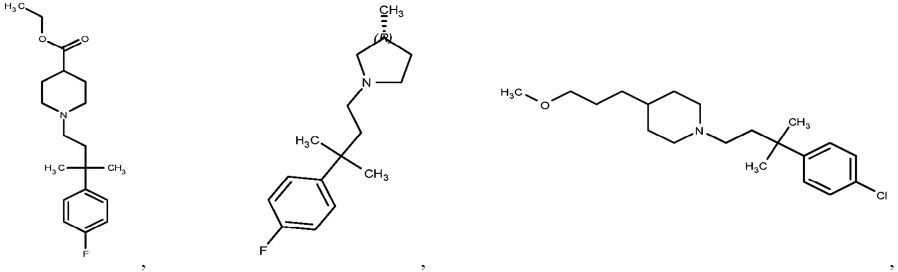

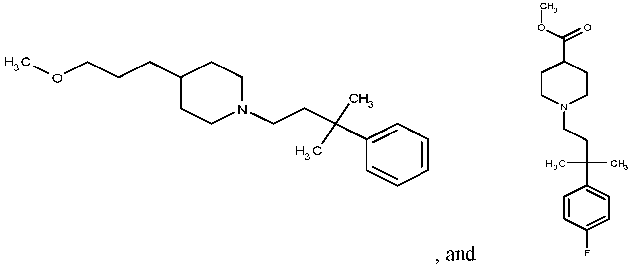

In some embodiments a compound for use in the invention is a compound selected from the group consisting of:

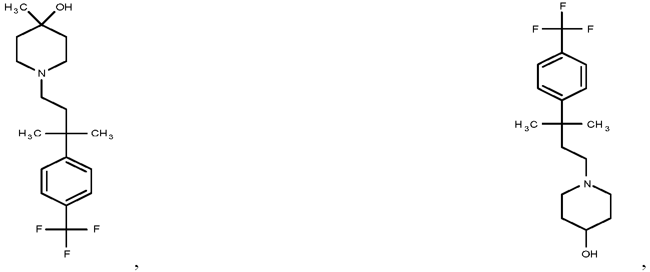

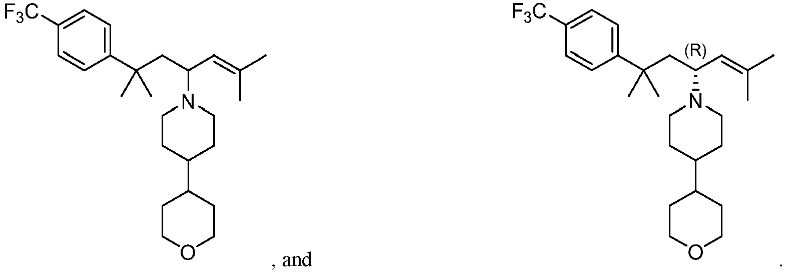

In some embodiments a compound for use in the invention is a compound of Formula IIA or pharmaceutically acceptable salt thereof:

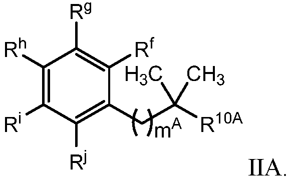

Each of substituents R^{f}, R^{g}, R^{h}, R and R^{j} of Formula IIA is independently selected from the group consisting of, H, hydroxyl, halo, alkyl, alkoxy, CF₃, SO₂CH₃, and morpholino.

Substituent R^{10A} of Formula IIA is an optionally substituted cyclic amino group and m^{A} is an integer from 0 to 3.

In some embodiments each of substituents R^{f}, R^{g}, R^{h}, Rⁱ and R^{j} of Formula IIA is independently selected from the group consisting of, H, hydroxyl, and alkoxy. In some embodiments each of substituents R^{f}, R^{g}, R^{h}, Rⁱ and R^{j} of Formula IIA is independently selected from the group consisting of, H, hydroxyl, and methoxy. In some embodiments each of substituents R^{f}, R^{g}, and R^{j} is H and each of R^{g}, and R^{h} is independently selected from the hydroxyl, or methoxy.

In some embodiments, R^{10A} of Formula IIA is an optionally substituted aziridinyl, optionally substituted pyrolidinyl, optionally substituted imidizolidinyl, optionally substituted piperidinyl, optionally substituted piperazinyl, optionally substituted oxopiperazinyl, or optionally substituted morpholinyl, and any of the individual substituted or unsubstituted piperdinyl, substituted or unsubstituted morpholinyl, substituted or unsubstituted piperazinyl, substituted or unsubstituted pyrrolidinyl, substituted or unsubstituted bicyclic, or substituted or unsubstituted fused rings described above in relation to Formula I.

In some embodiments, R^{10A} of Formula IIA is an optionally substituted fused ring, such as: 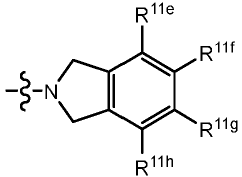
wherein each of R^{11e}, R^{11f}, R^{11g}, and R^{11h} is independently selected from, hydrogen, hydroxy, sulfonyl, optionally substituted C₁-C₁₀ alkyl, optionally substituted C₅-C₁₀ aryl optionally substituted C₃-C₁₀ heteroaryl, optionally substituted C₃-C₁₀ cycloalkyl or optionally substituted C₃-C₁₀ heterocycloalkyl. In certain embodiments R^{10A} is not 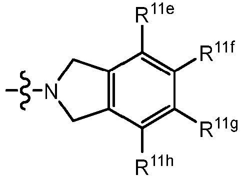
when m^{A} is 2.

In some embodiments, R^{10A} f Formula IIA is

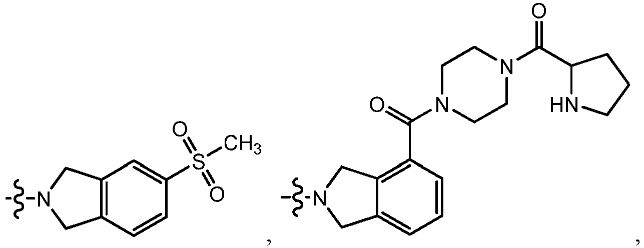

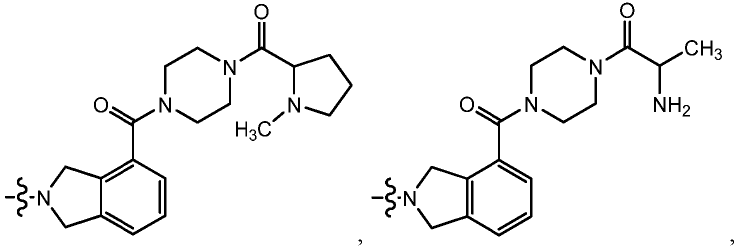

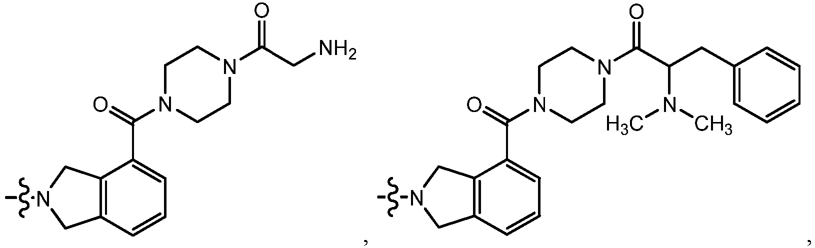

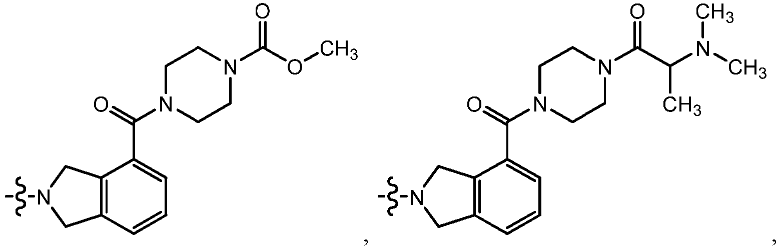

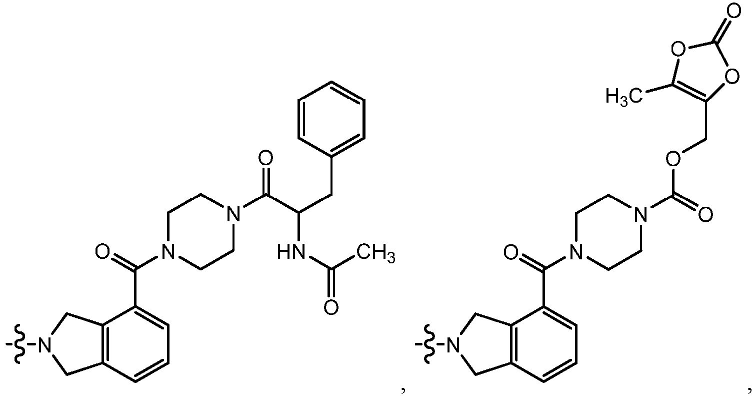

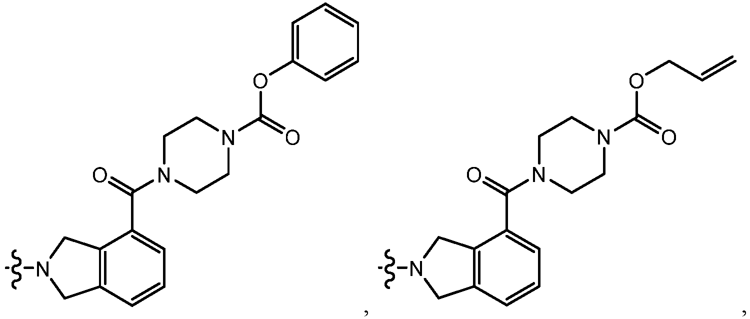

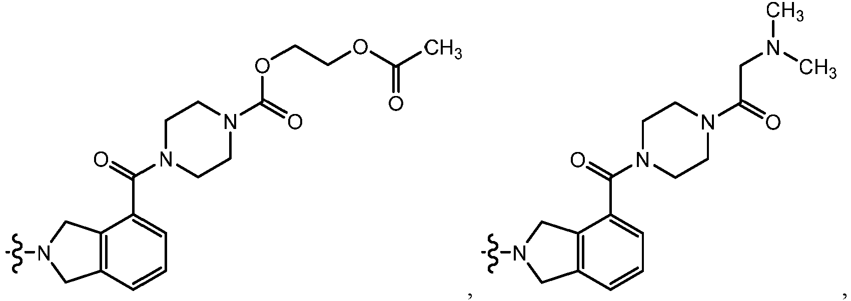

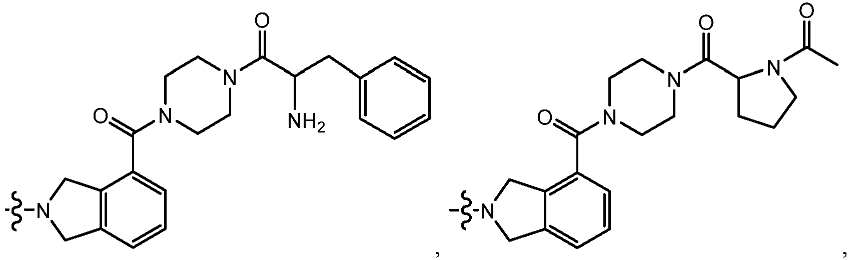

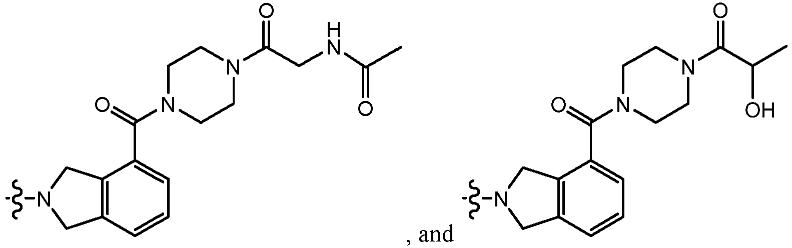

In some embodiments a compound for use in the invention is a compound of Formula IIa:

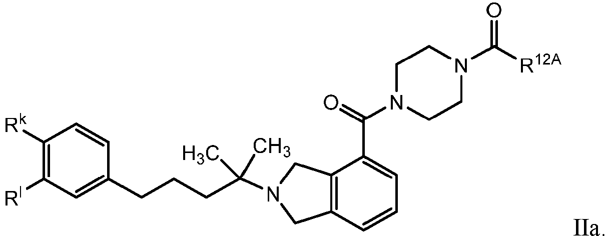

Each of substituents R^{k} and R^{l} of Formula IIa is independently selected from the group consisting of H, hydroxyl, halo, alkyl, alkoxy, CF₃, SO₂CH₃, and morpholino.

Substituent R^{12A} of Formula IIa is selected from the group consisting of aryloxy, alkenyloxy, alkoxy, aminoalkyl, N,N-dimethylaminoalkyl, pyrrolidinyl, n-methylpyrrolidinyl, N-acylpyrrolidinyl, carboxyaminoalkyl, hydroxyalkyl, - O(CH₂)₂OC(O)CH₃, 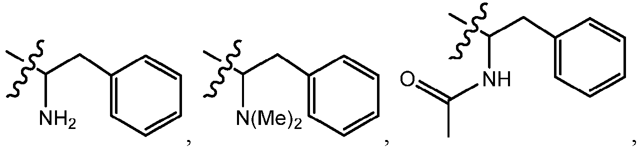 and

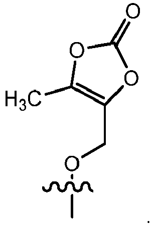

In some embodiments each of substituents R^{k} and R^{l} of Formula IIa is independently selected from the group consisting of H, hydroxyl and methoxy. In some embodiments R^{1A} is methoxy and R^{k} is hydroxyl.

In some embodiments substituent R^{12A} of Formula IIa is selected from the group consisting of phenyloxy, -OCH₂CH=CH₂, methoxy, -CH₂NH₂, -CH(NH₂)CH₃, - CH₂N(Me)₂, -CH(CH₃)N(Me)₂, -CH₂NHC(O)CH₃, -CH(OH)CH₃, -O(CH₂)₂OC(O)CH₃,

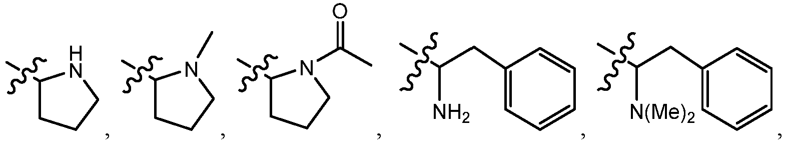

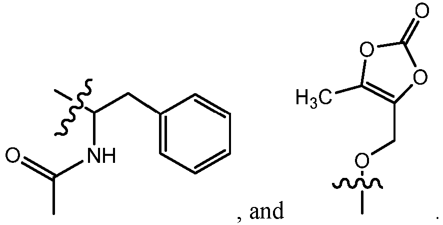

In some embodiments a compound for use in the invention is a compound selected from the group consisting of:

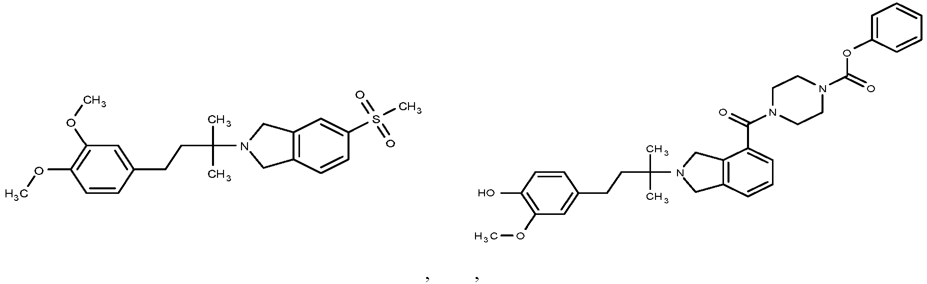

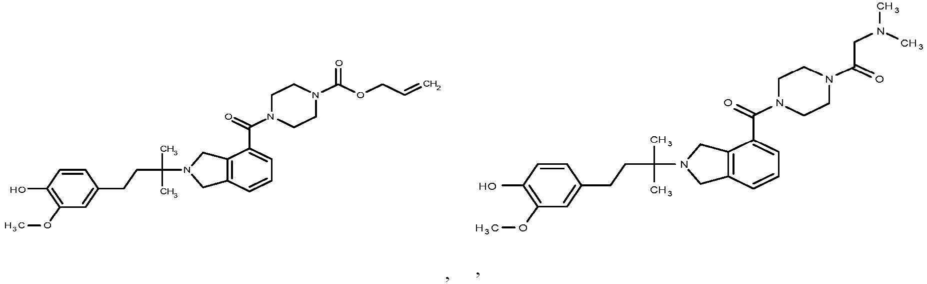

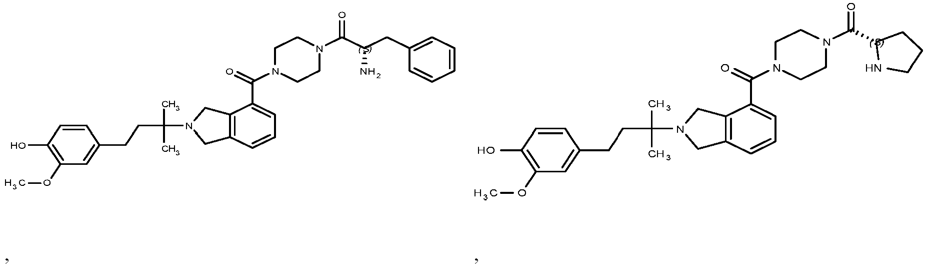

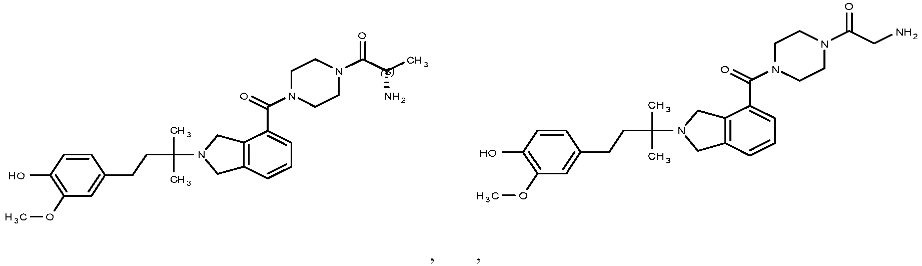

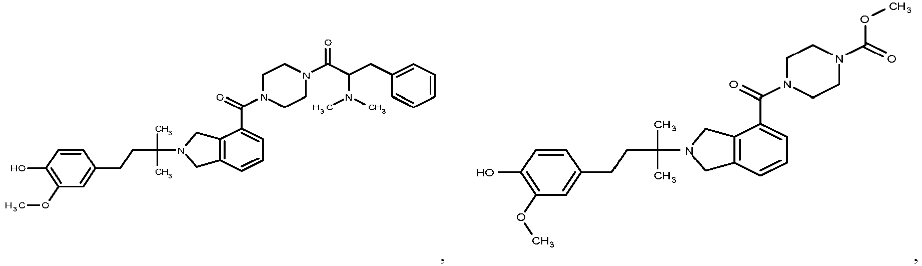

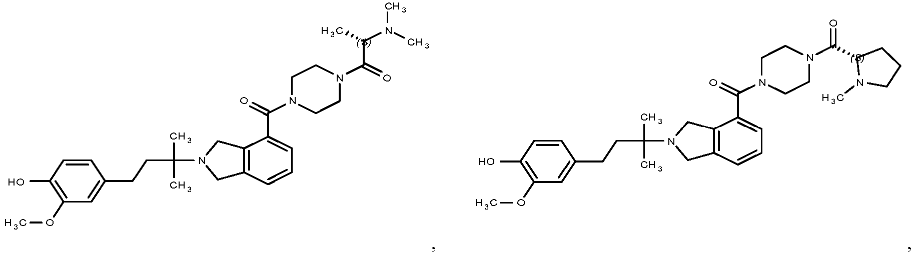

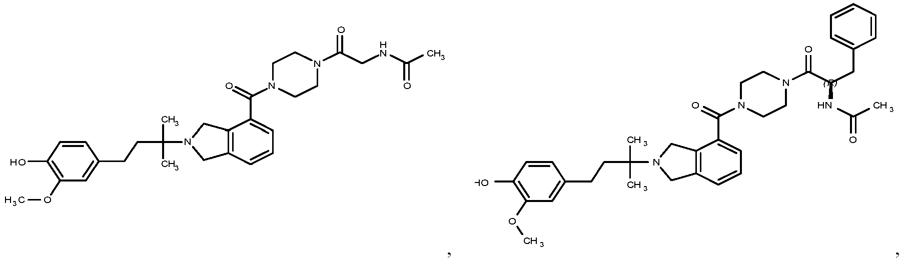

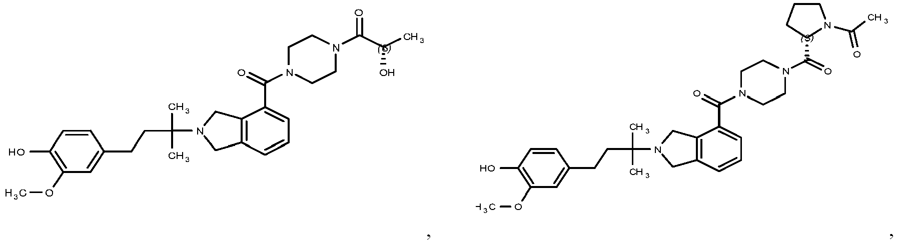

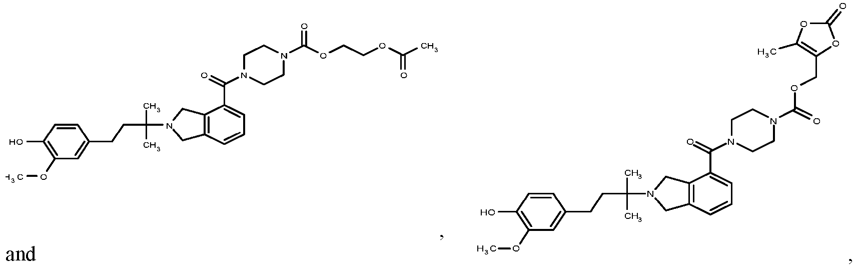

In some embodiments a compound for use in the invention is a compound selected from the group consisting of:

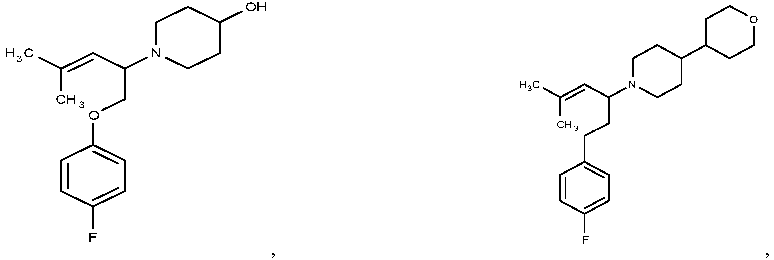

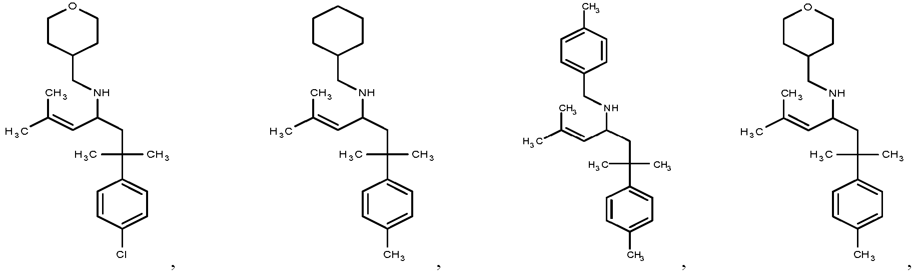

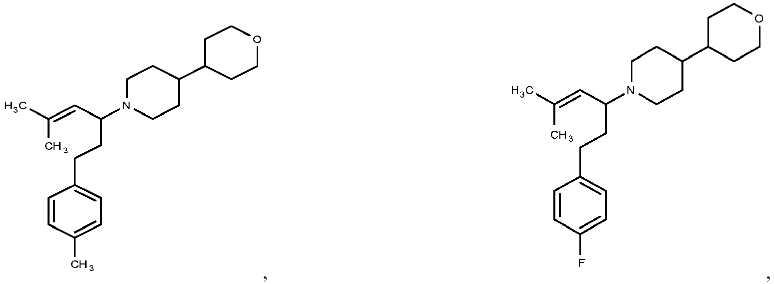

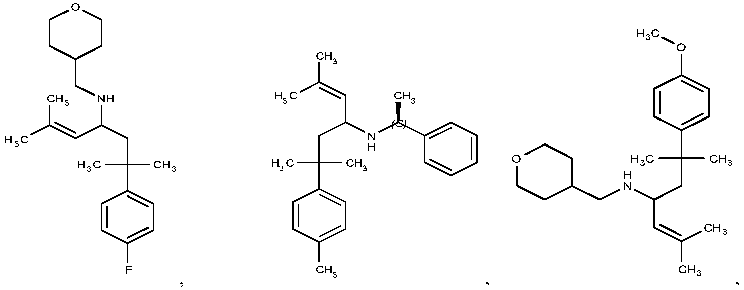

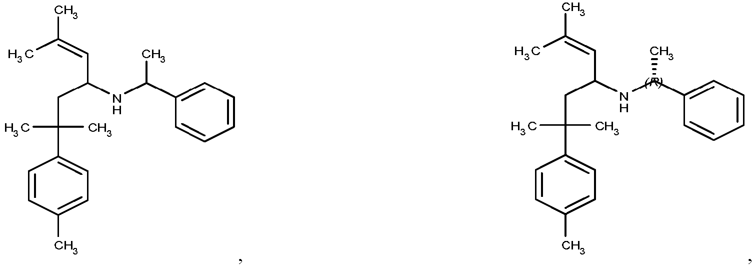

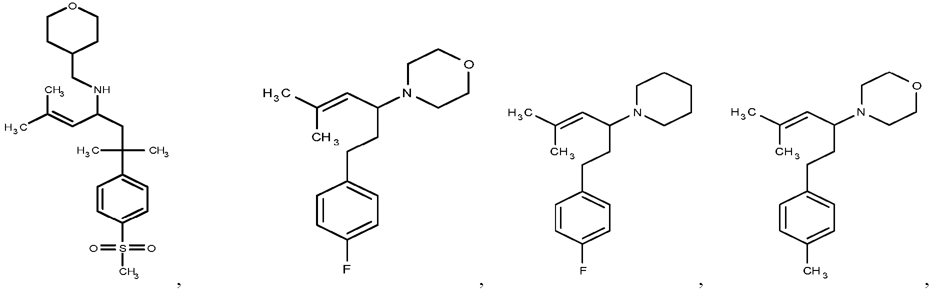

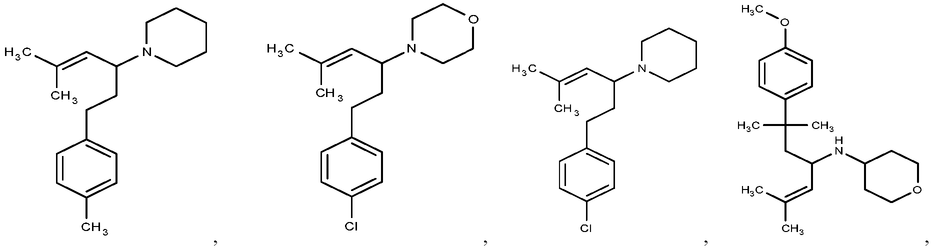

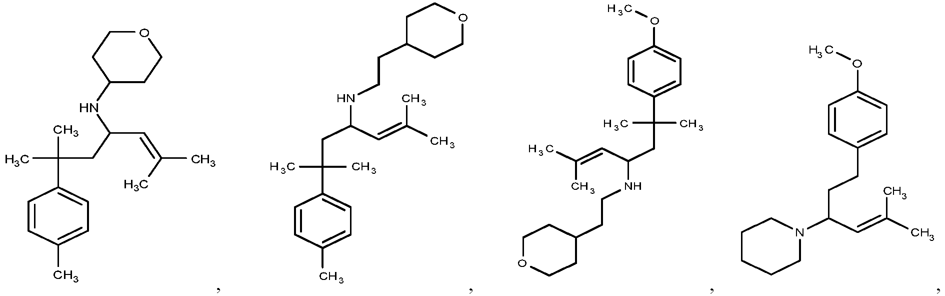

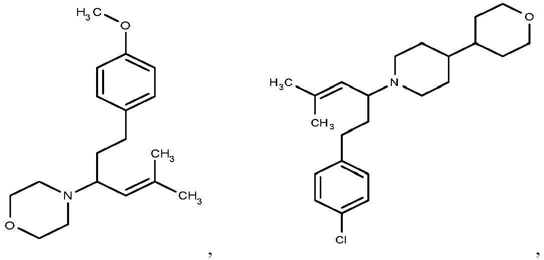

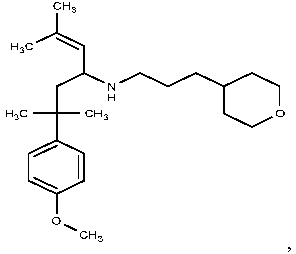

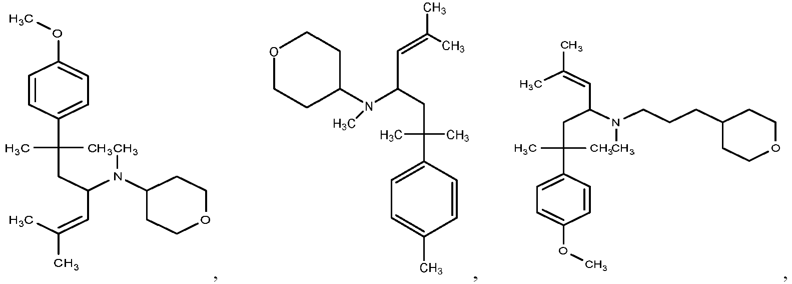

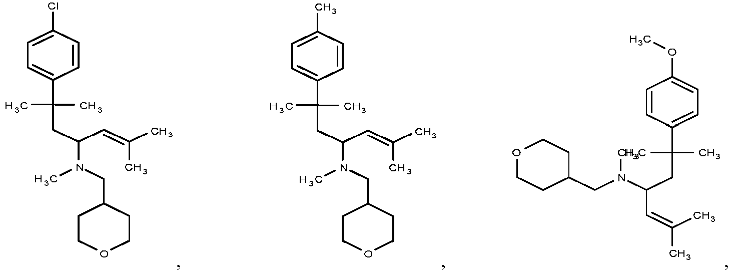

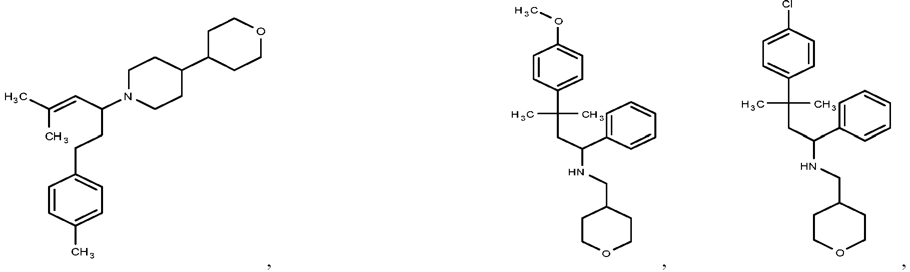

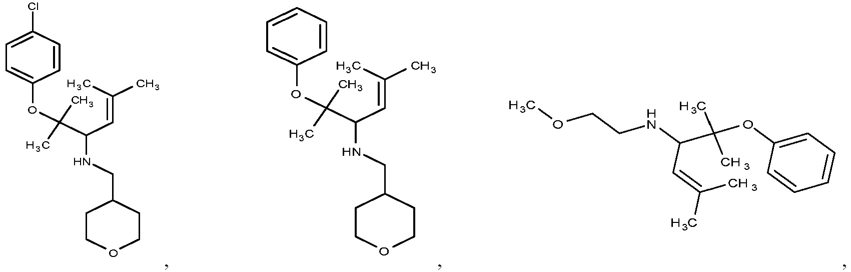

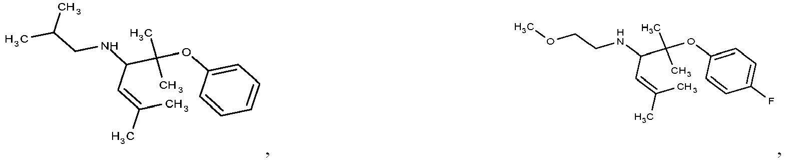

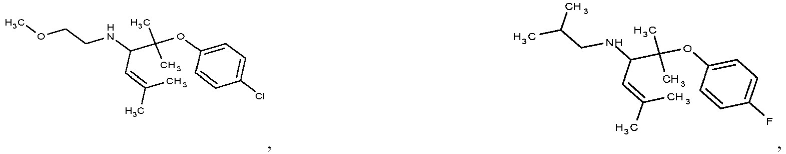

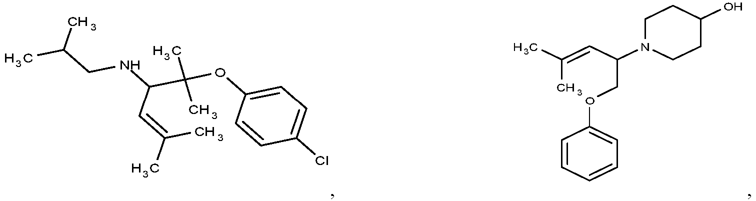

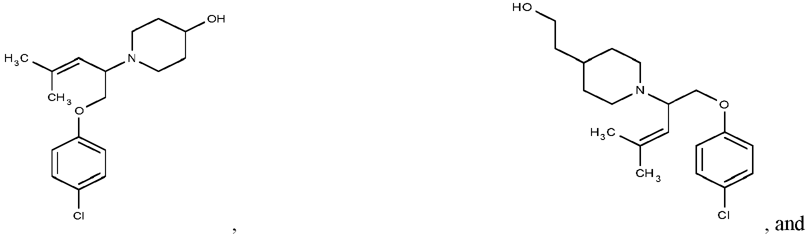

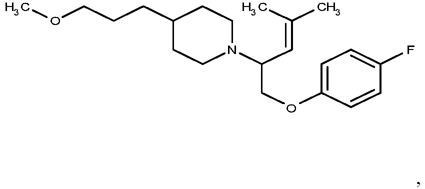

Additional embodiments include salts, solvates, stereoisomers, prodrugs, and active metabolites of the compounds according to any embodiment described herein.

Some embodiments are directed to free base forms of the compounds according to any embodiment described herein. Other embodiments include salts of such compounds including, for example, pharmaceutically acceptable acid addition salts or pharmaceutically acceptable addition salts of free bases. Examples of pharmaceutically acceptable acid addition salts include, but are not limited to, salts derived from nitric, phosphoric, sulfuric, or hydrobromic, hydroiodic, hydrofluoric, phosphorous, as well as salts derived from nontoxic organic acids such as aliphatic mono- and dicarboxylic acids, phenyl-substituted alkanoic acids, hydroxyl alkanoic acids, alkanedioic acids, aromatic acids, aliphatic and aromatic sulfonic acids, and acetic, maleic, succinic, or citric acids. Nonlimiting examples of such salts include napadisylate, besylate, sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, trifluoroacetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate, and the like. Additional salt forms of the compounds described above include salts of amino acids such as arginate and the like and gluconate, galacturonate (*see e.g.,* Berge, et al. "Pharmaceutical Salts," J. Pharma. Sci. 1977;66: 1).

Pharmaceutically acceptable base addition salts are formed with metals or amines, such as alkali and alkaline earth metals or organic amines. Examples of metals used as cations are sodium, potassium, magnesium, calcium, and the like. Examples of suitable amines include N,N'-dibenzylethylenediamine, chloroprocaine, choline, diethanolamine, dicyclohexylamine, ethylenediamine, N-methylglucamine, and procaine. The base addition salts of said acidic compounds are prepared by contacting the free acid form with a sufficient amount of the desired base to produce the salt in the conventional manner. The free acid form may be regenerated by contacting the salt form with an acid and isolating the free acid.

Various embodiments include total and partial salts, *i.e.* salts with 1, 2 or 3, preferably 2, equivalents of base per mole of acid of a compound or salt described above, with 1, 2 or 3 equivalents, preferably 1 equivalent, of acid per mole of base of a compound of according to any embodiment described herein. Typically, a pharmaceutically acceptable salt of a compound according to any embodiment described herein, may be readily prepared by using a desired acid or base as appropriate. The salt may precipitate from solution and be collected by filtration or may be recovered by evaporation of the solvent. For example, an aqueous solution of an acid such as hydrochloric acid may be added to an aqueous suspension of a compound according to any embodiment described herein, and the resulting mixture evaporated to dryness (lyophilized) to obtain the acid addition salt as a solid. Alternatively, a compound according to any embodiment described herein, may be dissolved in a suitable solvent, for example an alcohol such as isopropanol, and the acid may be added in the same solvent or another suitable solvent. The resulting acid addition salt may then be precipitated directly, or by addition of a less polar solvent such as diisopropyl ether or hexane, and isolated by filtration.

Many organic compounds can form complexes with solvents in which they are reacted or from which they are precipitated or crystallized. These complexes are known as "solvates." For example, a complex with water is known as a "hydrate." Various embodiments include solvates of a compound according to any embodiment described herein. In some embodiments, salts of these compounds can form solvates.

Further embodiments include N-oxides of the compounds according to any embodiment described herein. N-oxides include heterocycles containing an otherwise unsubstituted sp² N atom. Examples of such N-oxides include pyridyl N-oxides, pyrimidyl N-oxides, pyrazinyl N-oxides and pyrazolyl N-oxides.

Compounds according to any embodiment described herein, may have one or more chiral centers and, depending on the nature of individual substituents, they can also have geometrical isomers. Thus, embodiments include stereoisomers, diastereomers, and enantiomers of the compounds according to any embodiment described herein. A chiral compound can exist as either an individual enantiomer or as a mixture of enantiomers. A mixture containing equal proportions of the enantiomers is called a "racemic mixture." A mixture containing unequal portions of the enantiomers is described as having an "enantiomeric excess" (ee) of either the R or S compound. The excess of one enantiomer in a mixture is often described with a % enantiomeric excess. The ratio of enantiomers can also be defined by "optical purity" wherein the degree at which the mixture of enantiomers rotates plane polarized light is compared to the individual optically pure R and S compounds. The compounds can also be a substantially pure (+) or (-) enantiomer of the compounds described herein. In some embodiments, a composition can include a substantially pure enantiomer that is at least 90%, 91%, 92%, 93%, 94%, 95%, 96%, 97%, 98%, or 99% of one enantiomer. In certain embodiments, a composition may include a substantially pure enantiomer that is at least 99.5% one enantiomer.

The description above encompasses all individual isomers of the compounds according to any embodiment described herein, and the description or naming of a particular compound in the specification and claims is intended to include both individual enantiomers and mixtures thereof. Methods for the determination of stereochemistry and the resolution or stereotactic synthesis of stereoisomers are well-known in the art. Diastereomers differ in both physical properties and chemical reactivity. A mixture of diastereomers can be separated into enantiomeric pairs based on solubility, fractional crystallization or chromatographic properties, e.g., thin layer chromatography, column chromatography or HPLC. Purification of complex mixtures of diastereomers into enantiomers typically requires two steps. In a first step, the mixture of diastereomers is resolved into enantiomeric pairs, as described above. In a second step, enantiomeric pairs are further purified into compositions enriched for one or the other enantiomer or, more preferably resolved into compositions comprising pure enantiomers. Resolution of enantiomers typically requires reaction or molecular interaction with a chiral agent, e.g. solvent or column matrix. Resolution may be achieved, for example, by converting the mixture of enantiomers, e.g., a racemic mixture, into a mixture of diastereomers by reaction with a pure enantiomer of a second agent, i.e., a resolving agent. The two resulting diastereomeric products can then be separated. The separated diastereomers are then reconverted to the pure enantiomers by reversing the initial chemical transformation.

Resolution of enantiomers can also be accomplished by differences in their non-covalent binding to a chiral substance, e.g., by chromatography on homochiral adsorbants. The noncovalent binding between enantiomers and the chromatographic adsorbant establishes diastereomeric complexes, leading to differential partitioning in the mobile and bound states in the chromatographic system. The two enantiomers therefore move through the chromatographic system, e.g. column, at different rates, allowing for their separation

Further embodiments include prodrugs of the compounds according to any embodiment described herein, *i.e.* compounds which release an active compound according to any of the embodiments described herein, *in vivo* when administered to a mammalian subject. A prodrug is a pharmacologically active or more typically an inactive compound that is converted into a pharmacologically active agent by a metabolic transformation. Prodrugs of a compound according to any embodiment described herein, are prepared by modifying functional groups present in the compound according to any embodiment described herein, in such a way that the modifications may be cleaved *in vivo* to release the parent compound. *In vivo,* a prodrug readily undergoes chemical changes under physiological conditions (e.g. are hydrolyzed or acted on by naturally occurring enzyme(s)) resulting in liberation of the pharmacologically active agent. Prodrugs include compounds according to any embodiment described herein, wherein a hydroxyl, amino, or carboxy group is bonded to any group that may be cleaved *in vivo* to regenerate the free hydroxyl, amino or carboxy group, respectively. Examples of prodrugs include, but are not limited to esters (e.g., acetate, formate, and benzoate derivatives) of compounds according to any embodiment described herein, or any other derivative which upon being brought to the physiological pH or through enzyme action is converted to the active parent drug. Conventional procedures for the selection and preparation of suitable prodrug derivatives are described in the art (see, for example, Bundgaard. Design of Prodrugs. Elsevier, 1985).

In some embodiments, one or more hydrogen atoms of a compound according to any embodiment described herein, is replaced by a deuterium. It is well established that deuteration of physiologically active compounds offer the advantage of retaining the pharmacological profile of their hydrogen counterparts while positively impacting their metabolic outcome. Selective replacement of one or more hydrogen with deuterium, in a compound according to any embodiment described herein, could improve the safety, tolerability and efficacy of the compound when compared to its all hydrogen counterpart.

Methods for incorporation of deuterium into compounds is well established. Using metabolic studies establish in the art, a compound according to any embodiment described herein, can be tested to identify sites for selective placement of a deuterium isotope, wherein the isotope will not be metabolized. Moreover these studies identify sites of metabolism as the location where a deuterium atom would be placed.

### Pharmaceutical Compositions for use in the invention

Some embodiments describe a pharmaceutical composition comprising: a compound according to any embodiment described herein, a pharmaceutically acceptable salt thereof, a solvate thereof, a stereoisomer thereof, a prodrug thereof, or an active metabolites thereof; and a pharmaceutically acceptable carrier or diluent. The pharmaceutical compositions can be prepared in a manner well known in the pharmaceutical art, and can be administered by a variety of routes, depending upon whether local or systemic treatment is desired and upon the area to be treated.

While it is possible that a compound as described in any embodiment herein, may be administered as the bulk substance, it is preferable to present the compound in a pharmaceutical formulation, *e.g*., wherein the active agent is in an admixture with a pharmaceutically acceptable carrier selected with regard to the intended route of administration and standard pharmaceutical practice.

In particular, the disclosure provides a pharmaceutical composition comprising a therapeutically effective amount of at least one compound according to any embodiment described herein, and optionally, a pharmaceutically acceptable carrier.

### Combinations

For the pharmaceutical compositions and methods of the disclosure, a compound according to any embodiment described herein, may be used in combination with other therapies and/or active agents.

In some embodiments, the compound according to any embodiment described herein, can be combined with one or more of therapeutic agents useful for the treatment of neurological disorders including but not limited to donepezil, memantine and anti-abeta antibodies..

Accordingly, the disclosure provides, in a further aspect, a pharmaceutical composition comprising at least one compound according to any embodiment described herein, or pharmaceutically acceptable derivative thereof; a second active agent; and, optionally a pharmaceutically acceptable carrier.

When combined in the same formulation it will be appreciated that the two or more compounds must be stable and compatible with each other and the other components of the formulation. When formulated separately they may be provided in any convenient formulation, in such manner as are known for such compounds in the art.

Preservatives, stabilizers, dyes and flavoring agents may be provided in any pharmaceutical composition described herein. Examples of preservatives include sodium benzoate, ascorbic acid and esters of p-hydroxybenzoic acid. Antioxidants and suspending agents may be also used.

With respect to combinations including biologics such as monoclonal antibodies or fragments, suitable excipients will be employed to prevent aggregation and stabilize the antibody or fragment in solution with low endotoxin, generally for parenteral administration, for example, intravenous, administration. For example, see Formulation and Delivery Issues for Monoclonal Antibody Therapeutics, Daugherty et al., in Current Trends in Monoclonal Antibody Development and Manufacturing, Part 4, 2010, Springer, New York pp 103-129.

The compounds according to any embodiment described herein, may be milled using known milling procedures such as wet milling to obtain a particle size appropriate for tablet formation and for other formulation types. Finely divided (nanoparticulate) preparations of the compounds may be prepared by processes known in the art, for example see WO 02/00196 (SmithKline Beecham).

Compounds according to any embodiment described herein, or pharmaceutically acceptable salts thereof, a solvate thereof, a stereoisomer thereof, a prodrug thereof, or an active metabolites thereof, can be formulated for any route of administration.

### Routes of Administration and Unit Dosage Forms

The routes for administration (delivery) include, but are not limited to, one or more of: oral (e.g., as a tablet, capsule, or as an ingestible solution), topical, mucosal (e.g., as a nasal spray or aerosol for inhalation), parenteral (e.g., by an injectable form including but not limited to intraperitoneal, intramuscular, intravenous, intraspinal, intraspinal, intracerebroventricular, intrathecal or other depot administration etc.), or gastrointestinal, .

Therefore, the pharmaceutical compositions according to any embodiment described herein, include those in a form especially formulated for the mode of administration. In certain embodiments, the pharmaceutical compositions of the disclosure are formulated in a form that is suitable for oral delivery. In some embodiments, the compound is an orally bioavailable compound, suitable for oral delivery. In other embodiments, the pharmaceutical compositions of the disclosure are formulated in a form that is suitable for parenteral delivery.

The compounds according to any embodiment described herein, may be formulated for administration in any convenient way for use in human or veterinary medicine and the disclosure therefore includes within its scope pharmaceutical compositions comprising a compound according to any embodiment described herein, adapted for use in human or veterinary medicine. Such pharmaceutical compositions may be presented for use in a conventional manner with the aid of one or more suitable carriers. Acceptable carriers for therapeutic use are well-known in the pharmaceutical art, and are described, for example, in Remington's Pharmaceutical Sciences, Mack Publishing Co. (A. R. Gennaro edit. 1985). The choice of pharmaceutical carrier can be selected with regard to the intended route of administration and standard pharmaceutical practice. The pharmaceutical compositions may comprise as, in addition to, the carrier any suitable binder(s), lubricant(s), suspending agent(s), coating agent(s), and/or solubilizing agent(s).

There may be different pharmaceutical composition/formulation requirements depending on the different delivery systems. It is to be understood that not all of the compounds need to be administered by the same route. Likewise, if the pharmaceutical composition comprises more than one active component, then those components may be administered by different routes. By way of example, the pharmaceutical composition of the disclosure may be formulated to be delivered via a local ocular route, for example, as a subconjunctival ocular injection or intravitreal ocular injection, in which the pharmaceutical composition is formulated for delivery for injection into the eye. Alternatively, the formulation may be designed to be delivered systemically, in which the pharmaceutical composition is formulated for delivery by, for example, an intravenous or oral routes. Alternatively, the formulation may be designed to be delivered by multiple routes.

The combination of a compound according to any embodiment described herein, and an antibody or antibody fragment molecule can be formulated and administered by any of a number of routes and are administered at a concentration that is therapeutically effective in the indication or for the purpose sought. To accomplish this goal, the antibodies may be formulated using a variety of acceptable excipients known in the art. Typically, the antibodies are administered by injection, for example, intravenous injection. Methods to accomplish this administration are known to those of ordinary skill in the art. For example, Gokarn et al., 2008, J Pharm Sci 97(8):3051-3066, incorporated herein by reference, describe various high concentration antibody self buffered formulations. For example, monoclonal antibodies in self buffered formulation at e.g., 50 mg/mL mAb in 5.25% sorbitol, pH 5.0; or 60 mg/mL mAb in 5% sorbitol, 0.01% polysorbate 20, pH 5.2; or conventional buffered formulations, for example, 50 mg/mL mAb1 in 5.25% sorbitol, 25 or 50 mM acetate, glutamate or succinate, at pH 5.0; or 60 mg/mL in 10 mM acetate or glutamate, 5.25% sorbitol, 0.01% polysorbate 20, pH 5.2; other lower concentration formulations can be employed as known in the art..

Because some compounds of the disclosure cross the blood brain barrier they can be administered by a variety of methods including for example systemic (e.g., by iv, SC, oral, mucosal, transdermal route) or localized methods (e.g., intracranially).

Where the compound according to any embodiment described herein, is to be delivered mucosally through the gastrointestinal mucosa, it should be able to remain stable during transit though the gastrointestinal tract; for example, it should be resistant to proteolytic degradation, stable at acid pH and resistant to the detergent effects of bile. For example, compounds according to any embodiment described herein, prepared for oral administration may be coated with an enteric coating layer. The enteric coating layer material may be dispersed or dissolved in either water or in a suitable organic solvent. As enteric coating layer polymers, one or more, separately or in combination, of the following can be used; e.g., solutions or dispersions of methacrylic acid copolymers, cellulose acetate phthalate, cellulose acetate butyrate, hydroxypropyl methylcellulose phthalate, hydroxypropyl methylcellulose acetate succinate, polyvinyl acetate phthalate, cellulose acetate trimellitate, carboxymethylethylcellulose, shellac or other suitable enteric coating layer polymer(s). In some embodiments, the aqueous enteric coating layer is a methacrylic acid copolymer.

Where appropriate, the pharmaceutical compositions according to any embodiment described herein, can be administered by inhalation, by use of a skin patch, orally in the form of tablets containing excipients such as starch or lactose, or in capsules or ovules either alone or in admixture with excipients, or in the form of elixirs, solutions or suspensions containing flavoring or coloring agents, or they can be injected parenterally, for example intravenously, intramuscularly or subcutaneously. For buccal or sublingual administration the pharmaceutical compositions according to any embodiment described herein, may be administered in the form of tablets or lozenges, which can be formulated in a conventional manner.

Where the pharmaceutical composition according to any embodiment described herein, is to be administered parenterally, such administration includes without limitation: intravenously, intraarterially, intrathecally, intraventricularly, intracranially, intramuscularly or subcutaneously administering the compound of the disclosure; and/or by using infusion techniques. Antibodies or fragments are typically administered parenterally, for example, intravenously.

Pharmaceutical compositions according to any embodiment described herein, suitable for injection or infusion may be in the form of a sterile aqueous solution, a dispersion or a sterile powder that contains the active ingredient, adjusted, if necessary, for preparation of such a sterile solution or dispersion suitable for infusion or injection. This preparation may optionally be encapsulated into liposomes. In all cases, the final preparation must be sterile, liquid, and stable under production and storage conditions. To improve storage stability, such preparations may also contain a preservative to prevent the growth of microorganisms. Prevention of the action of micro-organisms can be achieved by the addition of various antibacterial and antifungal agents, e.g., paraben, chlorobutanol, or acsorbic acid. In many cases isotonic substances are recommended, e.g., sugars, buffers and sodium chloride to assure osmotic pressure similar to those of body fluids, particularly blood. Prolonged absorption of such injectable mixtures can be achieved by introduction of absorption-delaying agents, such as aluminum monostearate or gelatin.

Dispersions can be prepared in a liquid carrier or intermediate, such as glycerin, liquid polyethylene glycols, triacetin oils, and mixtures thereof. The liquid carrier or intermediate can be a solvent or liquid dispersive medium that contains, for example, water, ethanol, a polyol (e.g., glycerol, propylene glycol or the like), vegetable oils, non-toxic glycerine esters and suitable mixtures thereof. Suitable flowability may be maintained, by generation of liposomes, administration of a suitable particle size in the case of dispersions, or by the addition of surfactants.

For parenteral administration, the compound according to any embodiment described herein, is best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

Sterile injectable solutions can be prepared by mixing a compound according to any embodiment described herein, with an appropriate solvent and one or more of the aforementioned carriers, followed by sterile filtering. In the case of sterile powders suitable for use in the preparation of sterile injectable solutions, preferable preparation methods include drying in vacuum and lyophilization, which provide powdery mixtures of the compounds and desired excipients for subsequent preparation of sterile solutions.

The compounds according to any embodiment described herein, may be formulated for use in human or veterinary medicine by injection (e.g., by intravenous bolus injection or infusion or via intramuscular, subcutaneous or intrathecal routes) and may be presented in unit dose form, in ampoules, or other unit-dose containers, or in multi-dose containers, if necessary with an added preservative. The pharmaceutical compositions for injection may be in the form of suspensions, solutions, or emulsions, in oily or aqueous vehicles, and may contain formulatory agents such as suspending, stabilizing, solubilizing and/or dispersing agents. Alternatively the active ingredient may be in sterile powder form for reconstitution with a suitable vehicle, e.g., sterile, pyrogen-free water, before use.

The compounds according to any embodiment described herein, can be administered in the form of tablets, capsules, troches, ovules, elixirs, solutions or suspensions, for immediate-, delayed-, modified-, sustained-, pulsed-or controlled-release applications.

The compounds according to any embodiment described herein, may also be presented for human or veterinary use in a form suitable for oral or buccal administration, for example in the form of solutions, gels, syrups, or suspensions, or a dry powder for reconstitution with water or other suitable vehicle before use. Solid pharmaceutical compositions such as tablets, capsules, lozenges, troches, pastilles, pills, boluses, powder, pastes, granules, bullets or premix preparations may also be used. Solid and liquid pharmaceutical compositions for oral use may be prepared according to methods well-known in the art. Such pharmaceutical compositions may also contain one or more pharmaceutically acceptable carriers and excipients which may be in solid or liquid form.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycolate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia.

Additionally, lubricating agents such as magnesium stearate, stearic acid, glyceryl behenate and talc may be included.

The pharmaceutical compositions according to any embodiment described herein, may be administered orally, in the form of rapid or controlled release tablets, microparticles, mini tablets, capsules, sachets, and oral solutions or suspensions, or powders for the preparation thereof. Oral preparations may optionally include various standard pharmaceutical carriers and excipients, such as binders, fillers, buffers, lubricants, glidants, dyes, disintegrants, odorants, sweeteners, surfactants, mold release agents, antiadhesive agents and coatings. Some excipients may have multiple roles in the pharmaceutical compositions, e.g., act as both binders and disintegrants.

Examples of pharmaceutically acceptable disintegrants for oral pharmaceutical compositions according to any embodiment described herein, include, but are not limited to, starch, pre-gelatinized starch, sodium starch glycolate, sodium carboxymethylcellulose, croscarmellose sodium, microcrystalline cellulose, alginates, resins, surfactants, effervescent compositions, aqueous aluminum silicates and cross-linked polyvinylpyrrolidone.

Examples of pharmaceutically acceptable binders for oral pharmaceutical compositions according to any embodiment described herein, include, but are not limited to, acacia; cellulose derivatives, such as methylcellulose, carboxymethylcellulose, hydroxypropylmethylcellulose, hydroxypropylcellulose or hydroxyethylcellulose; gelatin, glucose, dextrose, xylitol, polymethacrylates, polyvinylpyrrolidone, sorbitol, starch, pre-gelatinized starch, tragacanth, xanthine resin, alginates, magnesium aluminum silicate, polyethylene glycol or bentonite.

Examples of pharmaceutically acceptable fillers for oral pharmaceutical compositions according to any embodiment described herein, include, but are not limited to, lactose, anhydrolactose, lactose monohydrate, sucrose, dextrose, mannitol, sorbitol, starch, cellulose (particularly microcrystalline cellulose), dihydro- or anhydro-calcium phosphate, calcium carbonate and calcium sulphate.

Examples of pharmaceutically acceptable lubricants useful in the pharmaceutical compositions according to any embodiment described herein, include, but are not limited to, magnesium stearate, talc, polyethylene glycol, polymers of ethylene oxide, sodium lauryl sulphate, magnesium lauryl sulphate, sodium oleate, sodium stearyl fumarate, and colloidal silicon dioxide.

Examples of suitable pharmaceutically acceptable odorants for the oral pharmaceutical compositions according to any embodiment described herein, include, but are not limited to, synthetic aromas and natural aromatic oils such as extracts of oils, flowers, fruits (e.g., banana, apple, sour cherry, peach) and combinations thereof, and similar aromas. Their use depends on many factors, the most important being the organoleptic acceptability for the population that will be taking the pharmaceutical compositions.

Examples of suitable pharmaceutically acceptable dyes for the oral pharmaceutical compositions according to any embodiment described herein, include, but are not limited to, synthetic and natural dyes such as titanium dioxide, beta-carotene and extracts of grapefruit peel.

Examples of useful pharmaceutically acceptable coatings for the oral pharmaceutical compositions according to any embodiment described herein, typically used to facilitate swallowing, modify the release properties, improve the appearance, and/or mask the taste of the pharmaceutical compositions include, but are not limited to, hydroxypropylmethylcellulose, hydroxypropylcellulose and acrylate-methacrylate copolymers.

Suitable examples of pharmaceutically acceptable sweeteners for the oral pharmaceutical compositions according to any embodiment described herein, include, but are not limited to, aspartame, saccharin, saccharin sodium, sodium cyclamate, xylitol, mannitol, sorbitol, lactose and sucrose.

Suitable examples of pharmaceutically acceptable buffers include, but are not limited to, citric acid, sodium citrate, sodium bicarbonate, dibasic sodium phosphate, magnesium oxide, calcium carbonate and magnesium hydroxide.

Suitable examples of pharmaceutically acceptable surfactants include, but are not limited to, sodium lauryl sulphate and polysorbates.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the agent may be combined with various sweetening or flavoring agents, coloring matter or dyes, with emulsifying and/or suspending agents and with diluents such as water, ethanol, propylene glycol and glycerin, and combinations thereof.

As indicated, a compounds according to any embodiment described herein, can be administered intranasally or by inhalation and is conveniently delivered in the form of a dry powder inhaler or an aerosol spray presentation from a pressurized container, pump, spray or nebulizer with the use of a suitable propellant, e.g., dichlorodifluoromethane, trichlorofluoromethane, dichlorotetrafluoroethane, a hydrofluoroalkane such as 1,1,1,2-tetrafluoroethane (HFA 134AT) or 1,1,1,2,3,3,3-heptafluoropropane (HFA 227EA), carbon dioxide or other suitable gas. In the case of a pressurized aerosol, the dosage unit may be determined by providing a valve to deliver a metered amount. The pressurized container, pump, spray or nebulizer may contain a solution or suspension of the active compound, e.g., using a mixture of ethanol and the propellant as the solvent, which may additionally contain a lubricant, e.g., sorbitan trioleate.

Capsules and cartridges (made, for example, from gelatin) for use in an inhaler or insufflator may be formulated to contain a powder mix of a compound according to any embodiment described herein, and a suitable powder base such as lactose or starch.

For topical administration by inhalation of compounds according to any embodiment described herein, may be delivered for use in human or veterinary medicine via a nebulizer.

The pharmaceutical compositions of the disclosure may contain from 0.01 to 99% weight per volume of the active material. For topical administration, for example, the pharmaceutical composition will generally contain from 0.01-10%, more preferably 0.01-1% of the active material.

A compound according to any embodiment described herein, can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The pharmaceutical composition or unit dosage form, according to any embodiment described herein, may be administered according to a dosage and administration regimen defined by routine testing in the light of the guidelines given above in order to obtain optimal activity while minimizing toxicity or side effects for a particular patient. The dosage of the compounds or unit dosage form may vary according to a variety of factors such as underlying disease conditions, the individual's condition, weight, sex and age, and the mode of administration. The exact amount to be administered to a patient will vary depending on the state and severity of the disorder and the physical condition of the patient. A measurable amelioration of any symptom or parameter can be determined by a person skilled in the art or reported by the patient to the physician. It will be understood that any clinically or statistically significant attenuation or amelioration of any symptom or parameter is within the scope of the disclosure. Clinically significant attenuation or amelioration means perceptible to the patient and/or to the physician.

In some embodiments, the amount of the compound to be administered can range between about 0.01 and about 25 mg/kg/day. Generally, dosage levels of between 0.01 to 25 mg/kg of body weight daily are administered to the patient, e.g., humans. In some embodiments the therapeutically effective amount is between a lower limit of about 0.01 mg/kg of body weight, about 0.1 mg/kg of body weight, about 0.2 mg/kg of body weight, about 0.3 mg/kg of body weight, about 0.4 mg/kg of body weight, about 0.5 mg/kg of body weight, about 0.60 mg/kg of body weight, about 0.70 mg/kg of body weight, about 0.80 mg/kg of body weight, about 0.90 mg/kg of body weight, about 1 mg/kg of body weight, about 2.5 mg/kg of body weight, about 5 mg/kg of body weight, about 7.5 mg/kg of body weight, about 10 mg/kg of body weight, about 12.5 mg/kg of body weight, about 15 mg/kg of body weight, about 17.5 mg/kg of body weight, about 20 mg/kg of body weight, about 22.5 mg/kg of body weight, and about 25 mg/kg of body weight; and an upper limit of 25 mg/kg of body weight, about 22.5 mg/kg of body weight, about 20 mg/kg of body weight, about 17.5 mg/kg of body weight, about 15 mg/kg of body weight, about 12.5 mg/kg of body weight, about 10 mg/kg of body weight, about 7.5 mg/kg of body weight, about 5 mg/kg of body weight, about 2.5 mg/kg of body weight, about 1 mg/kg of body weight, about 0.9 mg/kg of body weight, about 0.8 mg/kg of body weight, about 0.7 mg/kg of body weight, about 0.6 mg/kg of body weight, about 0.5 mg/kg of body weight, about 0.4 mg/kg of body weight, about 0.3 mg/kg of body weight, about 0.2 mg/kg of body weight, about 0.1 mg/kg of body weight, and about 0.01 mg/kg of body weight. In some embodiments, the therapeutically effective amount is about 0.1 mg/kg/day to about 10 mg/kg/day; in some embodiments the therapeutically effective amount is about 0.2 and about 5 mg/kg/day. It will be understood that the pharmaceutical formulations of the disclosure need not necessarily contain the entire amount of the compound that is effective in treating the disorder, as such effective amounts can be reached by administration of a plurality of divided doses of such pharmaceutical formulations. The compounds may be administered on a regimen of 1 to 4 times per day, such as once, twice, three times or four times per day.

In some embodiments of the disclosure, a compound according to any embodiment described herein, is formulated in capsules or tablets, usually containing about 10 to about 200 mg of the compounds. In some embodiments the capsule or tablet contains between a lower limit of about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, about 100 mg, about 105 mg, about 110 mg, about 115 mg; about 120 mg, about 125 mg, about 130 mg, about 135 mg, about 140 mg, about 145 mg, about 150 mg, about 155 mg, about 160 mg, about 165 mg, about 170 mg, about 175 mg, about 180 mg, about 185 mg, about 190 mg, about 195 mg, and about 200 mg, and an upper limit of about 200 mg, about 195 mg, about 190 mg, about 185 mg, about 180 mg, about 175 mg, about 170 mg, about 165 mg, about 160 mg, about 155 mg, about 150 mg, about 145 mg, about 140 mg, about 135 mg, about 130 mg, about 125 mg, about 120 mg, about 115 mg, about 110 mg, about 105 mg, about 100 mg, about 95 mg, about 90 mg; about 85 mg, about 80 mg, about 75 mg, about 70 mg, about 65 mg, about 60 mg, about 55 mg, about 50 mg, about 45 mg, about 40 mg, about 35 mg, about 30 mg, about 25 mg, about 20 mg, about 15 mg, and about 10 mg of a compound according to any embodiment herein.

In some embodiments, a compound according to any embodiment herein is administered to a patient at a total daily dose of 50 mg to 500 mg. In some embodiments, the daily dose is between a lower limit of about 50 mg, about 55 mg, about 60 mg, about 65 mg, about 70 mg, about 75 mg, about 80 mg, about 85 mg, about 90 mg, about 95 mg, about 100 mg, about 105 mg, about 110 mg, about 115 mg; about 120 mg, about 125 mg, about 130 mg, about 135 mg, about 140 mg, about 145 mg, about 150 mg, about 155 mg, about 160 mg, about 165 mg, about 170 mg, about 175 mg, about 180 mg, about 185 mg, about 190 mg, about 195 mg, about 200 mg, about 205 mg, about 210 mg, about 215 mg; about 220 mg, about 225 mg, about 230 mg, about 235 mg, about 240 mg, about 245 mg, about 250 mg, about 255 mg, about 260 mg, about 265 mg, about 270 mg, about 275 mg, about 280 mg, about 285 mg, about 290 mg, about 295 mg, 300 mg, about 305 mg, about 310 mg, about 315 mg; about 320 mg, about 325 mg, about 330 mg, about 335 mg, about 340 mg, about 345 mg, about 350 mg, about 355 mg, about 360 mg, about 365 mg, about 370 mg, about 375 mg, about 380 mg, about 385 mg, about 390 mg, about 395, about 400 mg, about 405 mg, about 410 mg, about 415 mg; about 420 mg, about 425 mg, about 430 mg, about 435 mg, about 440 mg, about 445 mg, about 450 mg, about 455 mg, about 460 mg, about 465 mg, about 470 mg, about 475 mg, about 480 mg, about 485 mg, about 490 mg, about 495 mg, and about 500 mg and an upper limit of about 500 mg, about 495 mg, about 490 mg, about 485 mg, about 480 mg, about 475 mg, about 470 mg, about 465 mg, about 460 mg, about 455 mg, about 450 mg, about 445 mg, about 440 mg, about 435 mg, about 430 mg, about 425 mg, about 420 mg, about 415 mg, about 410 mg, about 405 mg, about 400 mg, about 395 mg, about 390 mg, about 385 mg, about 380 mg, about 375 mg, about 370 mg, about 365 mg, about 360 mg, about 355 mg, about 350 mg, about 345 mg, about 340 mg, about 335 mg, about 330 mg, about 325 mg, about 320 mg, about 315 mg, about 310 mg, about 305 mg about 300 mg, about 295 mg, about 290 mg, about 285 mg, about 280 mg, about 275 mg, about 270 mg, about 265 mg, about 260 mg, about 255 mg, about 250 mg, about 245 mg, about 240 mg, about 235 mg, about 230 mg, about 225 mg, about 220 mg, about 215 mg, about 210 mg, about 205 mg 200 mg, about 195 mg, about 190 mg, about 185 mg, about 180 mg, about 175 mg, about 170 mg, about 165 mg, about 160 mg, about 155 mg, about 150 mg, about 145 mg, about 140 mg, about 135 mg, about 130 mg, about 125 mg, about 120 mg, about 115 mg, about 110 mg, about 105 mg, about 100 mg, about 95 mg, about 90 mg; about 85 mg, about 80 mg, about 75 mg, about 70 mg, about 65 mg, about 60 mg, about 55 mg, and about 50 mg of a compound according to any embodiment herein. In some embodiments, the total daily dose is about 50 mg to 150 mg. In some embodiments, the total daily dose is about 50 mg to 250 mg. In some embodiments, the total daily dose is about 50 mg to 350 mg. In some embodiments, the total daily dose is about 50 mg to 450 mg. In some embodiments, the total daily dose is about 50 mg.

A pharmaceutical composition for parenteral administration contains from about 0.01% to about 100% by weight of the active compound according to any embodiment described herein, based upon 100% weight of total pharmaceutical composition.

Generally, transdermal dosage forms contain from about 0.01% to about 100% by weight of the active compound according to any embodiment described herein, versus 100% total weight of the dosage form.

The pharmaceutical composition or unit dosage form may be administered in a single daily dose, or the total daily dosage may be administered in divided doses. In addition, co administration or sequential administration of another compound for the treatment of the disorder may be desirable. To this purpose, the combined active principles are formulated into a simple dosage unit.

Provided herein is embodiment A, a method of treating a neurologic disease, comprising administering to a subject in need thereof, a therapeutically effective amount of a compound selected from the group consisting of: a compound of Formula I, 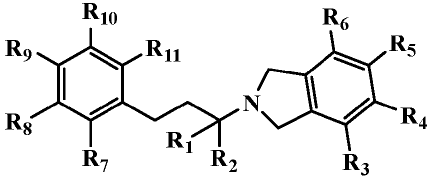 or a pharmaceutically acceptable salt thereof: wherein: each of R1 and R2 is independently selected from H, C1-C6 alkyl, or CH2OR'; wherein each R' if present in R1, and R2 is independently H or C1-C6 alkyl; each of R3, R4, R5, and R6 is independently selected from the group consisting of H, C1-C6 alkyl, OH, OCH3, OCH(CH3)2, OCH2CH(CH3)2, OC(CH3)3, O(C1-C6 alkyl), OCF3, OCH2CH2OH, O(C1-C6 alkyl)OH, O(C1-C6 haloalkyl), F, Cl, Br, I, CF3, CN, NO2, NH2, C1-C6 haloalkyl, C1-C6 hydroxyalkyl, C1-C6 alkoxy C1-C6alkyl, aryl, heteroaryl, C3-C7 cycloalkyl, heterocycloalkyl, alkylaryl, CO2R', C(O)R', NH(C1-C4 alkyl), N(C1-C4 alkyl)2, NH(C3-C7 cycloalkyl), NHC(O)(C1-C4 alkyl), CONR'2, NC(O)R', NS(O)nR', S(O)nNR'2, S(O)nR', C(O)O(C1-C4 alkyl), OC(O)N(R')2, C(O)(C1-C4 alkyl), and C(O)NH(C1-C4 alkyl); wherein each R' if present in R3, R4, R5, and R6 is independently selected from the group consisting of H, CH3, CH2CH3, C3-C6 alkyl, C1-C6 haloalkyl, or optionally substituted aryl, alkylaryl, piperazin-1-yl, piperidin-1-yl, morpholinyl, heterocycloalkyl, heteroaryl, C1-C6 alkoxy, NH(C1-C4 alkyl), and N(C1-C4 alkyl)2, wherein the optionally substituted group is selected from C1-C6 alkyl or C2-C7 acyl; or R3 and R4, together with the C atom to which they are attached form a 4-, 5-, 6- 7-or 8- membered cycloalkyl, aryl, heteroaryl, or heterocycloalkyl that is optionally substituted with 1, 2, 3, 4, or 5 substituents independently selected from OH, amino, halo, C1-C6 alkyl, C1-C6 haloalkyl, C1-C6 alkoxy, C1-C6 haloalkoxy, aryl, arylalkyl, heteroaryl, heteroarylalkyl, cycloalkyl, and heterocycloalkyl or R3 and R4 are linked together to form a -O-C1-C2 methylene-O- group; or R4 and R5, together with the C atom to which they are attached form a 4-, 5-, 6- 7-or 8- membered cycloalkyl, aryl, heteroaryl, or heterocycloalkyl that is optionally substituted with 1, 2, 3, 4, or 5 substituents independently selected from OH, amino, halo, C1-C6 alkyl, C1-C6 haloalkyl, C1-C6 alkoxy, C1-C6 haloalkoxy, aryl, arylalkyl, heteroaryl, heteroarylalkyl, cycloalkyl, and heterocycloalkyl or R4 and R5 are linked together to form a -O-C1-2 methylene-O- group; each of R7, R8, R9, R10, and R11 is independently selected from the group consisting of H, C1-C6 alkyl, OH, OCH3, OCH(CH3)2, OCH2CH(CH3)2, OC(CH3)3, O(C1-C6 alkyl), OCF3, OCH2CH2OH, O(C1-C6 alkyl)OH, O(C1-C6 haloalkyl), O(CO)R', F, Cl, Br, I, CF3, CN, NO2, NH2, C1-C6 haloalkyl, C1-C6 hydroxyalkyl, C1-C6 alkoxy C1-C6 alkyl, aryl, heteroaryl, C3-C7 cycloalkyl, heterocycloalkyl, alkylaryl, heteroaryl, CO2R', C(O)R', NH(C1-C4 alkyl), N(C1-C4 alkyl)2, NH(C3-C7 cycloalkyl), NHC(O)(C1-C4 alkyl), CONR'2, NC(O)R', NS(O)nR', S(O)nNR'2, S(O)nR', C(O)O(C1-C4 alkyl), OC(O)N(R')2, C(O)(C1-C4 alkyl), and C(O)NH(C1-C4 alkyl); wherein each R' if present in R7, R8, R9, R10, and R11 is independently selected from the group consisting of H, CH3, CH2CH3, C3-C6 alkyl, C1-C6 haloalkyl, aryl, alkylaryl, piperazin-1-yl, piperidin-1-yl, morpholinyl, heterocycloalkyl, heteroaryl, C1-C6 alkoxy, NH(C1-C4 alkyl), and N(C1-C4 alkyl)2; or R7 and R8, together with the N or C atoms to which they are attached form a 4-, 5-, 6- 7- or 8-membered cycloalkyl, aryl, heterocycloalkyl or heteroaryl group that is optionally substituted with 1, 2, 3, 4, or 5 substituents independently selected from OH, amino, halo, C1-C6 alkyl, C1-C6 haloalkyl, C1-C6 alkoxy, C1-C6 haloalkoxy, aryl, arylalkyl, heteroaryl, heteroarylalkyl, cycloalkyl, and heterocycloalkyl or R7 and R8 are linked together to form a - O-C1-2 methylene-O- group; or R8 and R9, together with the N or C atoms to which they are attached form a 4-, 5-, 6- 7-or 8- membered cycloalkyl, aryl, heterocycloalkyl or heteroaryl group that is optionally substituted with 1, 2, 3, 4, or 5 substituents independently selected from OH, amino, halo, C1-C6 alkyl, C1-C6 haloalkyl, C1-C6 alkoxy, C1-C6 haloalkoxy, aryl, arylalkyl, heteroaryl, heteroarylalkyl, cycloalkyl, and heterocycloalkyl or R8 and R9 are linked together to form a -O-C1-2 methylene-O- group; each n is independently 0, 1, or 2; with the proviso that R7, R8, R9, R10, and R11 are not all H; and with the proviso that the following compounds, or pharmaceutically acceptable salts thereof are excluded: 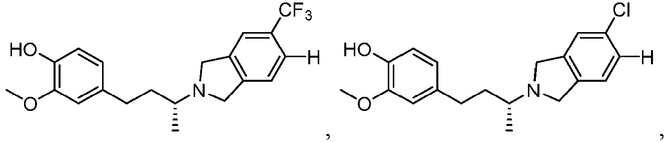 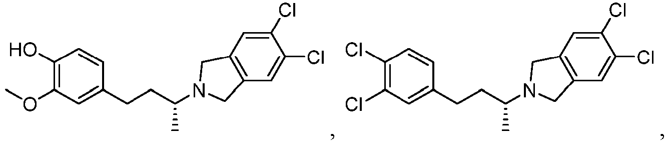 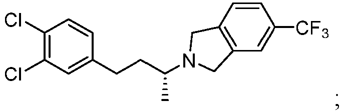 or

Provided herein is an embodiment B, a method of treating dry age-related macular degeneration (dry AMD), comprising administering to a subject in need thereof, a therapeutically effective amount of a compound selected from the group consisting of a compound of Formula IA: 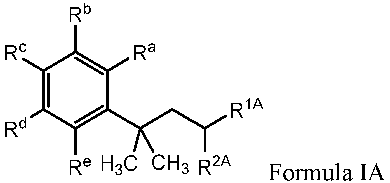 or pharmaceutically acceptable salt thereof: wherein: each of Ra, Rb, Rc, Rd and Re is independently selected from the group consisting of, H, hydroxyl, Cl, F, methyl, -OCH3, - OC(CH3)3, O-CH(CH3)2, CF3, SO2CH3, and morpholino; R1A is selected from the group consisting of hydrogen, alkyl, phenyl, or -CH=C(CH3)2; and R2A is an optionally substituted cyclic amino group.

In an embodiment C, the method of embodiment A, wherein the compound is a compound of Formula I or a pharmaceutically acceptable salt thereof.

In an embodiment D, the method of any one of embodiments A to C, wherein the compound is 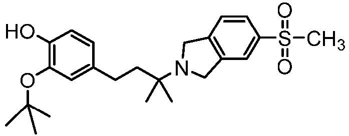 or a pharmaceutically acceptable salt thereof.

In an embodiment E, the method of any one of embodiments A to D, wherein the pharmaceutically acceptable salt is selected from the group consisting of hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate and pamoate salts.

In an embodiment F, the method of any one of embodiments A to E, wherein the pharmaceutically acceptable salt is the fumarate salt.

In an embodiment G, the method of any one of embodiments A to F, wherein the compound is

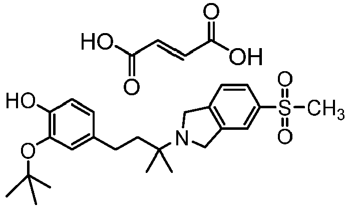

In an embodiment H, the method of embodiment B, wherein the compound is a compound of Formula IA or a pharmaceutically acceptable salt thereof.

In an embodiment I, the method of either one of embodiments A, or H, wherein the R^{2A} is optionally substituted piperidinyl.

In an embodiment J, the method of any one of embodiments A, H, or I, wherein the R^{2A} s selected from the group consisting of

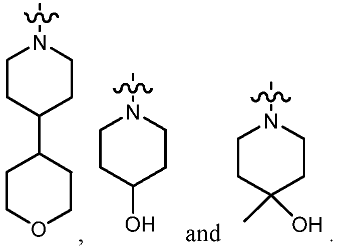

In an embodiment K, the method of any one of embodiments A, H, I, or J, wherein the compound is selected from the group consisting of 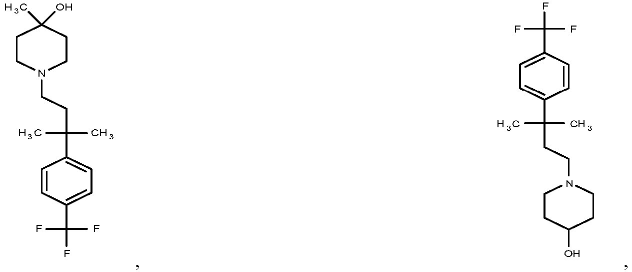 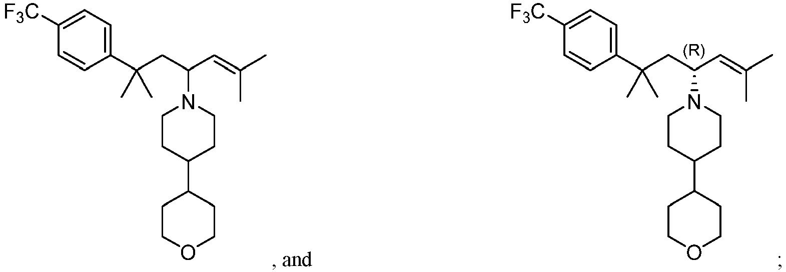 or a pharmaceutically acceptable salt thereof.

Provided herein is embodiment L, a method of treating a neurologic disease, comprising administering to a subject in need thereof, a therapeutically effective amount of a compound the compound is selected from the group consisting of:

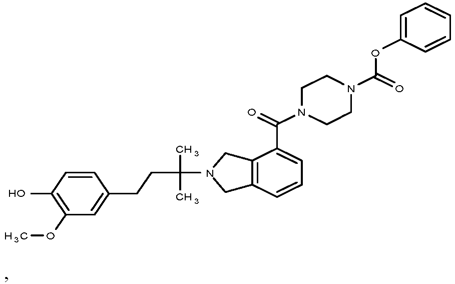

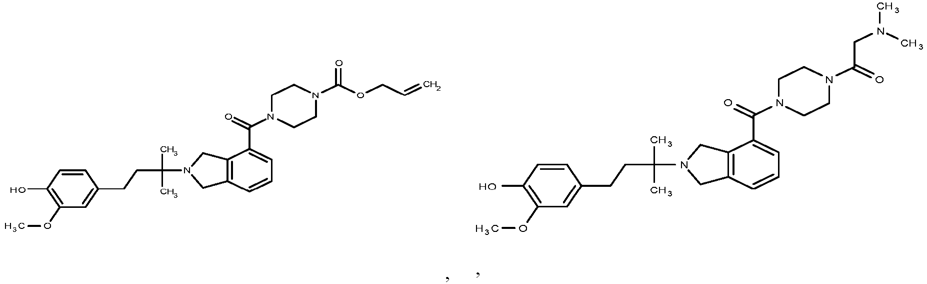

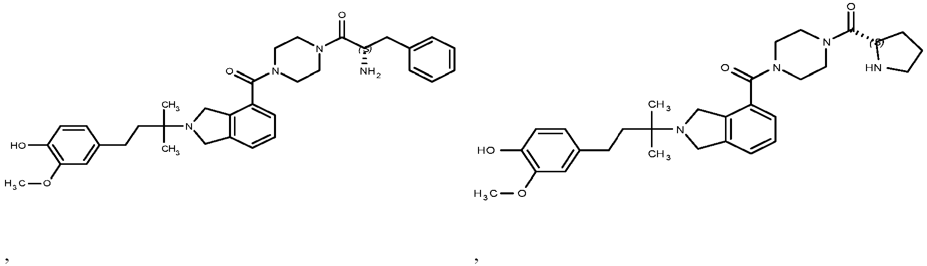

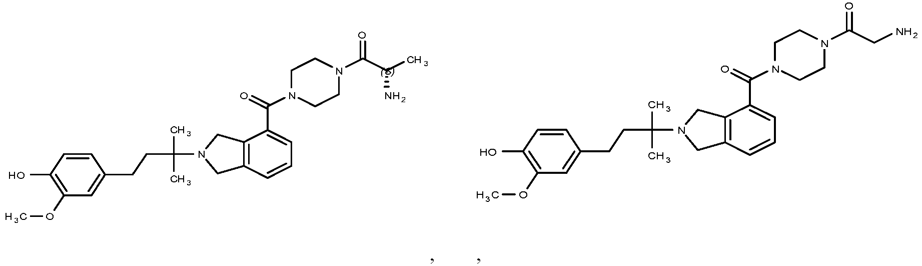

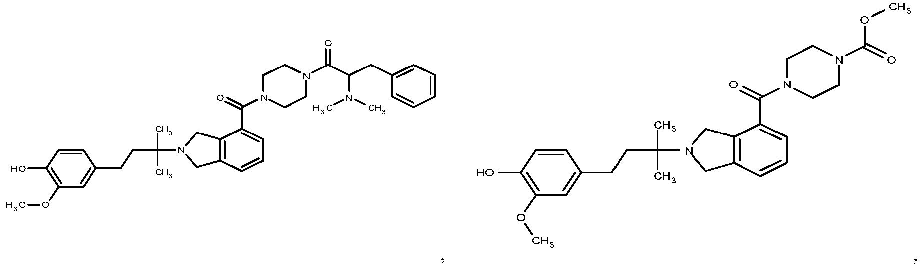

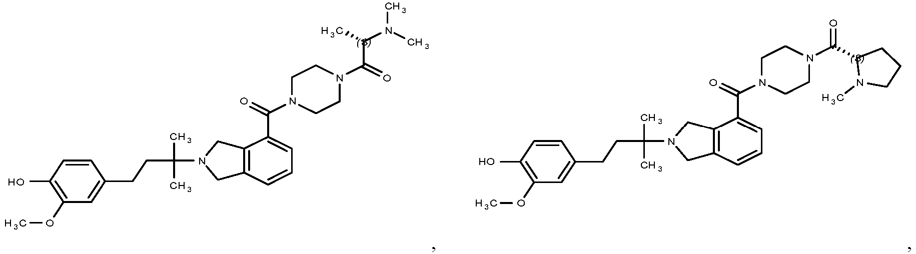

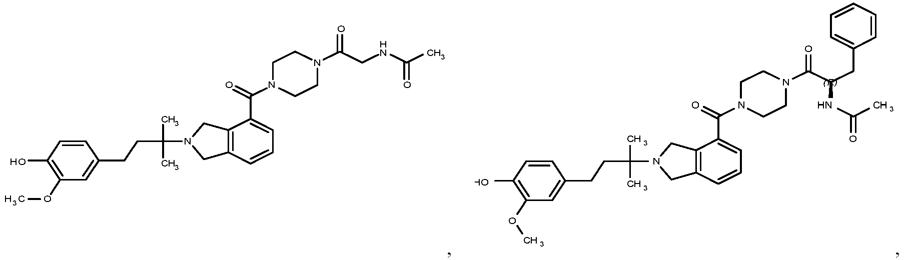

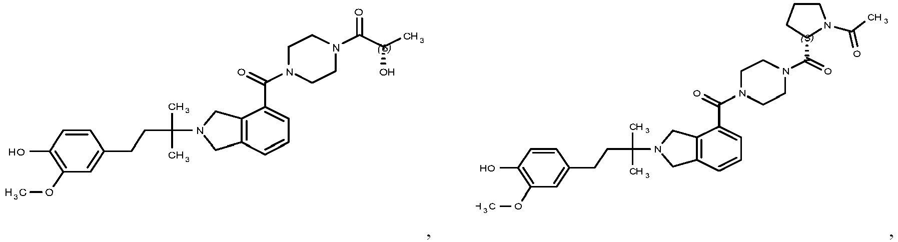

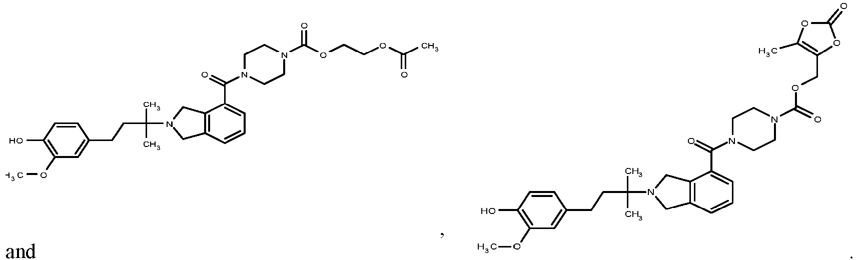

In an embodiment M, a method of treating neurologic disease comprising administering to a subject in need thereof, a therapeutically effective amount of a pharmaceutical composition comprising a compound according to any one of embodiments A to L and a pharmaceutically acceptable excipient.

Provided herein is embodiment N, a method of treating neurologic disease comprising administering to a subject in need thereof, a therapeutically effective amount of a pharmaceutical composition comprising a compound selected from the group comprising: 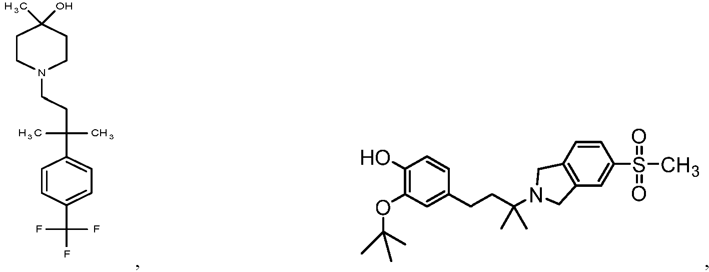 and 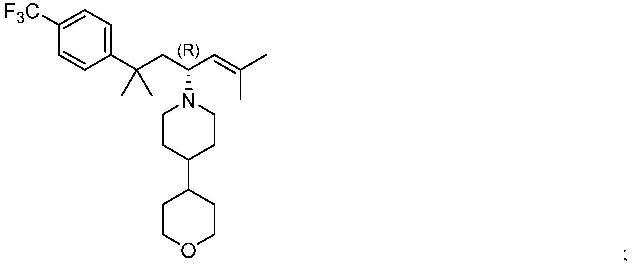 or a pharmaceutically acceptable salt thereof, and a pharmaceutically acceptable excipient.

In an embodiment O, the method of embodiment N, wherein the pharmaceutically acceptable salt is selected from the group consisting of hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate and pamoate salts.

In an embodiment P, the method of embodiment N, wherein the pharmaceutically acceptable salt is the fumarate salt.

In an embodiment Q, the method of embodiment P, wherein the compound is 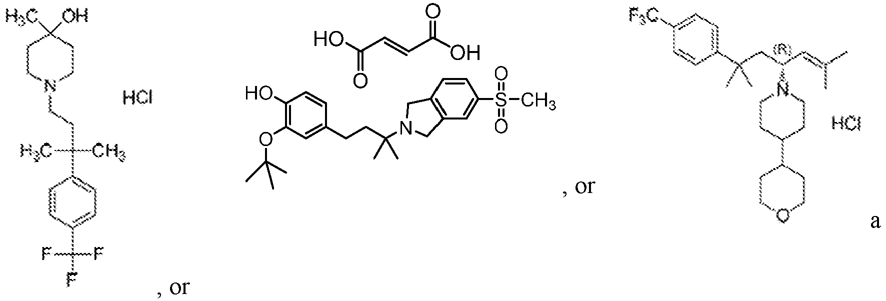 pharmaceutically acceptable salt thereof.

Provided herein is an embodiment R, a use of a compound selected from 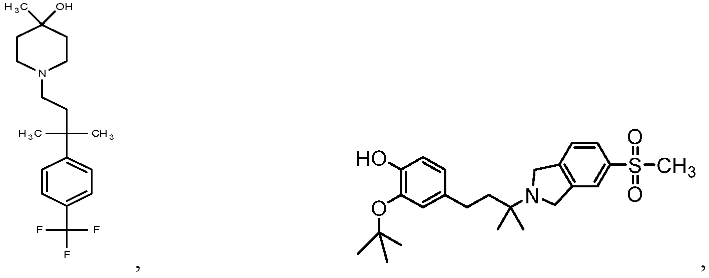 and 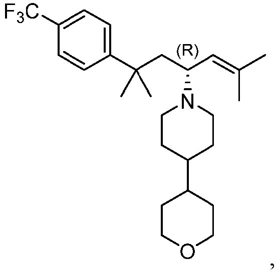 or a pharmaceutically acceptable salt thereof in the manufacture of a medicament for the treatment of neurologic disease.

Provided herein is an embodiment S, a use of a composition comprising a compound selected from 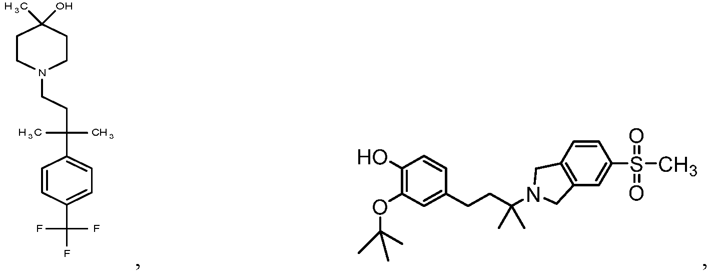 and 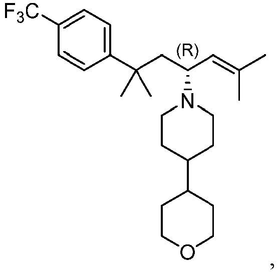 or a pharmaceutically acceptable salt thereof and a pharmaceutically acceptable excipient; in the manufacture of a medicament for neurologic disease.

In an embodiment T, the use of the compound or composition of either of embodiments R or S, wherein the compound is a pharmaceutically acceptable salt thereof.

In an embodiment U, the use of any one of embodiments R to T, wherein the pharmaceutically acceptable salt is selected from the group consisting of hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate and pamoate salts.

In an embodiment V, the use of embodiment U, wherein the pharmaceutically acceptable salt is the fumarate salt.

In an embodiment W, the use of either of embodiments R or S, wherein the compound is

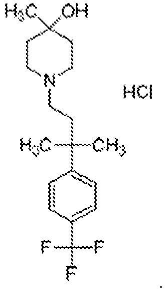

In an embodiment X, the use of either of embodiments R or S, wherein the compound is

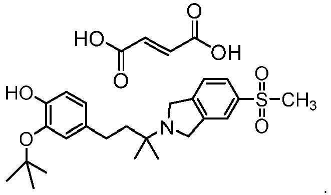

In an embodiment Y, the use of either of embodiments R or S, wherein the compound is

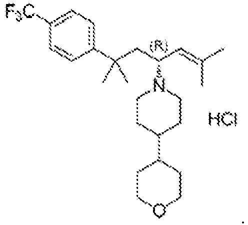

In an embodiment Z, the method of any one of embodiments A to Y wherein the neurological disease is selected from Age-Associated Memory Impairment (AAMI), Age-Related Cognitive Decline (ARCD), agitation synucleinopathies, Alzheimer's disease (AD), Amyotrophic lateral sclerosis (ALS) dementia, autosomal-dominant Parkinson's disease, chemotherapy-induced neuropathy (CIPN), Cognitive Impairment No Dementia (CIND), dementia, Creutzfeldt-Jakob disease (CJD), Diffuse Lewy Body Disease (DLBD) also known as Dementia with Lewy Bodies (DLB), disorders or conditions characterized by the presence of Lewy bodies, Down syndrome, dyskinesia, epilepsy, frontotemporal dementia (FTD), HIV-associated Neurocognitive Disorder (HAND), HIV dementia, Huntington's disease, Incidental LBD, Inherited LBD, Lewy body dysphagia, Mild Cognitive Impairment (MCI), multiple sclerosis, multiple system atrophy (MSA), Neuropathies (including but not limited to peripheral neuropathy, diabetic neuropathy and retinal neuropathy), Olivopontocerebellar Atrophy, Parkinson's disease (PD), preclinical Alzheimer's Disease (PCAD), psychiatric disorders (including but not limited to schizophrenia, bipolar disorders, depression, mania, anxiety disorders, post-traumatic stress disorders, delirium, eating disorders, autism, REM sleep behavior disorder, halucinations, attention-deficit hyperactivity disorders, and psychosis), Pure Autonomic Failure, seizures, Shy-Drager Syndrome, Striatonigral Degeneration, synucleinopathies, traumatic brain injury (TBI), combined Alzheimer's and Parkinson disease and/or MSA, vascular dementia, diseases, disorders or conditions associated with abnormal expression, stability, activities and/or cellular processing of α-synuclein, diseases, disorders or conditions characterized by the presence of Lewy bodies, and combinations thereof.

In an embodiment AA, the use of a compound or composition according to any one of embodiments A to Z, in the manufacture of a medicament for the treatment of neurologic disease.

In an embodiment BB, the use of embodiments A to AA, wherein the compound is administered orally.

### EXAMPLES

Compounds according to any embodiment described herein, may be prepared by the general and specific methods outlined in, for example, WO2013/029057, WO2015/116923 and WO2018/213281, each of which is incorporated by reference in their entirety, said methods constituting a further aspect of the disclosure. The following sigma-2 receptor modulators were used throughout the examples:

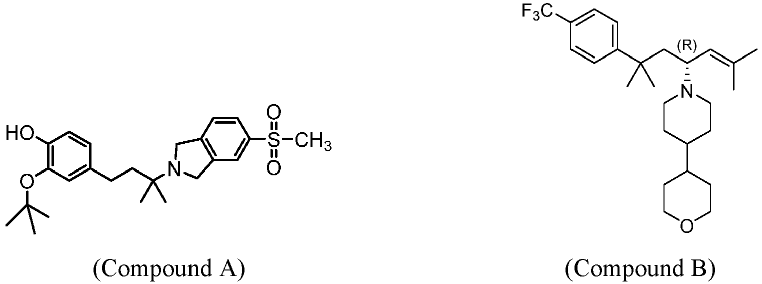

### Example 1: Displacement of Alpha-Synuclein Oligomers

### Experimental Design:

Rat neuronal cultures were treated with vehicle (an α seeding control (7.1nM) without α -synuclein oligomers (α-synOs)) or synthetic α-synOs for 24 hours. Cultures were immunostained for the neurite marker microtubule associated protein 2 (MAP2; green) and for α-synOs using an alpha-synuclein, oligomer-specific antibody (ASYO5 antibody, Agrisera; red).

### Analysis and Results:

Compound A and Compound B block α-synO binding to neuronal synapses and rescue α-synO-induced trafficking deficits in a concentration-dependent manner. Untreated vehicle control cultures without any α -SynO addition lack α-synO immunoreactivity (FIG. 1A). Neurons treated with 1 µM α-synO show punctate binding of a-synOs along neurites in the absence (FIG. 1B) but not presence (FIG. 1C) of Compound A (10 µM). Compound B (FIG. 1D) and Compound A (FIG. 1E) block α-synO binding to neuronal synapses in a concentration-dependent manner and rescue α-synO-induced trafficking deficits in a concentration-dependent manner. α-synOs (1.0 µM final concentration) caused significant deficits in vesicle trafficking (black circle) when compared with untreated vehicle control (square). Compound B exhibited an Emax=92% and EC50=0.31 µM) (FIG. 1F) and Compound A exhibited an Emax=87% and EC50=650nM (FIG. 1G) ameliorated this α-synO deficit in a concentration-dependent manner. FIGs. 1D, 1E, 1F, 1G: * p<0.05, **p<0.01, ***p<0.01, ****p<0.001, one-way ANOVA for drug concentration vs control with Dunnett's post-test.

### Example 2:

### Experimental Design:

Compounds A and B demonstrate efficacy in animal models of Parkinson's. Examples include models of overexpression of α-synuclein or models that mimic the propagation of α-synuclein throughout the brain. One such overexpression model is the rat AAV1/2 A53T-α-synuclein model (Koprich, J.B., Johnston, T.H., Huot, P., Reyes, M.G., Espinosa, M., Brotchie, J.M., "Progressive neurodegeneration or endogenous compensation in an animal model of Parkinson's disease produced by decreasing doses of alpha-synuclein," PLoS One, 6:1-9 (2011), which is hereby incorporated herein by reference in its entirety), where induction of α-synuclein overexpression is accompanied by loss of nigral dopaminergic cells, lower dopamine transporter levels, lower dopamine levels and motor deficits. Another example uses intrastriatal injection of preformed fibrils of α-synuclein to model propagation of Parkinson's pathology throughout the brain (Duffy, M.F., Collier, T.J., Patterson, J.R., Kemp, C.J., Fischer D.L., Stoll, A.C., Sortwell, C.E., "Quality over quantity: Advantages of using alpha-synuclein preformed fibrils triggered synucleinopathy to model idiopathic Parkinson's disease," Front Neurosci, 12:1-10 (2018) and Patterson, J.R., Duffy, M.F., Kemp, C.J., Howe, J.W., Collier, T.J., Stoll, A.C., Miller, K.M., Patel, P., Levine, N., Moore, D.J., Luk, K.C., Fleming, S.M., Kanaan, N.M, Paumier, K.L., El Agnaf, O.M.A., Sortwell, C.E., "Time course and magnitude of alpha-synuclein inclusion formation and nigrostriatal degeneration in the rate model of synucleinopathy triggered by intrastriatal alpha-synuclein preformed fibrils," Neurobiol Dis, 130 (2019), which are hereby incorporated herein by reference in their entirety). The model using injection of preformed α-synuclein fibrils recapitulates several aspects of idiopathic Parkinson's disease, such as increased alpha-synuclein oligomer concentrations, increased pSer129 levels and a reduction in striatal dopamine and dopamine transporter expression in a manner that propagates outward from the injection site.

### Results

Treatments: Animals in both models of Parkinson's disease with either Compound A or Compound B reduces some or all deficits that are characteristic of Parkinson's disease and/or slow propagation of such deficits throughout the brain.
Further aspects of the invention are provided in the numbered clauses below.
1. A method of treating a neurologic disease, comprising administering to a subject in need thereof, a therapeutically effective amount of a compound selected from the group consisting of:
   A. a compound of Formula I, 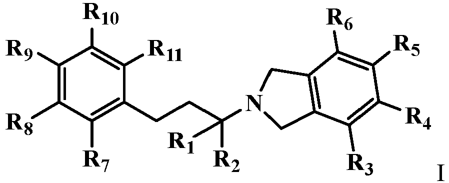 or a pharmaceutically acceptable salt thereof:
      wherein:
      each of R₁ and R₂ is independently selected from H, C₁-C₆ alkyl, or CH₂OR'; wherein each R' if present in R₁, and R₂ is independently H or C₁-C₆ alkyl;
      each of R₃, R₄, R₅, and R₆ is independently selected from the group consisting of H, C₁-C₆ alkyl, OH, OCH₃, OCH(CH₃)₂, OCH₂CH(CH₃)₂, OC(CH₃)₃, O(C₁-C₆ alkyl), OCF₃, OCH₂CH₂OH, O(C₁-C₆ alkyl)OH, O(C₁-C₆ haloalkyl), F, Cl, Br, I, CF₃, CN, NO₂, NH₂, C₁-C₆ haloalkyl, C₁-C₆ hydroxyalkyl, C₁-C₆ alkoxy C₁-C₆alkyl, aryl, heteroaryl, C₃-C₇ cycloalkyl, heterocycloalkyl, alkylaryl, CO₂R', C(O)R', NH(C₁-C₄ alkyl), N(C₁-C₄ alkyl)₂, NH(C₃-C₇ cycloalkyl), NHC(O)(C₁-C₄ alkyl), CONR'₂, NC(O)R', NS(O)ₙR', S(O)ₙNR'₂, S(O)ₙR', C(O)O(C₁-C₄ alkyl), OC(O)N(R')₂, C(O)(C₁-C₄ alkyl), and C(O)NH(C₁-C₄ alkyl), wherein each R' if present in R₃, R₄, R₅, and R₆ is independently selected from the group consisting of H, CH₃, CH₂CH₃, C₃-C₆ alkyl, C₁-C₆ haloalkyl, or optionally substituted aryl, alkylaryl, piperazin-1-yl, piperidin-1-yl, morpholinyl, heterocycloalkyl, heteroaryl, C₁-C₆ alkoxy, NH(C₁-C₄ alkyl), and N(C₁-C₄ alkyl)₂, wherein the optionally substituted group is selected from C₁-C₆ alkyl or C₂-C₇ acyl;
      or R₃ and R₄, together with the C atom to which they are attached form a 4-, 5-, 6- 7-or 8- membered cycloalkyl, aryl, heteroaryl, or heterocycloalkyl that is optionally substituted with 1, 2, 3, 4, or 5 substituents independently selected from OH, amino, halo, C₁-C₆ alkyl, C₁₋C₆ haloalkyl, C₁₋C₆ alkoxy, C₁₋C₆ haloalkoxy, aryl, arylalkyl, heteroaryl, heteroarylalkyl, cycloalkyl, and heterocycloalkyl or R₃ and R₄ are linked together to form a - O-C₁₋C₂ methylene-O- group;
      or R₄ and R₅, together with the C atom to which they are attached form a 4-, 5-, 6- 7-or 8- membered cycloalkyl, aryl, heteroaryl, or heterocycloalkyl that is optionally substituted with 1, 2, 3, 4, or 5 substituents independently selected from OH, amino, halo, C₁₋C₆ alkyl, C₁₋C₆ haloalkyl, C₁₋C₆ alkoxy, C₁₋C₆ haloalkoxy, aryl, arylalkyl, heteroaryl, heteroarylalkyl, cycloalkyl, and heterocycloalkyl or R₄ and R₅ are linked together to form a - O-C₁₋₂ methylene-O- group;
      each of R₇, R₈, R₉, R₁₀, and R₁₁ is independently selected from the group consisting of H, C₁-C₆ alkyl, OH, OCH₃, OCH(CH₃)₂, OCH₂CH(CH₃)₂, OC(CH₃)₃, O(C₁-C₆ alkyl), OCF₃, OCH₂CH₂OH, O(C₁-C₆ alkyl)OH, O(C₁-C₆ haloalkyl), O(CO)R', F, Cl, Br, I, CF₃, CN, NO₂, NH₂, C₁-C₆ haloalkyl, C₁-C₆ hydroxyalkyl, C₁-C₆ alkoxy C₁-C₆ alkyl, aryl, heteroaryl, C₃-C₇ cycloalkyl, heterocycloalkyl, alkylaryl, heteroaryl, CO₂R', C(O)R', NH(C₁-C₄ alkyl), N(C₁-C₄ alkyl)₂, NH(C₃-C₇ cycloalkyl), NHC(O)(C₁-C₄ alkyl), CONR'₂, NC(O)R', NS(O)ₙR', S(O)ₙNR'₂, S(O)ₙR', C(O)O(C₁-C₄ alkyl), OC(O)N(R')₂, C(O)(C₁-C₄ alkyl), and C(O)NH(C₁-C₄ alkyl); wherein each R' if present in R₇, R₈, R₉, R₁₀, and R₁₁ is independently selected from the group consisting of H, CH₃, CH₂CH₃, C₃-C₆ alkyl, C₁-C₆ haloalkyl, aryl, alkylaryl, piperazin-1-yl, piperidin-1-yl, morpholinyl, heterocycloalkyl, heteroaryl, C₁-C₆ alkoxy, NH(C₁-C₄ alkyl), and N(C₁-C₄ alkyl)₂;
      or R₇ and R₈, together with the N or C atoms to which they are attached form a 4-, 5-, 6- 7- or 8- membered cycloalkyl, aryl, heterocycloalkyl or heteroaryl group that is optionally substituted with 1, 2, 3, 4, or 5 substituents independently selected from OH, amino, halo, C₁₋C₆ alkyl, C₁₋C₆ haloalkyl, C₁₋C₆ alkoxy, C₁₋C₆ haloalkoxy, aryl, arylalkyl, heteroaryl, heteroarylalkyl, cycloalkyl, and heterocycloalkyl or R₇ and R₈ are linked together to form a -O-C₁₋₂ methylene-O- group;
      or R₈ and R₉, together with the N or C atoms to which they are attached form a 4-, 5-, 6- 7-or 8- membered cycloalkyl, aryl, heterocycloalkyl or heteroaryl group that is optionally substituted with 1, 2, 3, 4, or 5 substituents independently selected from OH, amino, halo, C₁₋C₆ alkyl, C₁₋C₆ haloalkyl, C₁₋C₆ alkoxy, C₁₋C₆ haloalkoxy, aryl, arylalkyl, heteroaryl, heteroarylalkyl, cycloalkyl, and heterocycloalkyl or R₈ and R₉ are linked together to form a -O-C₁₋₂ methylene-O- group;
      each n is independently 0, 1, or 2;
      with the proviso that R₇, R₈, R₉, R₁₀, and R₁₁ are not all H; and
      with the proviso that the following compounds, or pharmaceutically acceptable salts thereof are excluded: 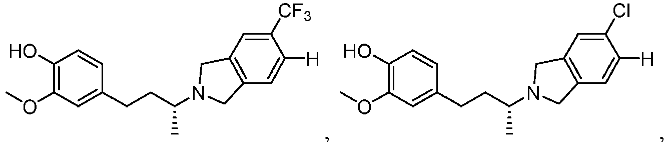 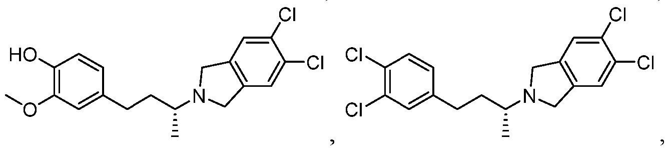 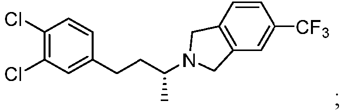 or
   B. a compound of Formula IA 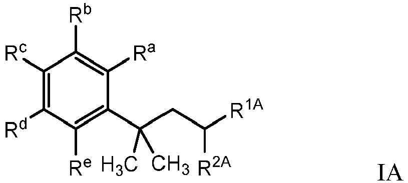 or pharmaceutically acceptable salt thereof:
      wherein:
      each of R^{a}, R^{b}, R^{c}, R^{d} and R^{e} is independently selected from the group consisting of, H, hydroxyl, Cl, F, methyl, -OCH₃, -OC(CH₃)₃, O-CH(CH₃)₂, CF₃, SO₂CH₃, and morpholino;
      R^{1A} is selected from the group consisting of hydrogen, alkyl, phenyl, or - CH=C(CH₃)₂; and
      R^{2A} is an optionally substituted cyclic amino group.
2. The method of clause 1 wherein the compound is a compound of Formula I or a pharmaceutically acceptable salt thereof.
3. The method of clause 1 wherein the compound is 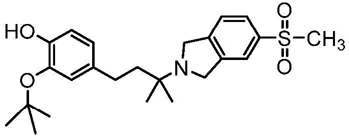 or a pharmaceutically acceptable salt thereof.
4. The method of any one of claims 1 to 3, wherein the pharmaceutically acceptable salt is selected from the group consisting of hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate and pamoate salts.
5. The method of any one of clauses 1 to 4, wherein the pharmaceutically acceptable salt is the fumarate salt.
6. The method of any one of clauses 1 to 5, wherein the compound is 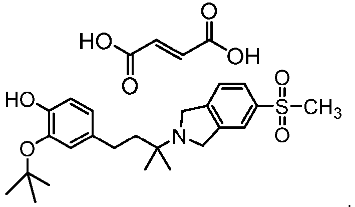
7. The method of clause 1, wherein the compound is a compound of Formula IA or a pharmaceutically acceptable salt thereof.
8. The method of either one of clauses 1 or 7, wherein the R^{2A} is optionally substituted piperidinyl.
9. The method of any one of clauses 1, 7, or 8, wherein the R^{2A} s selected from the group consisting of 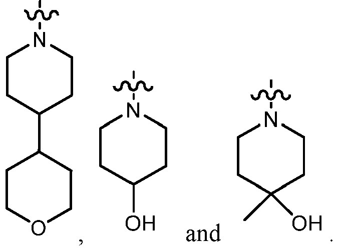
10. The method of any one of clauses 1, 7, 8, or 9, wherein the compound is selected from the group consisting of 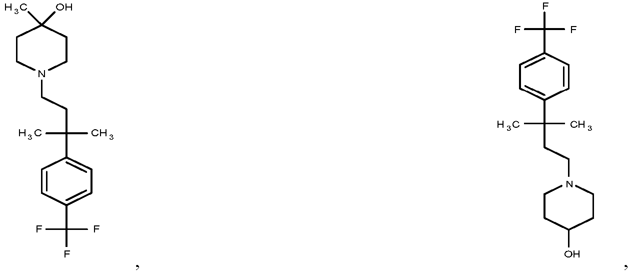 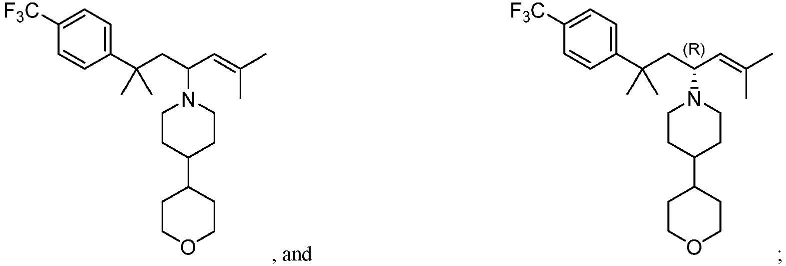 or a pharmaceutically acceptable salt thereof.
11. A method of treating a neurologic disease, comprising administering to a subject in need thereof, a therapeutically effective amount of a compound the compound is selected from the group consisting of: 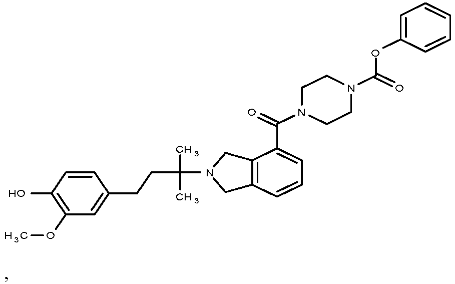 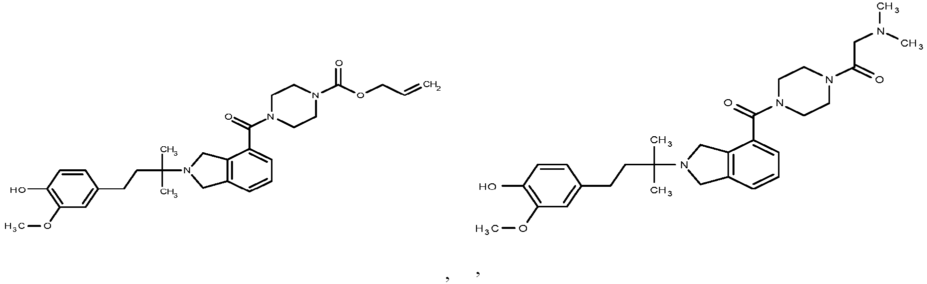 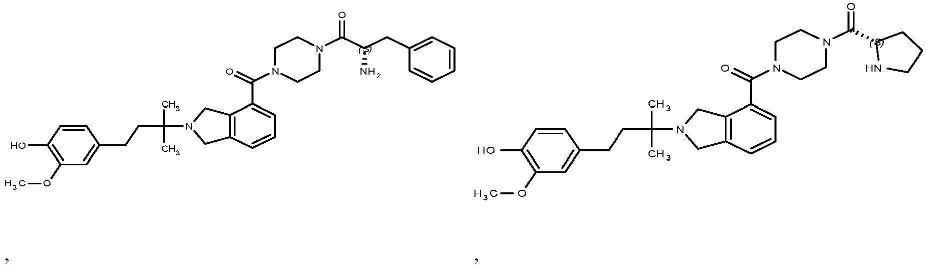 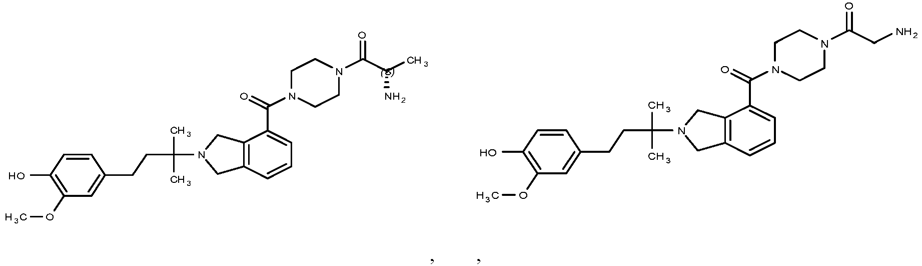 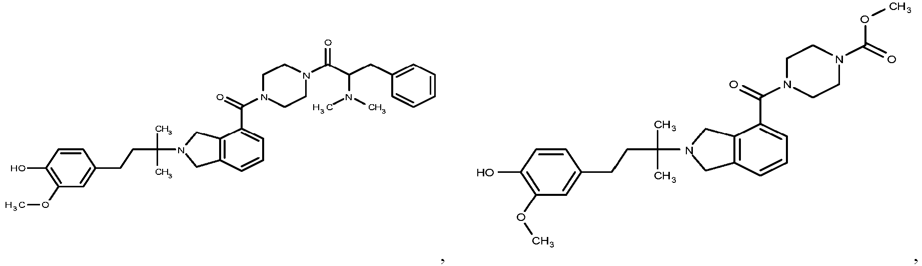 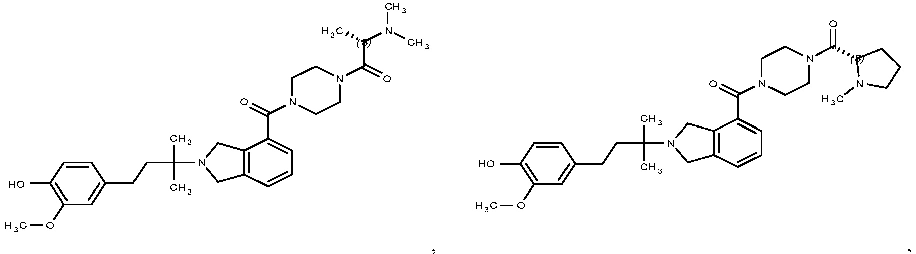 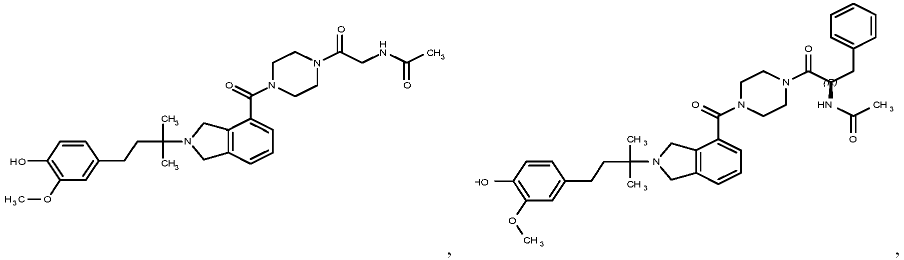 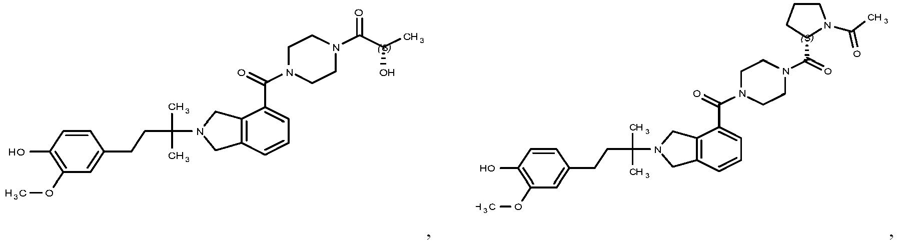 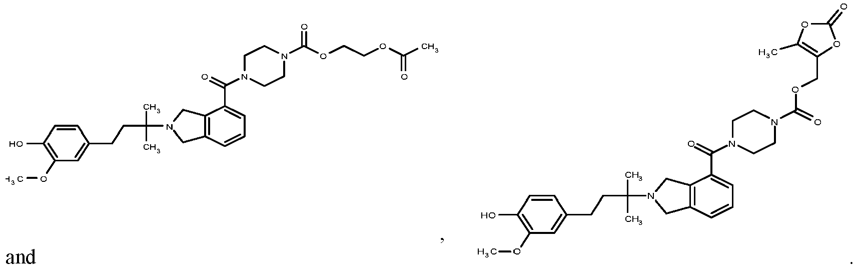
12. A method of treating neurologic disease comprising administering to a subject in need thereof, a therapeutically effective amount of a pharmaceutical composition comprising a compound according to any one of clauses 1 to 11 and a pharmaceutically acceptable excipient.
13. A method of treating neurologic disease comprising administering to a subject in need thereof, a therapeutically effective amount of a pharmaceutical composition comprising a compound selected from the group comprising: 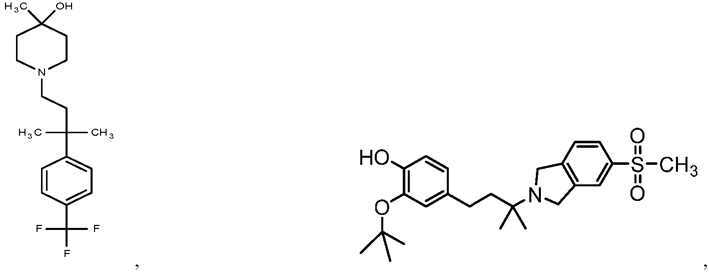 and 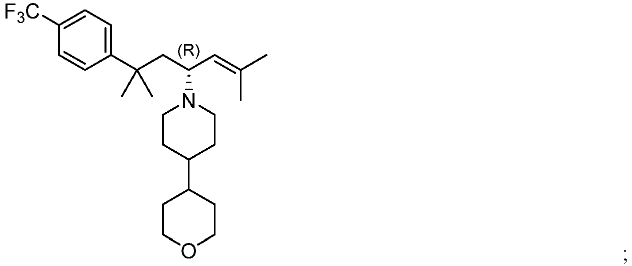 or a pharmaceutically acceptable salt thereof, and a pharmaceutically acceptable excipient.
14. The method of clause 13, wherein the pharmaceutically acceptable salt is selected from the group consisting of hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate and pamoate salts.
15. The method of clause 13, wherein the pharmaceutically acceptable salt is the fumarate salt.
16. The method of clause 15, wherein the compound is 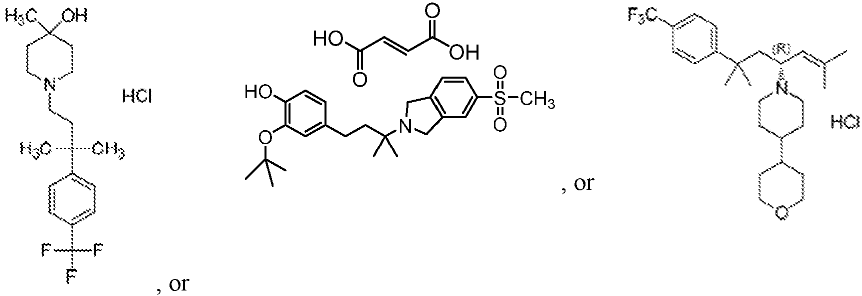 a pharmaceutically acceptable salt thereof.
17. Use of a compound selected from 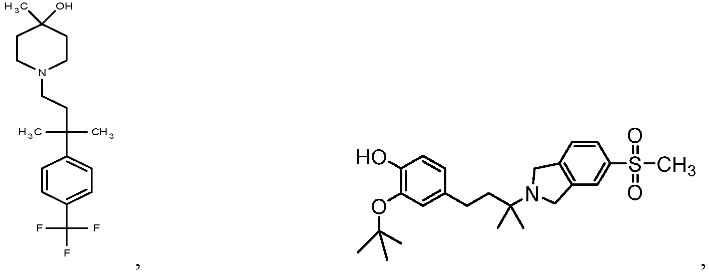 and 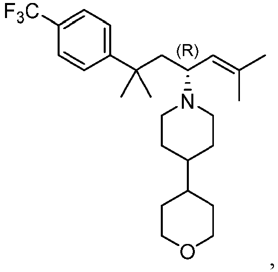 or a pharmaceutically acceptable salt thereof in the manufacture of a medicament for the treatment of neurologic disease.
18. Use of a composition comprising a compound selected from 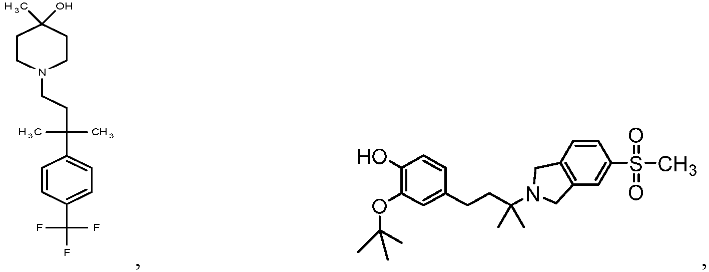 and 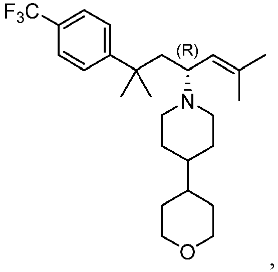 or a pharmaceutically acceptable salt thereof and a pharmaceutically acceptable excipient; in the manufacture of a medicament for neurologic disease.
19. The use of the compound or composition of either of clause 17 or 18, wherein the compound is a pharmaceutically acceptable salt thereof.
20. The use of any one of clauses 17 to 19, wherein the pharmaceutically acceptable salt is selected from the group consisting of hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate and pamoate salts.
21. The use of clause 20, wherein the pharmaceutically acceptable salt is the fumarate salt.
22. The use of either of clause 17 or 18, wherein the compound is 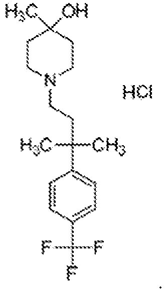
23. The use of either of clause 17 or 18, wherein the compound is 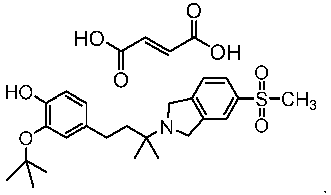
24. The use of either of clause 17 or 18, wherein the compound is 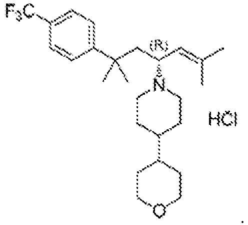
25. The method of any one of clauses 1 to 24 wherein the neurological disease is selected from Age-Associated Memory Impairment (AAMI), Age-Related Cognitive Decline (ARCD), agitation synucleinopathies, Alzheimer's disease (AD), Amyotrophic lateral sclerosis (ALS) dementia, autosomal-dominant Parkinson's disease, chemotherapy-induced neuropathy (CIPN), Cognitive Impairment No Dementia (CIND), dementia, Creutzfeldt-Jakob disease (CJD), Diffuse Lewy Body Disease (DLBD) also known as Dementia with Lewy Bodies (DLB), disorders or conditions characterized by the presence of Lewy bodies, Down syndrome, dyskinesia, epilepsy, frontotemporal dementia (FTD), HIV-associated Neurocognitive Disorder (HAND), HIV dementia, Huntington's disease, Incidental LBD, Inherited LBD, Lewy body dysphagia, Mild Cognitive Impairment (MCI), multiple sclerosis, multiple system atrophy (MSA), Neuropathies (including but not limited to peripheral neuropathy, diabetic neuropathy and retinal neuropathy), Olivopontocerebellar Atrophy, Parkinson's disease (PD), preclinical Alzheimer's Disease (PCAD), psychiatric disorders (including but not limited to schizophrenia, bipolar disorders, depression, mania, anxiety disorders, post-traumatic stress disorders, delirium, eating disorders, autism, REM sleep behavior disorder, halucinations, attention-deficit hyperactivity disorders, and psychosis), Pure Autonomic Failure, seizures, Shy-Drager Syndrome, Striatonigral Degeneration, synucleinopathies, traumatic brain injury (TBI), combined Alzheimer's and Parkinson disease and/or MSA, vascular dementia, diseases, disorders or conditions associated with abnormal expression, stability, activities and/or cellular processing of α-synuclein, diseases, disorders or conditions characterized by the presence of Lewy bodies, and combinations thereof.
26. Use of a compound or composition according to any one of clauses 1 to 24, in the manufacture of a medicament for the treatment of neurologic disease.
27. The use of clauses 1 through 26, wherein the compound is administered orally.

## Claims

1. A compound for use in a method of treating Parkinson's Disease, wherein the compound is: 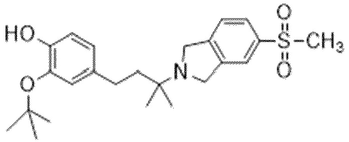 or a pharmaceutically acceptable salt thereof, the method comprising administering to a subject having said disease a total daily dose of 10 to 2000 mg of the compound or a pharmaceutically acceptable salt thereof.

2. The compound for use according to claim 1, wherein the pharmaceutically acceptable salt is selected from the group consisting of hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate and pamoate salts.

3. The compound for use according to claim 1 or 2, wherein the compound is

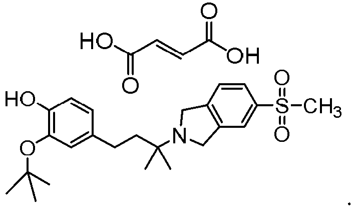

4. The compound for use according to any one of claims 1-3, wherein the total daily dose is 10 to 200 mg of the compound.

5. The compound for use according to any one of claims 1-3, wherein the total daily dose is 50 to 350 mg of the compound.

6. The compound for use according to claim 5, wherein the total daily dose is 50 to 150 mg of the compound.

7. The compound for use according to any one of claims 1-6, wherein the dose is administered in a single dose.

8. The compound for use according to any one of claims 1-6, wherein the dose is administered in multiple doses per day.

9. The compound for use according to any one of claims 1-6, wherein the compound or pharmaceutically acceptable salt thereof is administered in the form of a capsule or tablet.

10. The compound for use according to any of the preceding claims, wherein the compound or pharmaceutically acceptable salt thereof is administered orally.
